# EUROPEAN PATENT APPLICATION

(11) **EP 4 851 165 A1**
(43) Date of publication of application: **09.12.2026**
(21) Application number: 25747801.6
(22) Date of filing: 24.01.2025
(51) Int. Cl.: C07D 487/04, A61K 31/407, A61P 25/00

(54) **OREXIN RECEPTOR MODULATOR, AND PREPARATION METHOD THEREFOR AND USE THEREOF**

(30) Priority: 29.01.2024 CN 202410118856
(71) Applicant: Chengdu Kanghong Pharmaceutical Co Ltd, Chengdu, Sichuan 610036 (CN)
(72) Inventor: YU, Yongguo, Chengdu, Sichuan 610036 (CN); HUANG, Hong, Chengdu, Sichuan 610036 (CN); KE, Zunhong, Chengdu, Sichuan 610036 (CN)
(74) Representative: Dehns
(86) International application number: PCT/CN2025/074582
(87) International publication number: WO 2025/162233

(57) **Abstract**

Disclosed in the present invention are an orexin receptor modulator, and a preparation method therefor and the use thereof, and specifically disclosed is an orexin receptor antagonist as shown in the following formula. The orexin receptor antagonist provided by the present invention can be used for treating diseases related to the nervous system.

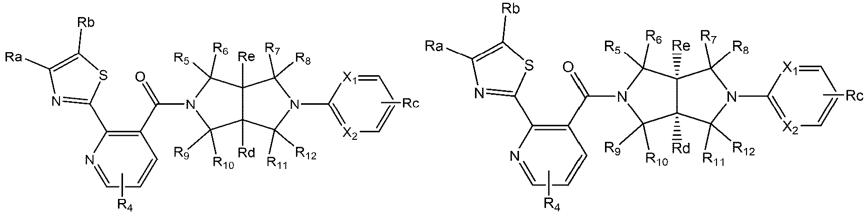

## Description

The present application claims the priority of Chinese Patent Application 2024101188562, filed on January 29, 2024. The present application incorporates by reference the entire disclosure of the above Chinese patent application.

### TECHNICAL FIELD

The present patent application relates to the field of pharmaceuticals, and relates to an orexin receptor modulator, a preparation method therefor and a use thereof in medicine.

### BACKGROUD OF THE INVENTION

Orexin, also known as hypocretin, includes orexin A and orexin B (or hypocretin-1 and hypocretin-2), is a neuropeptide secreted by hypothalamus, and has main physiological effects including: 1. regulating feeding, wherein the orexin can activate neurons that regulate food intake, thereby significantly promoting feeding in a dose-dependent manner; 2. participating in the regulation of energy metabolism, wherein the orexin can significantly increase metabolic rate; 3. participating in the regulation of sleep-wake cycles, wherein the orexin may inhibit rapid eye movement sleep and prolong wakefulness, so that blocking the action of orexin can promote sleep; 4. participating in endocrine regulation, wherein the orexin has a significant effect on the endocrine secretion of pituitary hormones; 5. being associated with reward sensation, learning and memory; 6. promoting gastric acid secretion; 7. promoting increased water intake; 8. elevating blood pressure; and 9. playing an important role in a reward system and a mechanisms of drug addiction; and the like.

The orexin produces physiological effects by acting on an orexin receptor (OXR). The orexin receptor is a G-protein coupled receptor, and there are two types of orexin receptors, which are respectively an OX1 receptor and an OX2 receptor, wherein the OX1 receptor selectively binds the orexin A, while the OX2 receptor may bind the orexin A and the orexin B. The OX1 receptor and the OX2 receptor almost exclusively exist in brain tissues, and are selectively expressed in the brain, wherein the OX1 receptor is expressed at high density in locus coeruleus, which is a nucleus of origin of noradrenergic neuron, while the OX2 receptor is expressed at high density in tuberomammillary nucleus, which is a nucleus of origin of histaminergic neuron. The expression of both the OX1 receptor and the OX2 receptor may be found in a raphe nucleus, which is a nucleus of origin of serotonergic neuron; and the expression of both the OX1 receptor and the OX2 receptor may also be found in a ventral tegmental area, which is a nucleus of origin of dopaminergic neuron.

Thus, it can be seen that the orexin receptor is of great pathological significance and associated with a variety of diseases, such as sleep disorder, depression, anxiety disorder, panic disorder, obsessive-compulsive disorder, affective neurosis, depressive neurosis, anxiety neurosis, mood disorder, panic attack disorder, behavioral disorder, emotional disturbance, post-traumatic stress disorder, sexual dysfunction, psychosis, schizophrenia, manic depression, delirium, dementia, drug dependence, addiction, cognitive impairment, Alzheimer's disease, Parkinson's disease, movement disorder, eating disorder, headache, migraine, pain, digestive system disease, epilepsy, inflammation, cardiovascular disease, diabetes, metabolic disease, immune-related disease, endocrine-related disease, hypertension, and the like.

At present, there are already a number of drugs that are in clinical stages or have been marketed, such as Suvorexant from Merck Company and Lemborexant from Eisai, while Seltorexant developed by Johnson & Johnson Company is in clinical stages.

### SUMMARY OF THE INVENTION

The present invention provides an orexin receptor antagonist, and it has been found that a compound having such structure exhibits good activity, selectivity, brain exposure, brain-to-plasma ratio (ratio of drug concentration in brain to drug concentration in plasma, abbreviated as B/P), low toxicity minimal side effects, long half-life, and the like.

The present invention relates to a compound as shown in the following formula, a stereoisomer thereof or a pharmaceutically acceptable salt thereof, wherein the compound has the following structure: 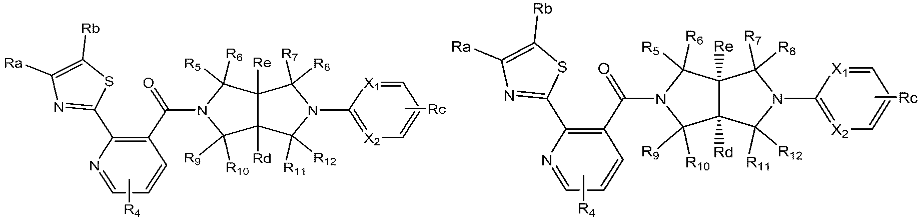
Ra, Rb and Rc are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, and the C3-8 cycloalkyl, C3-8 heterocyclyl, C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₄ is selected from H, deuterium, halogen, hydroxy, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C6-10 aryl and C5-10 heteroaryl, and the C3-8 cycloalkyl, C3-8 heterocyclyl, C6-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₅-R₁₂, Rₑ and R_{d} are each independently selected from hydrogen, deuterium, halogen, C1-6 alkyl, C1-6 haloalkyl and C1-6 deuterated alkyl; and
X₁ and X₂ are independently selected from CH or N; and
heteroatoms in the C3-8 heterocyclyl and C5-10 heteroaryl are one, two or three selceted from N, O and S, and the number of the heteroatoms is 1, 2 or 3.

In some embodiments, the compound has the following structure: 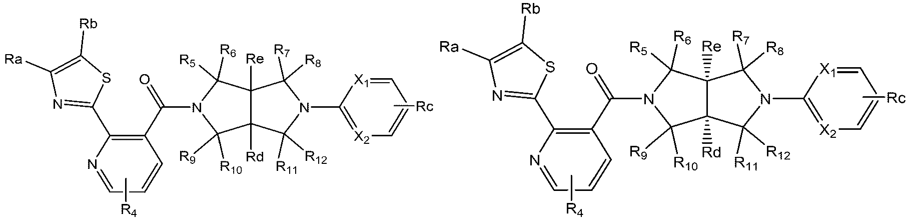
Ra, Rb and Rc are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, and the C3-8 cycloalkyl, C3-8 heterocyclyl, the C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₄ is selected from H, deuterium, halogen, hydroxy, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl and C3-8 heterocyclyl, and the C3-8 cycloalkyl and C3-8 heterocyclyl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₅-R₁₂, Rₑ and R_{d} are each independently selected from hydrogen, deuterium, halogen, C1-6 alkyl, C1-6 haloalkyl and C1-6 deuterated alkyl; and
X₁ and X₂ are selected from C or N.

In some embodiments, the compound of the present invention has a structure as shown in formula Ia or formula Ib: 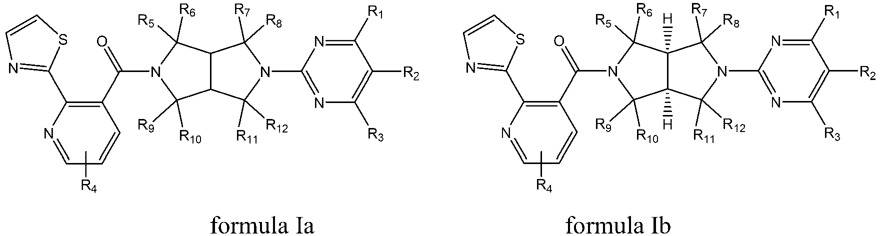
R₁, R₂ and R₃ are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, and the C3-8 cycloalkyl, C3-8 heterocyclyl, C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy; and
definitions of other substituents are as described above.

In some embodiments, the compound of the present invention has a structure as shown in formula IIIa or formula IIIb: 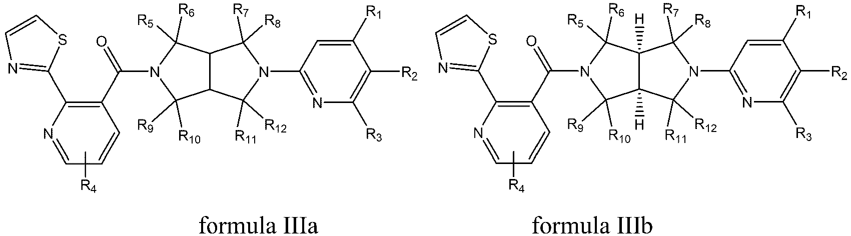
R₁, R₂ and R₃ are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, and the C3-8 cycloalkyl, C3-8 heterocyclyl, C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy; and
definitions of other substituents are as described above.

In some embodiments, the compound of the present invention has a structure as shown in formula IIa or formula IIb: 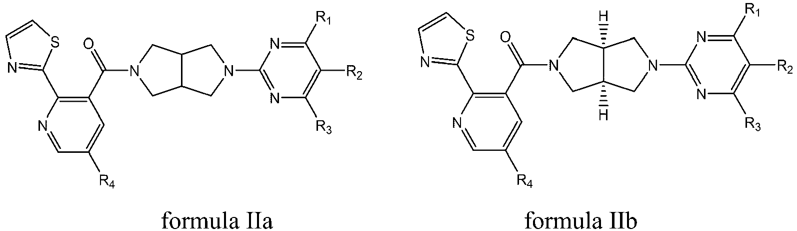 wherein, definitions of other substituents are as described above.

In some embodiments, in the compound, the stereoisomer thereof or the pharmaceutically acceptable salt thereof according to the present invention, R₁, R₂ and R₃ are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy and C3-8 cycloalkyl.

In some embodiments, in the compound, the stereoisomer thereof or the pharmaceutically acceptable salt thereof according to the present invention, R₁, R₂ and R₃ are independently selected from C1-3 alkyl, C1-3 haloalkyl, C1-3 deuterated alkyl, C1-3 hydroxyalkyl, C1-3 alkoxy, C1-3 haloalkoxy, C1-3 hydroxyalkoxy and C3-8 cycloalkyl.

In some embodiments, in the compound, the stereoisomer thereof or the pharmaceutically acceptable salt thereof according to the present invention, R₁ and R₃ are independently selected from C1-3 alkyl, C1-3 deuterated alkyl, C1-3 haloalkyl, C1-3 hydroxyalkyl, C1-3 alkoxy and C1-3 haloalkoxy, and R₂ is hydrogen.

In some embodiments, in the compound, the stereoisomer thereof or the pharmaceutically acceptable salt thereof according to the present invention, R₁ and R₃ are independently selected from methyl, deuterated methyl and hydroxymethyl, and R₂ is hydrogen.

In some embodiments, in the compound, the stereoisomer thereof or the pharmaceutically acceptable salt thereof according to the present invention, R₁, R₂ and R₃ are each independently selected from H, deuterium, halogen, C1-6 alkyl, C1-6 haloalkyl, C1-6 deuterated alkyl, C1-6 hydroxyalkyl, C1-6 alkoxy, C1-6 haloalkoxy and C1-6 hydroxyalkoxy.

In some embodiments, in the compound, the stereoisomer thereof or the pharmaceutically acceptable salt thereof according to the present invention, R₄ is selected from halogen, hydroxy, C1-3 alkyl, C1-3 haloalkyl, C1-3 deuterated alkyl, C1-3 hydroxyalkyl, C1-3 alkoxy, C1-3 haloalkoxy, C1-3 hydroxyalkoxy and C3-8 cycloalkyl, and the C3-8 cycloalkyl is optionally substituted with deuterium, halogen or C1-3 alkyl.

In some embodiments, in the compound, the stereoisomer thereof or the pharmaceutically acceptable salt thereof according to the present invention, R₄ is selected from fluoro, trifluoromethyl and cyclopropyl, and the cyclopropyl is optionally substituted with halogen or methyl.

In some embodiments, in the compound, the stereoisomer thereof or the pharmaceutically acceptable salt thereof according to the present invention, R₅-R₁₂ are each independently selected from hydrogen, deuterium or methyl.

In some embodiments, in the compound, the stereoisomer thereof or the pharmaceutically acceptable salt thereof according to the present invention, R₄ is hydrogen.

In some embodiments, in the compound, the stereoisomer thereof or the pharmaceutically acceptable salt thereof according to the present invention, R₁ and R₃ are each independently selected from hydrogen, C1-6 alkyl, C1-6 deuterated alkyl and C1-6 hydroxyalkyl.

In some embodiments, in the compound, the stereoisomer thereof or the pharmaceutically acceptable salt thereof according to the present invention, R₄ is H, C1-6 haloalkyl or C3-8 cycloalkyl.

In some embodiments, in the compound, the stereoisomer thereof or the pharmaceutically acceptable salt thereof according to the present invention, R₄ is H, trifluoromethyl or cyclopropyl.

In some embodiments, in the compound, the stereoisomer thereof or the pharmaceutically acceptable salt thereof according to the present invention, R₁ and R₃ are each independently selected from hydrogen, C1-3 alkyl and C1-3 deuterated alkyl.

In some embodiments, in the compound, the stereoisomer thereof or the pharmaceutically acceptable salt thereof according to the present invention, R₂ is hydrogen or halogen.

In some embodiments, in the compound, the stereoisomer thereof or the pharmaceutically acceptable salt thereof according to the present invention, R₄ is hydrogen, C1-3 alkyl or C1-3 haloalkyl.

In some embodiments, in the compound, the stereoisomer thereof or the pharmaceutically acceptable salt thereof according to the present invention, R₅-R₁₂ are each independently hydrogen.

In some embodiments, in the compound, the stereoisomer thereof or the pharmaceutically acceptable salt thereof according to the present invention, R₁ and R₃ are each independently selected from C1-3 alkyl and C1-3 deuterated alkyl.

In some embodiments, in the compound, the stereoisomer thereof or the pharmaceutically acceptable salt thereof according to the present invention, R₁ and R₃ are independently C1-3 deuterated alkyl.

In some embodiments, in the compound, the stereoisomer thereof or the pharmaceutically acceptable salt thereof according to the present invention, R₄ is hydrogen or C1-3 haloalkyl.

In some embodiments, in the compound, the stereoisomer thereof or the pharmaceutically acceptable salt thereof according to the present invention, R₁ and R₃ are each independently selected from hydrogen, methyl and deuterated methyl.

In some embodiments, in the compound, the stereoisomer thereof or the pharmaceutically acceptable salt thereof according to the present invention, R₁ and R₃ are independently methyl or deuterated methyl.

In some embodiments, in the compound, the stereoisomer thereof or the pharmaceutically acceptable salt thereof according to the present invention, R₁ and R₃ are deuterated methyl.

In some embodiments, in the compound, the stereoisomer thereof or the pharmaceutically acceptable salt thereof according to the present invention, R₁ and R₃ are independently H, -CH₃, -CHD₂, -CH₂D or -CD₃.

In some embodiments, in the compound, the stereoisomer thereof or the pharmaceutically acceptable salt thereof according to the present invention, R₁ and R₃ are independently H, -CH₃ or -CD₃.

In some embodiments, in the compound, the stereoisomer thereof or the pharmaceutically acceptable salt thereof according to the present invention, R₁ and R₃ are -CD₃.

In some embodiments, in the compound, the stereoisomer thereof or the pharmaceutically acceptable salt thereof according to the present invention, R₁ and R₃ are independently -CH₃ or -CD₃.

In some embodiments, in the compound, the stereoisomer thereof or the pharmaceutically acceptable salt thereof according to the present invention, R₄ is hydrogen or trifluoromethyl.

In some embodiments, in the compound, the stereoisomer thereof or the pharmaceutically acceptable salt thereof according to the present invention, R₂ is hydrogen or fluorine.

In some embodiments, in the compound, the stereoisomer thereof or the pharmaceutically acceptable salt thereof according to the present invention, R₂ is hydrogen.

In some embodiments, in the compound, the stereoisomer thereof or the pharmaceutically acceptable salt thereof according to the present invention, R₁ and R₃ are independently C1-3 alkyl, R₂ is halogen, and R₄ is hydrogen.

In some embodiments, in the compound, the stereoisomer thereof or the pharmaceutically acceptable salt thereof according to the present invention, R₁ and R₃ are methyl.

In some embodiments, in the compound, the stereoisomer thereof or the pharmaceutically acceptable salt thereof according to the present invention, R₂ is fluorine.

In some embodiments, in the compound, the stereoisomer thereof or the pharmaceutically acceptable salt thereof according to the present invention, R₄ is H, -F, -CF₃,

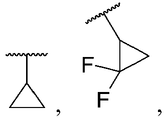

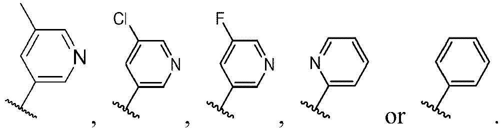

In some embodiments, in the compound, the stereoisomer thereof or the pharmaceutically acceptable salt thereof according to the present invention, R_{c} is H, -F, -Cl, -CH₃, -CH₂CH₃, -CD₃, -CF₃, 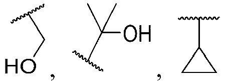 or -OCH₃.

In some embodiments, in the compound, the stereoisomer thereof or the pharmaceutically acceptable salt thereof according to the present invention, R₁ is H, -CH₃, -CH₂CH₃, -CD₃, -CF₃,

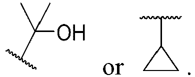

In some embodiments, in the compound, the stereoisomer thereof or the pharmaceutically acceptable salt thereof according to the present invention, R₂ is H, -Cl, -F, -CF₃ or -OCH₃.

In some embodiments, in the compound, the stereoisomer thereof or the pharmaceutically acceptable salt thereof according to the present invention, R₃ is H, -CH₃, -CD₃ or

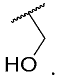

In another aspect, the present application provides a compound having the following structure, a stereoisomer thereof or a pharmaceutically acceptable salt thereof:

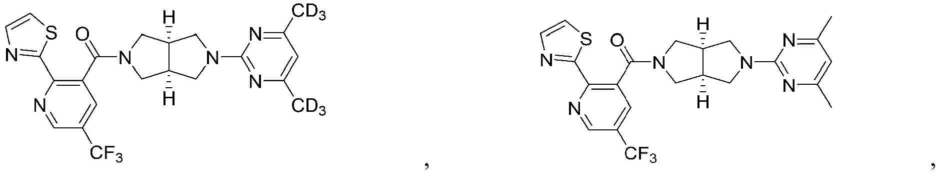

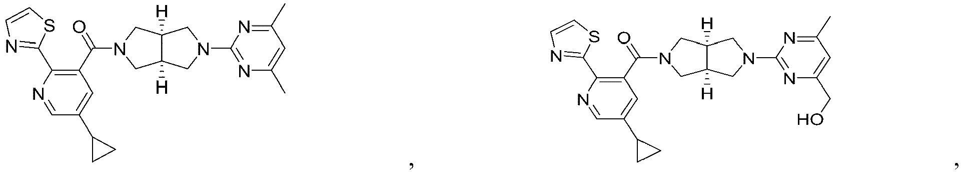

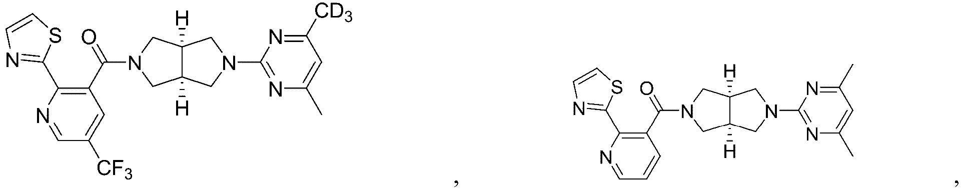

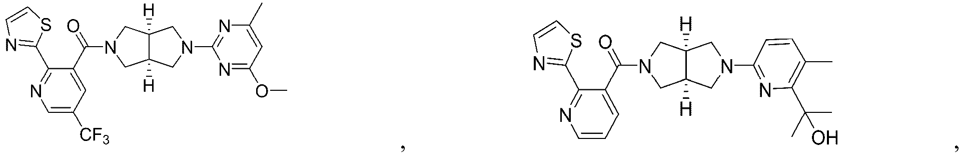

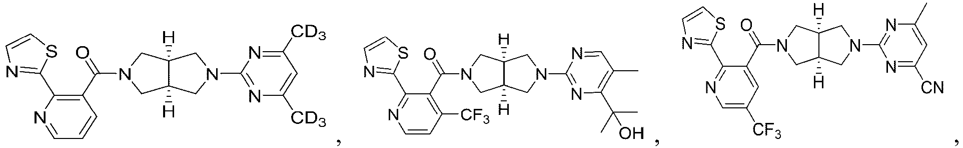

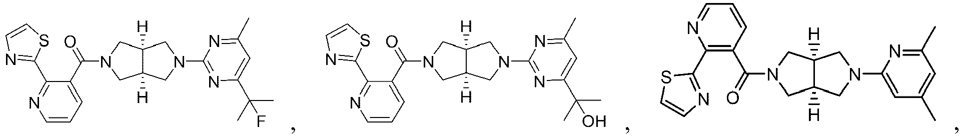

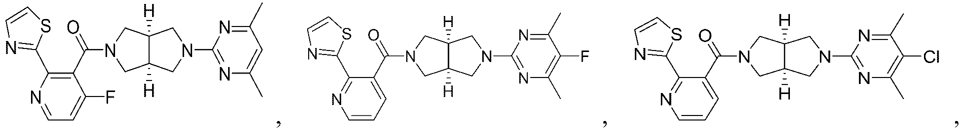

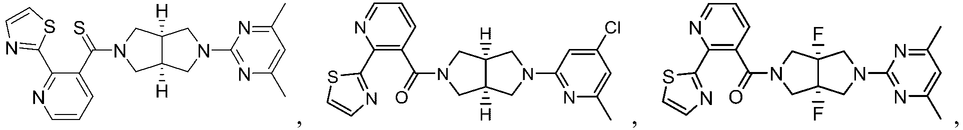

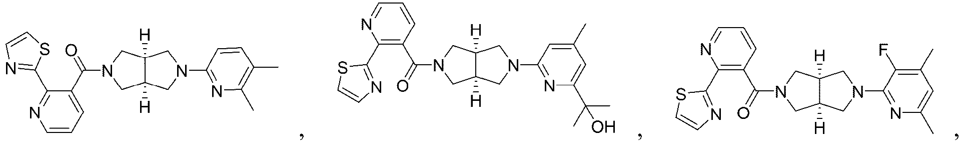

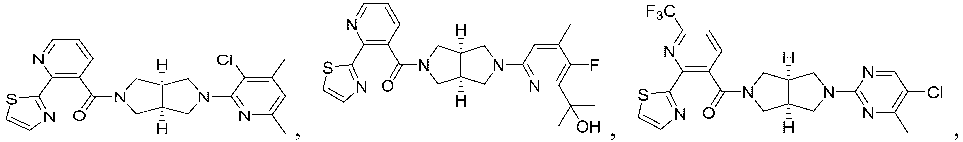

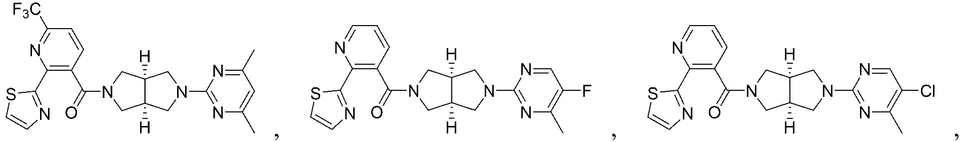

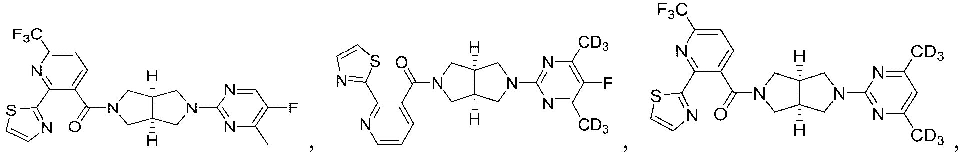

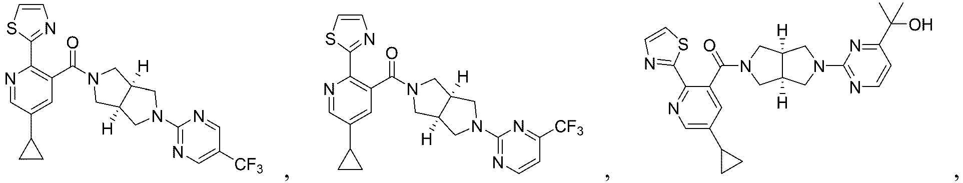

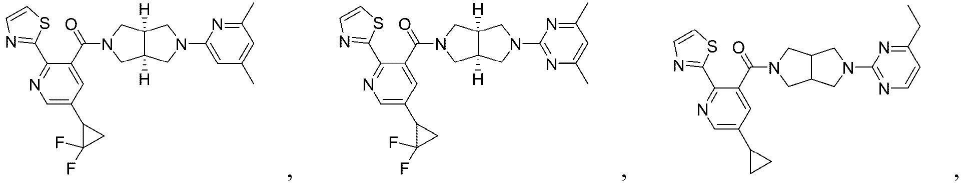

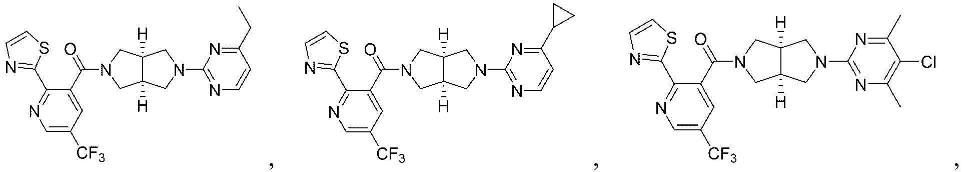

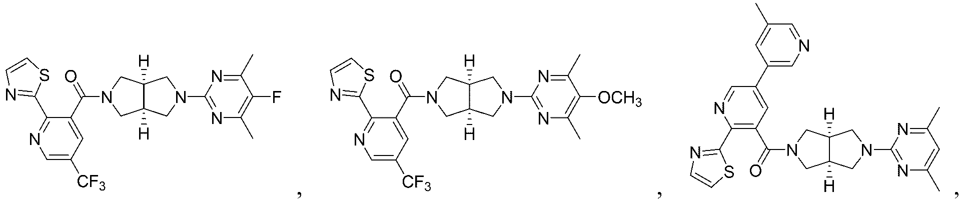

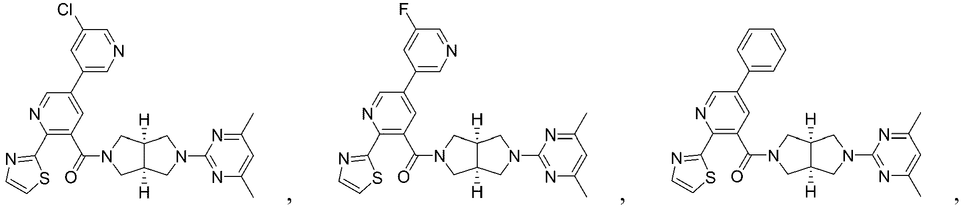

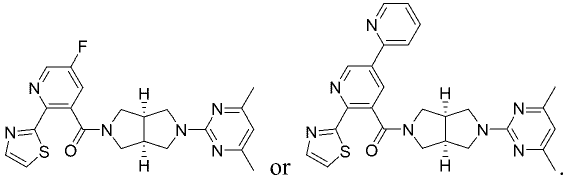

In another aspect, the present application provides a pharmaceutical composition, which includes the compound, the stereoisomer thereof or the pharmaceutically acceptable salt thereof above, and one or more pharmaceutically acceptable carriers or excipients.

In another aspect, the present application provides a use of the compound, the stereoisomer thereof or the pharmaceutically acceptable salt thereof above, or the pharmaceutical composition above, in manufacture of an orexin receptor antagonist; preferably in manufacture of an OX2 receptor antagonist.

In another aspect, the present application provides a use of the compound, the stereoisomer thereof or the pharmaceutically acceptable salt thereof above, or the pharmaceutical composition above, in manufacture of a drug for treating a nervous system disease, wherein the nervous system disease is preferably insomnia, depression or anxiety, or drug addiction, and more preferably major depressive disorder, primary and secondary insomnia, or depression accompanied by insomnia.

In another aspect, the present application provides a use of the compound, the stereoisomer thereof or the pharmaceutically acceptable salt thereof above, or the pharmaceutical composition above, in manufacture of a drug for treating an orexin receptor-related disease, wherein the disease is preferably a nervous system disease.

In some embodiments, the nervous system disease is insomnia, depression or anxiety, or drug addiction, and preferably major depressive disorder, primary and secondary insomnia, or depression accompanied by insomnia.

In another aspect, the present invention provides a preparation method for a compound as shown in formula I, which includes the following steps: 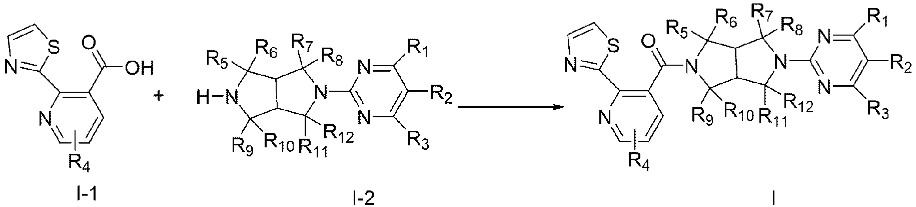
the preparation method further includes the following steps: 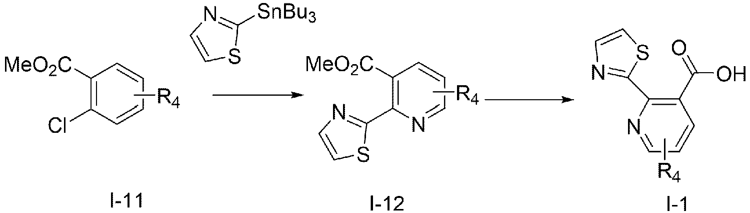
the preparation method further includes the following steps:

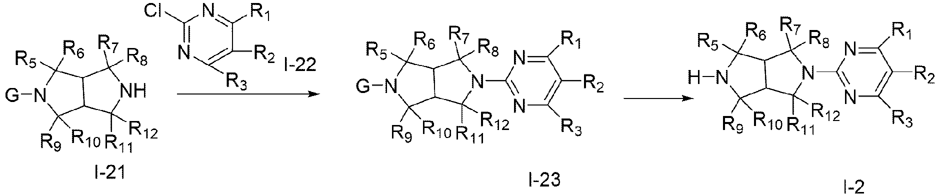

Alternatively, the present invention provides an intermediate having the following structure: 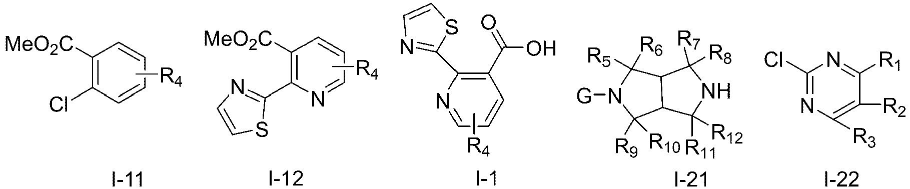 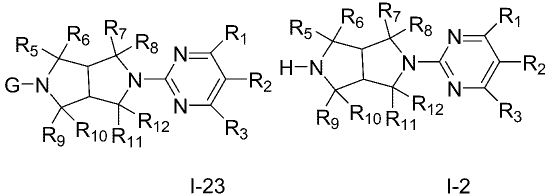 wherein, each substituent is as defined above.

In another aspect, the present invention provides a preparation method for a compound as shown in formula II, which includes the following steps: 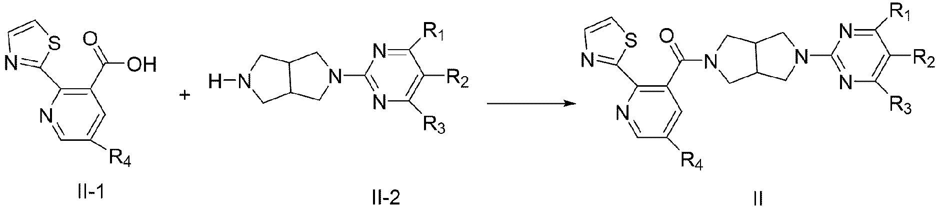
the preparation method further includes the following steps: 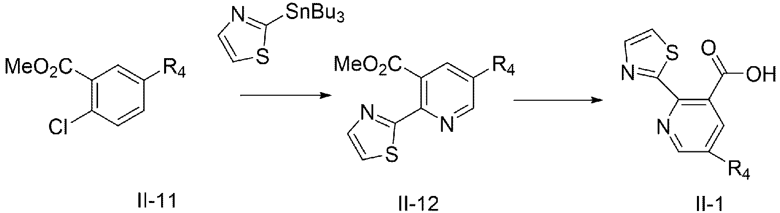
the preparation method further includes the following steps:

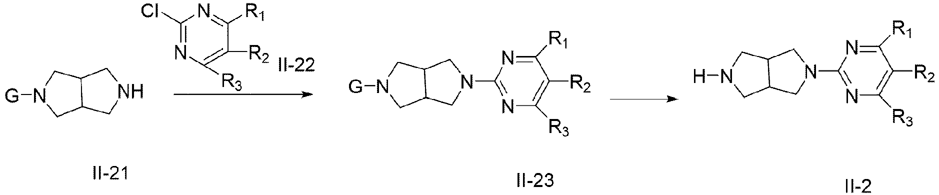

Alternatively, the present invention provides the following intermediate: 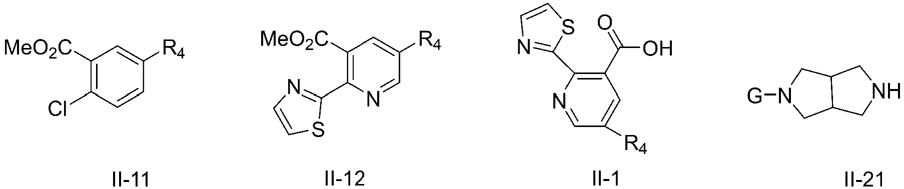 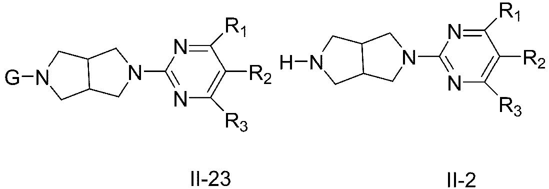 wherein, each substituent is as defined above.

### Descriptions of terms

Unless stated to the contrary, the terms used in the specification and the claims have the following meanings.

In the present invention, alkyl refers to a saturated aliphatic hydrocarbon group, which is a straight or branched chain group containing 1 to 20 carbon atoms, and is preferably alkyl containing 1 to 8 carbon atoms, more preferably alkyl containing 1 to 6 carbon atoms, and most preferably alkyl containing1 to 3 carbon atoms. Non-limiting examples thereof include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, and the like.

More preferably, the alkyl is low alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like.

The alkyl may be substituted or unsubstituted. When substituted, the substituent may be attached at any available attachment point, and the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate ester group. In the present invention, preferably, the alkyl is methyl, ethyl, isopropyl, tert-butyl, haloalkyl, deuterated alkyl and alkoxy-substituted alkyl, and hydroxy-substituted alkyl; and the hydroxy-substituted alkyl may be 2-hydroxyisopropyl or 1-hydroxyethyl.

In the present invention, cycloalkyl refers to a saturated or partially unsaturated monocyclic or polycyclic cyclic hydrocarbon substituent, and a cycloalkyl ring contains 3 to 20 carbon atoms, preferably 3 to 12 carbon atoms, more preferably 3 to 8 carbon atoms, and further preferably 3 to 6 carbon atoms.

Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; and polycyclic cycloalkyl includes spirocycloalkyl, fused cycloalkyl and bridged cycloalkyl.

In the present invention, spirocycloalkyl refers to a 5- to 20-membered polycyclic group in which single rings share one carbon atom (referred to as a spiro atom), and the rings may contain one or more double bonds, but none of the rings has a fully conjugated π-electron system. Preferably, the spirocycloalkyl is 6- to 14-membered, more preferably 7- to 10-membered. The spirocycloalkyl may be classified as monospirocycloalkyl, dispirocycloalkyl or polyspirocycloalkyl according to the number of spiro atoms shared between the rings, and is preferably the monospirocycloalkyl and the dispirocycloalkyl. More preferably, the spirocycloalkyl is 4/4-membered, 4/5-membered, 4/6-membered, 5/5-membered or 5/6-membered monospirocycloalkyl. Non-limiting examples of the spirocycloalkyl include: 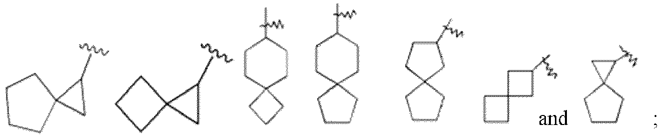 the spirocycloalkyl further includes spirocycloalkyl in which monospirocycloalkyl shares a spiro atom with heterocycloalkyl. Non-limiting examples thereof include:

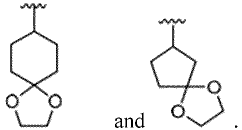

In the present invention, fused cycloalkyl refers to a 5- to 20-membered all-carbon polycyclic group in which each ring in the system shares an adjacent pair of carbon atoms with another ring in the system, wherein one or more rings may contain one or more double bonds, but none of the rings has a fully conjugated π-electron system. Preferably, the fused cycloalkyl is 6- to 14-membered, more preferably 7- to 10-membered. The fused cycloalkyl may be classified as bicyclic, tricyclic, tetracyclic or polycyclic fused cycloalkyl according to the number of constituent rings, is preferably the bicyclic or tricyclic fused cycloalkyl, and is more preferably 5/5-membered or 5/6-membered bicycloalkyl. Non-limiting examples of the fused cycloalkyl include:

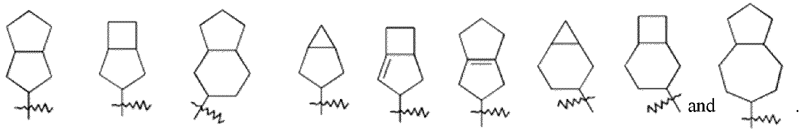

In the present invention, bridged cycloalkyl refers to a 5- to 20-membered all-carbon polycyclic group in which any two rings share two carbon atoms that are not directly connected to each other, and the rings may contain one or more double bonds, but none of the rings has a fully conjugated π-electron system. Preferably, the bridged cycloalkyl is 6- to 14-membered, more preferably 7- to 10-membered. The bridged cycloalkyl may be classified as bicyclic, tricyclic, tetracyclic or polycyclic bridged cycloalkyl according to the number of constituent rings, is preferably the bicyclic, tricyclic or tetracyclic bridged cycloalkyl, and is more preferably the bicyclic or tricyclic bridged cycloalkyl. Non-limiting examples of the bridged cycloalkyl include:

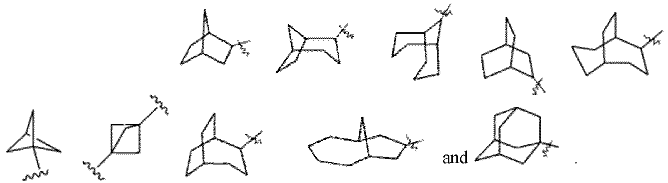

The cycloalkyl ring may be fused to an aryl, heteroaryl or heterocycloalkyl ring, wherein a ring connected to a parent structure is the cycloalkyl ring. Non-limiting examples thereof include indanyl, tetrahydronaphthyl, benzocycloheptanyl, and the like. The cycloalkyl may be optionally substituted or unsubstituted. When substituted, the substituent is preferably one or more of the following groups, which are independently alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate ester group.

In the present invention, heterocyclyl refers to a saturated or partially unsaturated monocyclic or polycyclic heterocyclic group containing 3 to 20 ring atoms, wherein one or more ring atoms are heteroatoms selected from nitrogen, oxygen or S(O)ₘ (wherein m is an integer from 0 to 2), excluding a ring moiety containing -O-O-, -O-S- or -S-S-, and the remaining ring atoms are carbon. Preferably, the heterocyclyl contains 3 to 12 ring atoms, wherein 1 to 4 of the ring atoms are heteroatoms; more preferably, the heterocyclyl contains 3 to 10 ring atoms; and further preferably, the heterocyclyl contains 3 to 8 ring atoms. Non-limiting examples of monocyclic heterocyclyl include pyrrolidinyl, pyrrolidinonyl, piperidin-2-onyl, 3,4-dihydropyridin-2(1H)-onyl, 4,5-dihydropyridazin-3(2H)-onyl, azetidinyl, oxetanyl, oxanyl, imidazolidinyl, tetrahydrofuranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, 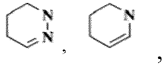 and the like; preferably pyrrolidinyl, pyrrolidinonyl, piperidin-2-onyl, 3,4-dihydropyridin-2(1H)-onyl, 4,5-dihydropyridazin-3(2H)-onyl, azetidinyl, oxetanyl, dihydropyrrolyl, tetrahydrofuranyl, pyrazolidinyl, morpholinyl, 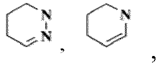 piperazinyl and pyranyl; more preferably dihydropyrrolyl, pyrrolidinyl, pyrrolidinonyl, piperidin-2-onyl, 3,4-dihydropyridin-2(1H)-onyl, 4,5-dihydropyridazin-3(2H)-onyl, azetidinyl, oxetanyl, oxanyl, morpholinyl, piperidinyl, piperazinyl, 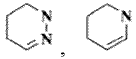 and pyranyl. Polycyclic heterocyclyl includes spiro, fused and bridged heterocyclyl, wherein the spiro, fused and bridged heterocyclyl involved is optionally connected to other groups via a single bond, or further fused via any two or more ring atoms on the ring to other cycloalkyl, heterocyclyl, aryl and heteroaryl.

In the present invention, spiroheterocyclyl refers to a 5- to 20-membered polycyclic heterocyclic group in which single rings share one atom (referred to as a spiro atom), wherein one or more ring atoms are heteroatoms selected from nitrogen, oxygen or S(O)ₘ (wherein m is an integer from 0 to 2), and the remaining ring atoms are carbon. The rings may contain one or more double bonds, but none of the rings has a fully conjugated π-electron system. Preferably, the spiroheterocyclyl is 6- to 14-membered, more preferably 7- to 10-membered. The spiroheterocyclyl may be classified as monospiroheterocyclyl, dispiroheterocyclyl or polyspiroheterocyclyl according to the number of spiro atoms shared between the rings, is preferably monospiroheterocyclyl or dispiroheterocyclyl, and is more preferably 4/4-membered, 4/5-membered, 4/6-membered, 5/5-membered or 5/6-membered monospiroheterocyclyl. Non-limiting examples of the spiroheterocyclyl include:

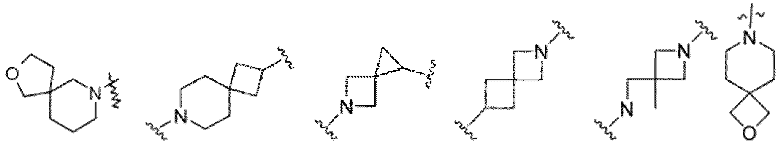

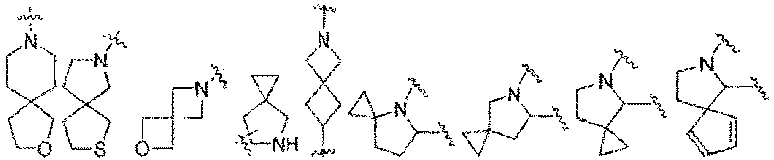

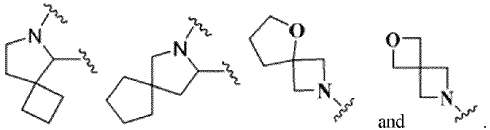

In the present invention, fused heterocyclyl refers to a 5- to 20-membered polycyclic heterocyclic group in which each ring in the system shares an adjacent pair of atoms with another ring in the system, wherein one or more rings may contain one or more double bonds, but none of the rings has a fully conjugated π-electron system, wherein one or more ring atoms are heteroatoms selected from nitrogen, oxygen or S(O)ₘ (wherein m is an integer from 0 to 2), and the remaining ring atoms are carbon. Preferably, the fused heterocyclyl is 6- to 14-membered, more preferably 7- to 10-membered. The fused heterocyclyl may be classified as bicyclic, tricyclic, tetracyclic or polycyclic fused heterocyclyl according to the number of constituent rings, is preferably the bicyclic or tricyclic fused heterocyclyl, and is more preferably 5/5-membered or 5/6-membered bicyclic fused heterocyclyl. Non-limiting examples of the fused heterocyclyl include:

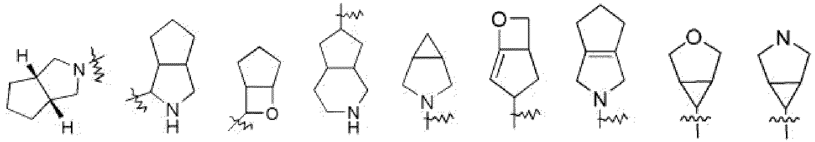

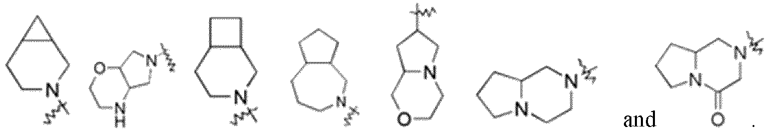

In the present invention, bridged heterocyclyl refers to a 5- to 14-membered polycyclic heterocyclic group in which any two rings share two atoms that are not directly connected to each other, which may contain one or more double bonds, but none of the rings has a fully conjugated π-electron system, wherein one or more ring atoms are heteroatoms selected from nitrogen, oxygen or S(O)ₘ (wherein m is an integer from 0 to 2), and the remaining ring atoms are carbon. Preferably, the bridged heterocyclyl is 6- to 14-membered, more preferably 7- to 10-membered. The bridged heterocyclyl may be classified as bicyclic, tricyclic, tetracyclic or polycyclic bridged heterocyclyl according to the number of constituent rings, is preferably the bicyclic, tricyclic or tetracyclic bridged heterocyclyl, and is more preferably bicyclic or tricyclic bridged heterocyclyl. Non-limiting examples of the bridged heterocyclyl include:

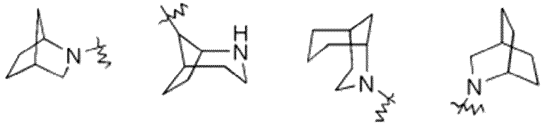

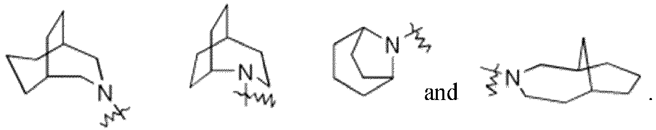

The heterocyclyl ring may be fused to an aryl, heteroaryl or cycloalkyl ring, wherein a ring connected to a parent structure is the heterocyclyl ring. Non-limiting examples thereof include:

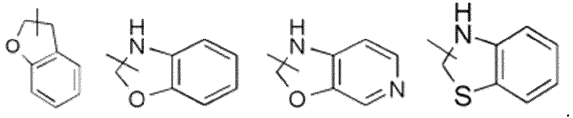

The heterocyclyl may be optionally substituted or unsubstituted. When substituted, the substituent is preferably one or more of the following groups, which are independently alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate ester group.

In the present invention, aryl refers to a 6- to 14-membered all-carbon monocyclic or fused polycyclic (referring to rings that share adjacent pairs of carbon atoms) group having a conjugated π-electron system, and is preferably 6- to 10-membered, more preferably 6- to 8-membered, such as phenyl and naphthyl, preferably the phenyl. The aryl ring may be fused to a heteroaryl, heterocyclyl or cycloalkyl ring, wherein a ring connected to a parent structure is the aryl ring. Non-limiting examples thereof include:

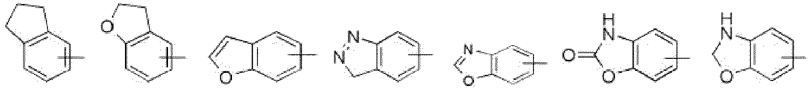

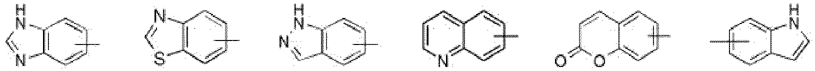

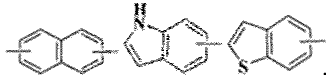

The aryl may be substituted or unsubstituted. When substituted, the substituent is preferably one or more of the following groups, which are independently alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate ester group.

In the present invention, heteroaryl refers to a heteroaromatic system containing 1 to 4 heteroatoms and 5 to 14 ring atoms, wherein the heteroatoms are selected from oxygen, sulfur and nitrogen. The heteroaryl is preferably 5- to 10-membered, more preferably 5- to 8-membered, and most preferably 5- or 6-membered, for example, pyrazinyl, pyridazinyl, imidazolyl, furanyl, thienyl, thiazolyl, pyrazolyl, oxazolyl, isoxazolyl, pyrrolyl, triazolyl, tetrazolyl, pyridinyl, pyrimidinyl, thiadiazolyl, oxadiazolyl, pyrazinyl, and the like, preferably pyrimidinyl, pyrazolyl, oxazolyl, isoxazolyl, oxadiazolyl and pyridinyl. The heteroaryl ring may be fused to an aryl, heterocyclyl or cycloalkyl ring, wherein a ring connected to a parent structure is the heteroaryl ring. Non-limiting examples thereof include:

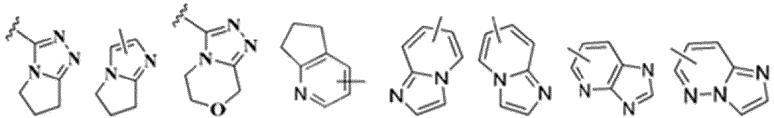

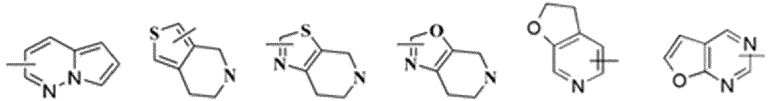

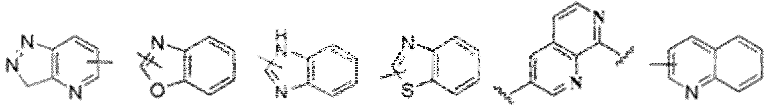

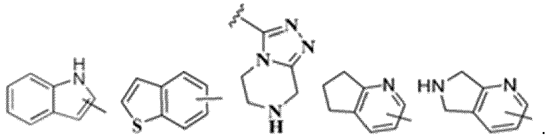

The heteroaryl may be optionally substituted or unsubstituted. When substituted, the substituent is preferably one or more of the following groups, which are independently alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate ester group.

In the present invention, alkoxy refers to -O-(alkyl) and -O-(unsubstituted cycloalkyl), wherein the alkyl is as defined above, and is preferably alkyl containing 1 to 8 carbon atoms, more preferably alkyl containing 1 to 6 carbon atoms, and most preferably alkyl containing 1 to 3 carbon atoms. Non-limiting examples of the alkoxy include: methoxy, ethoxy, propoxy, butoxy, cyclopropoxy, cyclobutoxy, cyclopentyloxy and cyclohexyloxy. The alkoxy may be optionally substituted or unsubstituted. When substituted, the substituent is preferably one or more of the following groups, which are independently alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate ester group.

Non-limiting examples of the alkoxy further include: propan-2-yloxy, and the like.

In the present invention, haloalkyl refers to alkyl substituted with one or more halogens, wherein the alkyl is as defined above. Non-limiting examples of the haloalkyl include: trifluoromethyl and trifluoroethyl.

Non-limiting examples of the haloalkyl further include: difluoromethyl, 1,1,2,2-tetrafluoroethyl, perfluoroethyl, and the like.

In the present invention, haloalkoxy refers to alkoxy substituted with one or more halogens, wherein the alkoxy is as defined above.

The haloalkoxy may be perhalogenated or partially halogenated, and the number of halogen substituents may be 1, 2, 3, 4, 5, 6, 7, 8, 9, 10, or the like; and the halogen is preferably F, Cl, Br or I, for example, trifluoromethoxy, difluoromethoxy, 1,1,2,2-tetrafluoroethoxy, perfluoroethoxy, and the like.

In the present invention, hydroxyalkyl refers to alkyl substituted with hydroxy, wherein the alkyl is as defined above.

In the present invention, alkenyl refers to chain alkenyl, which is also known as olefinyl, and is preferably alkenyl containing 2 to 8 carbon atoms, more preferably alkenyl containing 2 to 6 carbon atoms, further preferably alkenyl containing 2 to 4 carbon atoms, and most preferably alkenyl containing 2 to 3 carbon atoms. Non-limiting examples of the alkenyl include: vinyl and propenyl. The alkenyl may be further substituted with other relevant groups, such as alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate ester group.

In the present invention, alkynyl refers to chain alkynyl, which is also known as alkyne group, and refers to an unsaturated hydrocarbon group containing -C≡C-; and is preferably alkynyl containing 2 to 8 carbon atoms, more preferably alkynyl containing 2 to 6 carbon atoms, further preferably alkynyl containing 2 to 4 carbon atoms, and most preferably alkynyl containing 2 to 3 carbon atoms. The alkynyl may be further substituted with other relevant groups, such as alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate ester group.

In the present invention, hydroxy refers to an -OH group.

In the present invention, halogen refers to fluorine, chlorine, bromine or iodine.

In the present invention, amino refers to -NH₂.

In the present invention, cyano refers to -CN.

In the present invention, nitro refers to -NO₂.

In the present invention, carboxyl refers to -C(O)OH.

Any hydrogen atom in the present invention may be substituted with an isotope deuterium thereof, and any hydrogen atom in the example compounds of the present invention may also be substituted with a deuterium atom.

In the present invention, unless otherwise specified, according to the techniques described in the present invention or the techniques known in the art disclosed herein, substituents on a cyclic group (such as aryl, heteroaryl, fused ring, saturated or unsaturated cycloalkyl, or heterocycloalkyl) are intended to mean that the cyclic group is substituted with one or more substituents at any ring position of the cyclic group or on any ring of a fused ring group, and when there are multiple substituents, each substituent may be the same or different. For example, a cyclic group 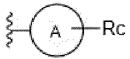 means that any substitutable position on a ring A is substituted with one or more Rc. For another example, a cyclic group 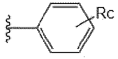 includes the following non-limiting examples: 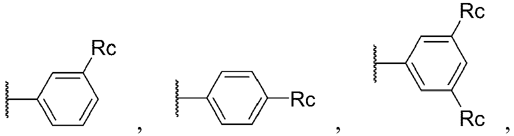 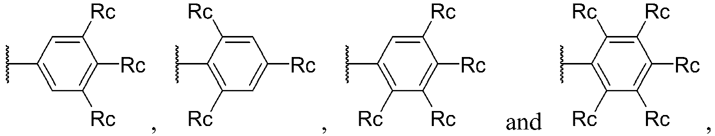 and when there is more than one Rc substituent on a phenyl ring, each Rc may be the same or different. In some embodiments, a cyclic group 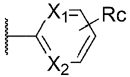 includes the following non-limiting examples: 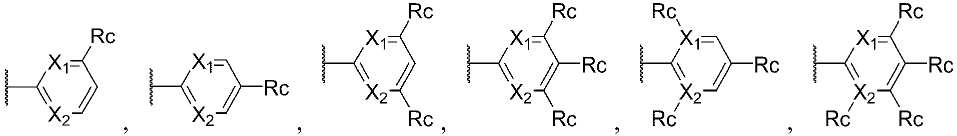 and 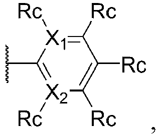 and when there is more than one Rc substituent on a ring, each Rc may be the same or different.

In the present invention, "optional" or "optionally" means that the event or circumstance described thereafter may but need not occur. For example, "a heterocyclic group optionally substituted with alkyl" means that the alkyl may but need not exist, and the description includes both the case where the heterocyclic group is substituted with the alkyl and the case where the heterocyclic group is not substituted with the alkyl.

In the present invention, "substituted" means that one or more hydrogen atoms, preferably at most 5 hydrogen atoms, and more preferably 1 to 3 hydrogen atoms, in a group, are independently substituted with the corresponding number of substituents. It goes without saying that the substituents are only in their possible chemical positions, and those skilled in the art can determine (by experiment or theory) possible or impossible substitutions without excessive efforts. For example, amino or hydroxy with free hydrogen may be unstable when bonded to a carbon atom with an unsaturated (such as olefinic) bond.

The compounds of the present patent application include isotopic derivatives thereof. The term "isotopic derivative" refers to a compound that differs in structure only in the presence of one or more isotopically enriched atoms. For example, compounds having a structure of the present patent application, in which hydrogen is substituted with "deuterium" or "tritium", or fluorine is substituted with an 18F-fluorine label (18F isotope), or a carbon atom is substituted with 11C-, 13C- or 14C-enriched carbon (11C-, 13C- or 14C-carbon label; and 11C-, 13C- or 14C-isotope), are within the scope of the present patent application. Such compounds may be used as, for example, analytical tools or probes in biological assays, or in-vivo diagnostic imaging tracers for diseases, or tracers for pharmacodynamic, pharmacokinetic or receptor studies. Various deuterated forms of compounds of the present patent application mean that each available hydrogen atom attached to a carbon atom may be independently substituted with a deuterium atom. Those skilled in the art can refer to relevant literature to synthesize the deuterated forms of compounds. When preparing the deuterated forms of compounds, commercially available deuterated starting materials may be used, or the compounds may be synthesized by conventional techniques with deuterated reagents, which include, but are not limited to, deuterated borane, a solution of trideuterated borane in tetrahydrofuran, lithium aluminum deuteride, deuterated iodoethane, deuterated iodomethane, and the like. Deuterated compounds may generally retain activity equivalent to that of non-deuterated compounds, and when deuterated at certain specific sites, the compounds may achieve better metabolic stability, thereby obtaining certain therapeutic advantages.

The compounds of the present patent application may exist in specific stereoisomeric forms. The term "stereoisomer" refers to isomers that are identical in structure but different in spatial arrangement of atoms, including cis and trans (or Z and E) isomers, (-)- and (+)-isomers, (R)- and (S)-enantiomers, diastereomers, (D)- and (L)-isomers, tautomers, atropisomers, conformers and mixtures thereof (such as racemates and mixtures of diastereomers). Substituents in the compounds of the present patent application may contain additional asymmetric atoms. All these stereoisomers and mixtures thereof are included in the scope of the present patent application. Optically active (-)- and (+)-isomers, (R)- and (S)-enantiomers, and (D)- and (L)-isomers may be prepared by chiral synthesis, chiral reagents or other conventional techniques. One isomer of a certain compound of the present patent application may be prepared by asymmetric synthesis or chiral auxiliaries, or when the molecule contains a basic functional group (such as amino) or an acidic functional group (such as carboxyl), a diastereomer salt is formed with an appropriate optically active acid or base, and then the diastereomer is resolved by a conventional method known in the art to obtain a pure isomer. In addition, the separation of enantiomer and diastereomer is usually finished by chromatography.

The structures of the compounds of the present application may be confirmed by conventional methods well known to those skilled in the art. For example, when an absolute configuration of compound is involved in the present application, the absolute configuration may be determined by conventional techniques in the art. For example, by single crystal X-ray diffraction (SXRD), diffraction intensity data of a cultured single crystal are collected using a Bruker D8 venture diffractometer, with CuKα radiation as a light source, and φ/ scanning as a scan mode. After collecting relevant data, the crystal structure is further analyzed by a direct method (Shelxs97) to determine the absolute configuration.

In the chemical structures of the compounds of the present patent application, the bond "/" denotes an unspecified configuration, which means that, if there is the chiral isomer in the chemical structure, the bond "/" may be "R" or "S", or may contain "R" and "S" configurations at the same time. Unless otherwise specified, in the present invention, a solid wedge bond ( 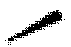 ) and a dashed wedge bond ( 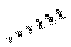 ) indicate cis-trans isomerism at a stereocenter. For example, the dashed wedge bond ( 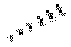 ) in 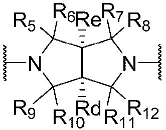 indicates that two substituents Rd and Re are cis-form, which means that Rd and Re are on the same side of a plane.

The compounds of the present patent application may exist in different tautomeric forms, and all such forms are included within the scope of the present patent application. The term "tautomer" or "tautomeric form" refers to structural isomers that exist in equilibrium and are readily interconverted from one isomeric form to another. It includes all possible tautomers, which refers to existing either as a single isomer or as a mixture of the tautomers in any proportion. Non-limiting examples thereof include: keto-enol, imine-enamine, lactam-lactim, and the like. All tautomeric forms are within the scope of the present patent application, and the naming of compounds does not exclude any tautomer.

In the present invention, the pharmaceutical composition refers to a mixture containing one or more compounds described herein or their physiologically/pharmaceutically acceptable salts or prodrugs and other chemical components, and other components such as physiologically/pharmaceutically acceptable carriers and excipients. The purpose of the pharmaceutical composition is to promote administration to an organism, which is beneficial for the absorption of active ingredients, thereby exerting biological activity.

In the present invention, the pharmaceutically acceptable salt refers to a salt of the compound of the present invention, and the salt is safe and effective for use in mammals, and possesses the expected biological activity.

Aiming at a drug or pharmacologically active agent, the term "therapeutically effective amount" refers to an amount of the drug or agent sufficient to achieve, or at least partially achieve, the desired effect. The determination of the therapeutically effective amount varies from individual to individual, which depends on the age and general condition of the recipient, and also depends on a particular active substance. An appropriate therapeutically effective amount in an individual case may be determined by those skilled in the art through routine testing.

The term "pharmaceutically acceptable" as used herein means that those compounds, materials, compositions and/or dosage forms fall within the scope of sound medical judgment, are suitable for contact with the tissues of a patient without excessive toxicity, irritation, allergic response or other problems or complications, have a reasonable benefit/risk ratio, and are effective for intended use.

The singular forms "a", "an" and "the" used herein include plural references, and vice versa, unless otherwise clearly indicated in the context.

In the present invention, "multiple" refers to two or more, for example, an integer number of 2, 3, 4, 5, 6, 7, 8, or the like.

When the term "about" is applied to parameters such as pH, concentration and temperature, the parameters may vary by ±10%, and more preferably within ±5% in some cases. Those skilled in the art will understand that, when the parameters are not critical, numerical values are generally given for illustrative purposes only and not for limitation.

### DESCRIPTION OF THE DRAWINGS

FIG. 1 shows changes in NREM sleep latency in male SD rats of Example 30.
FIG. 2 shows changes in REM sleep latency in male SD rats of Example 30.
FIG. 3 shows changes in wake duration in SD rats within the 2-hour after administration of Example 30.
FIG. 4 shows changes in wake duration in SD rats within the 6-hour after administration of Example 30.
FIG. 5 shows changes in NREM duration (non-rapid-eye-movement sleep duration) in SD rats within the 2-hour after administration of Example 30.
FIG. 6 shows changes in NREM duration (non-rapid-eye-movement sleep duration) in SD rats within the 6-hour after administration of Example 30.
FIG. 7 shows changes in REM duration (rapid-eye-movement sleep duration) in SD rats within the 2-hour after administration of Example 30.
FIG. 8 shows changes in REM duration (rapid-eye-movement sleep duration) in SD rats within the 6-hour after administration of Example 30.
FIG. 9 shows changes in NREM sleep latency in male SD rats of Example 31.
FIG. 10 shows changes in REM sleep latency in male SD rats of Example 31.
FIG. 11 shows changes in wake duration in SD rats within the 2-hour after administration of Example 31.
FIG. 12 shows changes in wake duration in SD rats within the 6-hour after administration of Example 31.
FIG. 13 shows changes in NREM duration (non-rapid-eye-movement sleep duration) in SD rats within the 2-hour after administration of Example 31.
FIG. 14 shows changes in NREM duration (non-rapid-eye-movement sleep duration) in SD rats within the 6-hour after administration of Example 31.
FIG. 15 shows changes in REM duration (rapid-eye-movement sleep duration) in SD rats within the 2-hour after administration of Example 31.
FIG. 16 shows changes in REM duration (rapid-eye-movement sleep duration) in SD rats within the 6-hour after administration of Example 31.
FIG. 17 shows changes in NREM sleep latency in SD rats of Example 35.
FIG. 18 shows changes in wake duration in SD rats within the 6-hour after administration of Example 35.
FIG. 19 shows changes in NREM duration (non-rapid-eye-movement sleep duration) in SD rats within the 6-hour after administration of Example 35.

### DESCRIPTION OF EXEMPLARY EMBODIMENTS

The abbreviations used herein have the following meanings:

| Abbreviation | Meaning |
|---|---|
| TLC | Thin-layer chromatography |
| LC-MS | Liquid chromatography-mass spectrometry |
| DMF | N,N-dimethylformamide |
| DMSO | Dimethyl sulfoxide |
| EA | Ethyl acetate |
| PE | Petroleum ether |
| THF | Tetrahydrofuran |
| DCM | Dichloromethane |
| DIEA | N,N-diisopropylethylamine |
| HATU | 2-(7-azabenzotriazol-1-yl)-N,N,N',N'-tetramethyluronium hexafluorophosphate |
| NMP | N-methylpyrrolidone |
| CF₃-TMS | Trifluoromethyltrimethylsilane |
| Fe(acac)₃ | Iron(III) acetylacetonate |

Unless otherwise specified, the reaction temperature is room temperature (20°C to 30°C).

Structures of compounds described in the following examples are confirmed by H nuclear magnetic resonance spectroscopy (¹H-NMR) or mass spectrometry (MS).

### Example 1 Synthesis of compound KH01

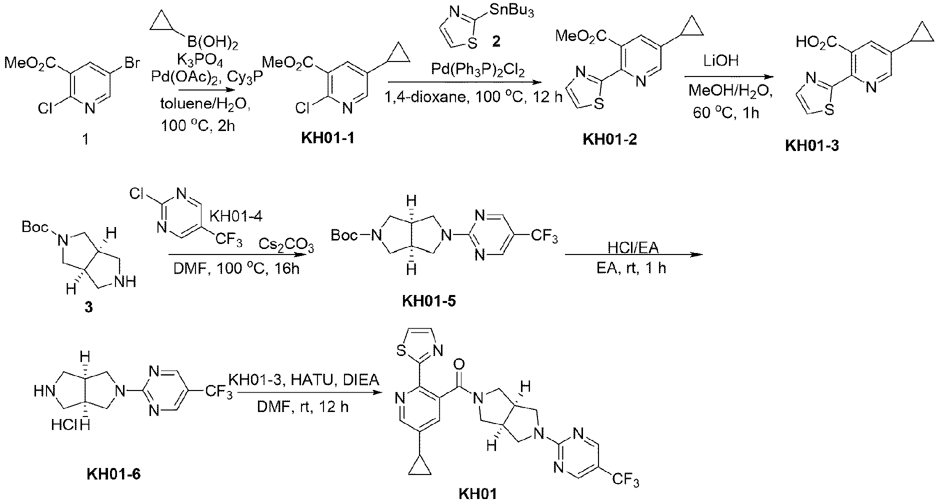

**Compound** KH01-1: Cyclopropylboronic acid (927 mg, 10.78 mmol), palladium acetate (68 mg, 0.30 mmol), potassium phosphate (3.805 g, 17.97 mmol), tricyclohexylphosphine (Cy₃P, 168 mg, 0.599 mmol) and water (1.5 mL) were added to a solution of compound 1 (1.5 g, 5.99 mmol) in toluene (15 mL), and the mixture was allowed to react at 100°C for 2 hours. After the reaction was complete, the reaction solution was cooled to room temperature and then water was added, and ethyl acetate was added for extraction of the organic phase. The organic phase was washed with saturated brine, dried with anhydrous sodium sulfate, and concentrated in vacuum under reduced pressure to obtain a crude product. The crude product was purified by silica gel column chromatography (petroleum ether: ethyl acetate=10: 1) to obtain **compound KH01-1**. LCMS (ESI, *m*/*z*): 212.0[M + 1]⁺. ¹H NMR (400 MHz, DMSO) δ 8.40 (d, *J* = 2.5 Hz, 1H), 7.85 (d, *J* = 2.5 Hz, 1H), 3.87 (s, 3H), 2.04 - 1.96 (m, 1H), 1.05 - 1.02 (m, 2H), 0.82 - 0.80 (m, 2H).

**Compound KH01-2:** Compound 2 (900 mg, 2.34 mmol) and bis(triphenylphosphine)palladium(II) dichloride (155 mg, 0.213 mmol) were added to a solution of compound **KH01-1** (450 mg, 2.13 mmol) in 1,4-dioxane (10 mL), and the mixture was allowed to react at 100°C for 12 hours. After the reaction was complete, the reaction solution was concentrated in vacuum under reduced pressure to obtain a crude product. The crude product was purified by silica gel column chromatography (petroleum ether: ethyl acetate=3: 1) to obtain **compound KH01-2**. LCMS (ESI, *m*/*z*): 261.1 [M + 1]⁺. ¹H NMR (400 MHz, DMSO) δ 8.56 (d, *J* = 2.1 Hz, 1H), 7.94 (d, *J* = 3.0 Hz, 1H), 7.86 (d, *J* = 3.1 Hz, 1H), 7.65 (d, *J* = 2.1 Hz, 1H), 3.79 (s, 3H), 2.12 - 2.05 (m, 1H), 1.10 - 1.07 (m, 2H), 0.89 - 0.87 (m, 2H).

**Compound KH01-3**: Compound **KH01-2** (500 mg, 1.92 mmol) and **LiOH** (138 mg, 5.76 mmol) were dissolved in methanol (5 mL) and water (5 mL), and the mixture was allowed to react at 60°C for 1 hour. After the reaction was complete, 1 M hydrochloric acid (6 mL) was added to quench the reaction, and the mixture was extracted with ethyl acetate (30 mL × 2). The organic phase was washed with brine, dried with anhydrous sodium sulfate, filtered and evaporated to dryness to obtain **compound KH01-3**. LCMS (ESI, *m*/*z*): 247.0 [M + 1]⁺.

**Compound KH01-5**: Compound **3** (500 mg, 2.36 mmol, CAS: 250275-15-1), **KH01-4** (430 mg, 2.36 mmol), and Cs₂CO₃ (844 mg, 2.59 mmol) were dissolved in DMF (5 mL), and the mixture was allowed to react at 100°C for 16 hours. After the reaction was complete, water (50 mL) was added to quench the reaction, and the mixture was extracted with ethyl acetate (20 mL × 2). The organic phase was washed with brine, dried with anhydrous sodium sulfate, filtered and concentrated. The obtained crude product was purified by silica gel column chromatography (petroleum ether: ethyl acetate=5/1-2/1) to obtain **KH01-5**. ¹H NMR (400 MHz, CDCl₃) δ 8.50 (s, 2H), 3.91 - 3.81 (m, 2H), 3.66 - 3.54(m, 4H), 3.36 - 3.26 (m, 2H), 3.06 - 2.96 (m, 2H), 1.45 (s, 9H).

**Compound KH01-6**: a 1 M solution of hydrochloric acid in ethyl acetate (17.6 mL, 17.6 mmol) was added to a solution of compound **KH01-5** (630 mg, 1.76 mmol) in ethyl acetate (5 mL), and the mixture was allowed to react at room temperature for 12 hours. After the reaction was complete, the reaction solution was concentrated under reduced pressure to obtain **KH01-6.** LCMS (ESI, *m*/*z):* 259.0 [M + 1]⁺.

**Compound KH01:** DIEA (790 mg, 6.11 mmol) was added to a solution of compound **KH01-6** (450 mg, 1.53 mmol), **KH01-3** (376 mg, 1.53 mmol) and HATU (700 mg, 1.84 mmol) in DMF (10 mL), and the mixture was allowed to react at room temperature overnight. After the reaction was complete, the reaction solution was purified by preparative reverse-phase chromatography to obtain **compound KH01.** LCMS (ESI, *m*/*z):* 487.0 [M + 1]⁺. ¹H NMR (400 MHz, DMSO-*d₆*) δ 8.74 - 8.64 (m, 2H), 8.48 (d, *J=* 1.9 Hz, 1H), 7.88 - 7.78 (m, 2H), 7.47 (d, *J= 2.1* Hz, 1H), 3.93 - 3.51 (m, 5H), 3.50 - 3.38 (m, 2H), 3.14 - 2.81 (m, 3H), 2.11 - 2.01 (m, 1H), 1.13 - 1.03 (m, 2H), 0.93 - 0.83 (m, 2H).

### Example 2 Synthesis of compound KH02

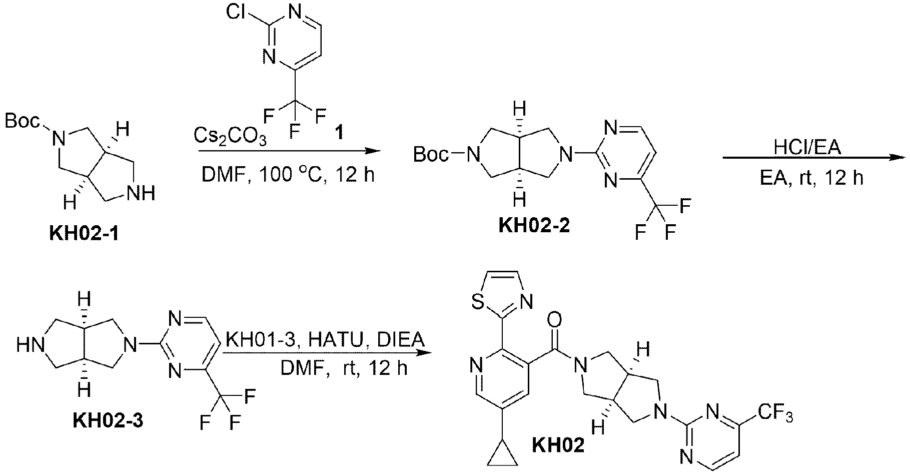

**Compound KH02-2:** Compound 1 (416 mg, 2.29 mmol) and cesium carbonate (680 mg, 2.09 mmol) were added to a solution of compound **KH02-1** (400 mg, 1.89 mmol) in DMF (8 mL), and the mixture was allowed to react at 100°C for 12 hours. After the reaction was complete, the reaction solution was cooled to room temperature and then water was added, and ethyl acetate was added for extraction of the organic phase. The organic phase was washed with saturated brine, dried with anhydrous sodium sulfate, and concentrated in vacuum under reduced pressure to obtain a crude product. The crude product was purified by silica gel column chromatography (petroleum ether: ethyl acetate=10: 1) to obtain **compound KH02-2.** LCMS (ESI, *m*/*z):* 302.9 [M + 1]⁺.

**Compound KH02-3:** a solution of hydrogen chloride in ethyl acetate (17.5 mL) was added to a solution of compound **KH02-2** (700 mg, 1.96 mmol) in EA (35 mL) , and the mixture was allowed to react at room temperature for 12 hours. After the reaction was complete, the reaction solution was concentrated in vacuum under reduced pressure to obtain **compound KH02-3.** LCMS (ESI, *m*/*z):* 259.1 [M + 1]⁺.

**Compound KH02: KH01-3** (190 mg, 0.88 mmol), HATU (422 mg, 1.11 mmol) and DIEA (0.5 mL, 2.96 mmol) were added to a solution of compound **KH02-3** (218 mg, 0.74 mmol) in DMF (4 mL), and the mixture was stirred at room temperature for 12 hours. After the reaction was complete, the reaction solution was purified by preparative reverse-phase chromatography to obtain **compound KH02.** LCMS (ESI, *m*/*z):* 487.1 [M + 1]⁺. ¹H NMR (400 MHz, DMSO-d6) δ 8.66 (d, *J=* 24.8 Hz, 1H), 8.48 (d, *J= 2.0* Hz, 1H), 7.78-7.66 (m, 2H), 7.47 (d, *J=* 1.9 Hz, 1H), 7.01 (d, *J=* 4.7 Hz, 1H), 3.88 - 3.51 (m, 5H), 3.42 (br s, 2H), 3.11 - 2.82 (m, 3H), 2.06 (br s, 1H), 1.07 (d, *J= 7.1* Hz, 2H), 0.88 (s, 2H).

### Example 3 Synthesis of compound KH03

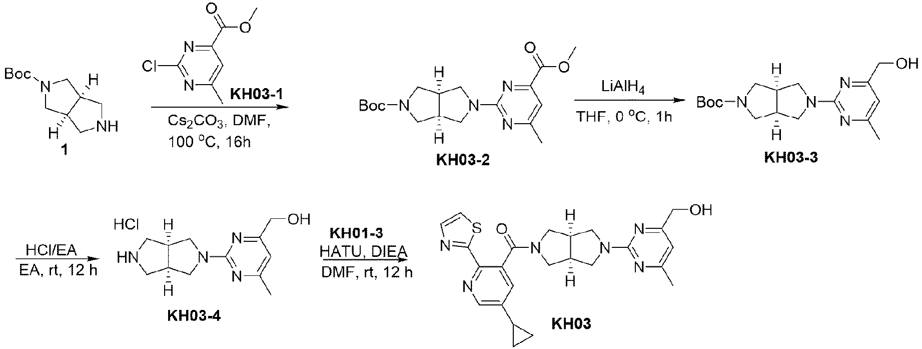

**Compound** KH03-2: Compound 1 (500 mg, 2.36 mmol), KH03-1 (440 mg, 2.36 mmol) and Cs₂CO₃ (844 mg, 2.59 mmol) were added to DMF (5 mL), and the mixture was allowed to react at 100°C for 16 hours. After the reaction was complete, water (50 mL) was added to quench the reaction, and the mixture was extracted with ethyl acetate (50 mL × 2). The organic phase was washed with brine, dried with anhydrous sodium sulfate, filtered and concentrated. The obtained crude product was purified by silica gel column chromatography (petroleum ether: ethyl acetate=5/1-1/1) to obtain KH03-2. ¹H NMR (400 MHz, CDCl₃) δ 7.03 (s, 1H), 3.93 (s, 3H), 3.90 - 3.80 (m, 2H), 3.66 - 3.54 (m, 4H), 3.36 -3.24 (m, 2H), 3.00 - 2.90 (m, 2H), 2.41 (s, 3H), 1.44 (s, 9H).

Compound KH03-3: Compound KH03-2 (400 mg, 1.1 mmol) was dissolved in tetrahydrofuran (5 mL) and cooled to 0°C, LiAlH₄ (83 mg, 2.2 mmol) was added, and the mixture was allowed to react at 0°C for 1 hour. After the reaction was complete, water (0.4 mL) was added to quench the reaction. Anhydrous sodium sulfate was added, and the mixture was stirred for 10 minutes, filtered and evaporated to dryness to obtain compound KH03-3. LCMS (ESI, *m*/*z):* 335.2 [M + 1]⁺.

Compound KH03-4: a 1 M solution of hydrochloric acid in ethyl acetate (7.48 mL, 7.48 mmol) was added to a solution of compound KH03-3 (250 mg, 0.747 mmol) in ethyl acetate (3 mL), and the mixture was allowed to react at room temperature for 12 hours. After the reaction was complete, the reaction solution was filtered to obtain compound KH03-4. LCMS (ESI, *m*/*z):* 235.1 [M + 1]⁺.

Compound KH03: DIEA (215 mg, 1.66 mmol) was added to a solution of compound **KH03-4** (150 mg, 0.554 mmol), KH01-3 (137 mg, 0.554 mmol) and HATU (253 mg, 0.665 mmol) in DMF (5 mL), and the mixture was allowed to react at room temperature overnight. After the reaction was complete, the reaction solution was purified by preparative reverse-phase chromatography to obtain compound KH03. LCMS (ESI, *m*/*z):* 463.3 [M + 1]⁺. ¹H NMR (400 MHz, DMSO-*d₆*) δ 8.47 (d, *J =* 1.5 Hz, 1H), 7.95 - 7.65 (m, 2H), 7.46 (s, 1H), 6.62 (s, 1H), 4.33 (s, 2H), 3.85 - 3.50 (m, 7H), 3.05 - 2.85 (m, 3H), 2.29 (s, 3H), 2.10 - 2.00 (m, 1H), 1.12 - 1.02 (m, 2H), 0.93 - 0.83 (m, 2H).

### Example 4 Synthesis of compound KH04

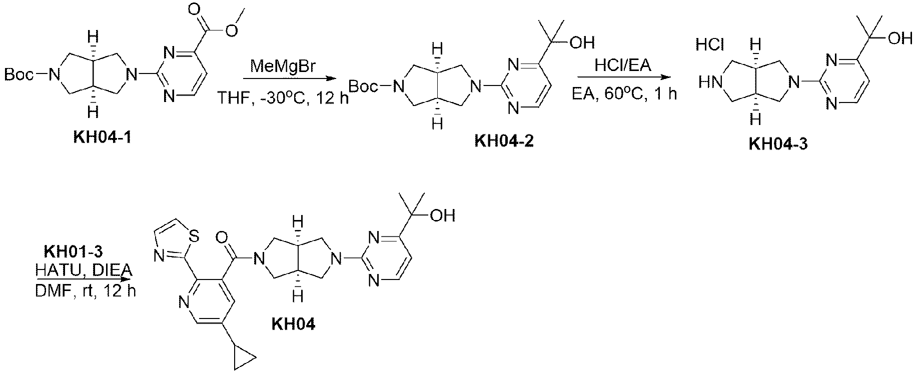

The synthesis method for **compound KH04-1** referred to that for **compound KH03-2.**

**Compound KH04-2:** methylmagnesium bromide (2 mL, 5.74 mmol) was added to a solution of compound **KH04-1** (500 mg, 1.44 mmol) in THF (10 mL) at -30°C, and the mixture was stirred at room temperature overnight. After the reaction was complete, a saturated aqueous ammonium chloride solution was added to the reaction solution to quench the reaction system, and the mixture was extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=3: 1) to obtain **compound KH04-2**. LCMS (ESI, *m*/*z*): 349.1 [M + 1]⁺. ¹H NMR (400 MHz, DMSO-d6) δ 8.29 (d, *J* = 5.1 Hz, 1H), 6.83 (d, *J* = 5.1 Hz, 1H), 5.14 (s, 1H), 3.69 (br s, 2H), 3.52 (br s, 2H), 3.33 (s, 2H), 3.16 (d, *J* = 9.6 Hz, 2H), 2.94 (s, 2H), 1.38 (d, *J* = 5.9 Hz, 15H).

**Compound KH04-3**: a solution of hydrogen chloride in ethyl acetate (12 mL) was added to a solution of compound **KH04-2** (480 mg, 1.38 mmol) in ethyl acetate (24 mL) , and the mixture was allowed to react at 60°C for 1 hour. After the reaction was complete, the reaction solution was concentrated in vacuum under reduced pressure to obtain **compound KH04-3**. LCMS (ESI, *m*/*z*): 249.1 [M + 1]⁺.

**Compound KH04**: **KH01-3** (150 mg, 0.61 mmol), HATU (348 mg, 0.91 mmol) and DIEA (0.5 mL, 2.44 mmol) were added to a solution of compound **KH04-3** (209 mg, 0.73 mmol) in DMF (3 mL), and the mixture was stirred at room temperature for 12 hours. After the reaction was complete, the reaction solution was purified by preparative reverse-phase chromatography to obtain **compound KH04**. LCMS (ESI, *m*/*z*): 477.0 [M + 1]⁺. ¹H NMR (400 MHz, DMSO-d6) δ 8.47 (d, *J* = 2.0 Hz, 1H), 8.30 (s, 1H), 7.89 (m, *J* = 57.8, 54.4 Hz, 2H), 7.47 (d, *J* = 2.1 Hz, 1H), 6.86 (d, *J* = 5.2 Hz, 1H), 3.887-3.58 (m, 4H), 3.34 (m, *J* = 11.6, 4.0 Hz, 3H), 3.10 - 2.85 (m, 3H), 2.06 (br s, 1H), 1.38 (s, 6H), 1.07 (d, *J* = 7.5 Hz, 2H), 0.89 (br s, 2H).

### Example 5 Synthesis of compound KH05

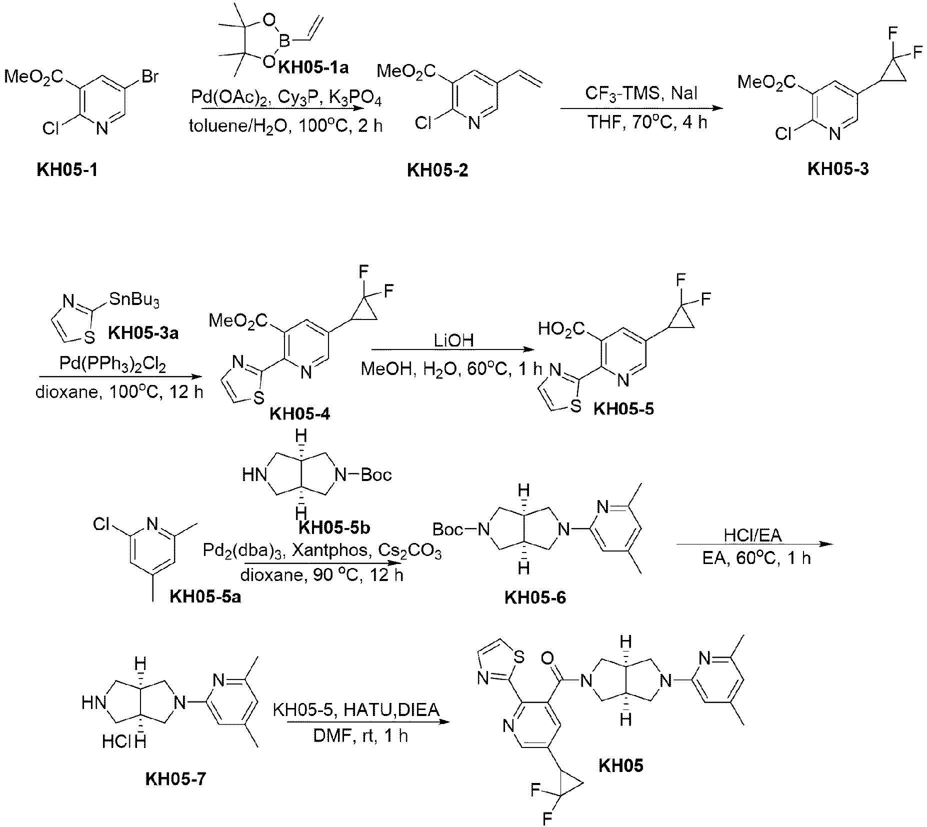

**Compound KH05-2:** compound **KH05-1a** (622 mg, 4.02 mmol), palladium acetate (46 mg, 0.2 mmol), potassium phosphate (2.56 g, 12 mmol), tricyclohexylphosphine (112 mg, 0.4 mmol) and water (1 mL) were added to a solution of compound **KH05-1** (1 g, 4.02 mmol) in toluene (10 mL), and the mixture was allowed to react at 100°C for 2 hours. After the reaction was complete, the reaction solution was cooled to room temperature and then water was added for dilution. The mixture was extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=10: 1) to obtain **compound KH05-2.** LCMS (ESI, *m*/*z):* 198.1 [M + 1]⁺. ¹H NMR (400 MHz, CDCl₃) δ 8.48 (d, *J =* 2.4 Hz, 1H), 8.18 (d, *J =* 2.4 Hz, 1H), 6.69 (m, *J =* 17.6, 11.0 Hz, 1H), 5.89 (d, *J =* 17.6 Hz, 1H), 5.49 (d, *J =* 11.0 Hz, 1H), 3.97 (s, 3H).

**Compound KH05-3:** CF₃-TMS (1.7 mL, 11.1 mmol) and sodium iodide (84 mg, 0.556 mmol) were added to a solution of compound **KH05-2** (550 mg, 2.78 mmol) in THF (5 mL), and the mixture was allowed to react at 70°C for 4 hours. After the reaction was complete, the reaction solution was concentrated in vacuum under reduced pressure to obtain a crude product. The crude product was purified by silica gel column chromatography (petroleum ether: ethyl acetate=10: 1) to obtain **compound KH05-3**. LCMS (ESI, *m*/*z*): 248.0 [M + 1]⁺. ¹H NMR (400 MHz, CDCl₃) δ 8.40 (d, *J* = 2.3 Hz, 1H), 7.98 (d, *J* = 2.3 Hz, 1H), 3.96 (s, 3H), 2.79-2.73 (m, 1H), 2.04-1.92 (m, 1H), 1.77-1.65 (m, 1H).

**Compound KH05-4**: compound **KH05-3a** (600 mg, 1.60 mmol) and bis(triphenylphosphine)palladium(II) dichloride (102 mg, 0.145 mmol) were added to a solution of compound **KH05-3** (360 mg, 1.45 mmol) in 1,4-dioxane (8 mL) , and the mixture was allowed to react at 100°C for 12 hours. After the reaction was complete, the reaction solution was concentrated in vacuum under reduced pressure to obtain a crude product. The crude product was purified by silica gel column chromatography (petroleum ether: ethyl acetate=10: 1) to obtain **compound KH05-4**. LCMS (ESI, *m*/*z*): 297.0 [M + 1]⁺. ¹H NMR (400 MHz, DMSO) δ 8.72 (d, *J* = 2.1 Hz, 1H), 7.98 (d, *J* = 3.2 Hz, 1H), 7.94 (d, *J* = 2.0 Hz, 1H), 7.92 (d, *J* = 3.2 Hz, 1H), 3.81 (s, 3H), 3.26-3.19 (m, 1H), 2.36-2.28 (m, 1H), 2.19-2.08 (m, 1H).

**Compound KH05-5**: lithium hydroxide monohydrate (143 mg, 3.4 mmol) and water (5 mL) were added to a solution of compound **KH05-4** (336 mg, 1.14 mmol) in methanol (10 mL), and the mixture was stirred at 60°C for 1 hour. After the reaction was complete, pH was adjusted to be 5 with dilute hydrochloric acid. The mixture was extracted with ethyl acetate, washed with saturated brine, dried with anhydrous sodium sulfate, and concentrated in vacuum under reduced pressure to obtain **compound KH05-5**. LCMS (ESI, *m*/*z*): 282.9 [M + 1]⁺. ¹H NMR (400 MHz, DMSO) δ 13.46 (s, 1H), 8.67 (d, *J* = 2.0 Hz, 1H), 7.97 (d, *J* = 3.2 Hz, 1H), 7.91 (d, *J* = 3.2 Hz, 1H), 7.90 (d, *J* = 2.0 Hz, 1H), 3.24-3.17 (m, 1H), 2.34-2.28 (m, 1H), 2.17 - 2.07 (m, 1H).

**Compound KH05-6**: compound **KH05-5b** (1.125 g, 5.30 mmol), Pd₂(dba)₃ (324 mg, 10.6 mmol), Xantphos (205 mg, 0.353 mmol) and cesium carbonate (3.737 g, 0.353 mmol) were added to a solution of **compound KH05-5a** (500 mg, 3.53 mmol) in 1,4-dioxane (17 mL), and the mixture was allowed to react at 90°C overnight. After the reaction was complete, the reaction solution was poured into water, extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=10: 1) to obtain **compound KH05-6**. LCMS (ESI, *m*/*z*): 318.1 [M + 1]⁺. ¹H NMR (400 MHz, DMSO) δ 6.27 (s, 1H), 6.05 (s, 1H), 3.52 (br s, 4H), 3.24 (br s, 2H), 3.13 (d, *J =* 9.9 Hz, 2H), 2.93 (s, 2H), 2.23 (s, 3H), 2.15 (s, 3H), 1.38 (s, 9H).

**Compound KH05-7:** a solution of hydrogen chloride in ethyl acetate (16 mL) was added to a solution of compound **KH05-6** (643 mg, 2.03 mmol) in ethyl acetate (32 mL), and the mixture was allowed to react at 60°C for 1 hour. After the reaction was complete, the reaction solution was concentrated in vacuum under reduced pressure to obtain **compound KH05-7.** LCMS (ESI, *m*/*z):* 218.2 [M + 1]⁺.

**Compound KH05: KH05-7** (153 mg, 0.6 mmol), HATU (285 mg, 0.75 mmol) and DIEA (0.34 mL, 2.0 mmol) were added to a solution of compound **KH05-5** (138 mg, 0.5 mmol) in DMF (3 mL), and the mixture was stirred at room temperature for 1 hour. After the reaction was complete, the reaction solution was purified by preparative reverse-phase chromatography to obtain **compound KH05.** LCMS (ESI, m/z): 482.0 [M + 1]⁺. ¹H NMR (400 MHz, DMSO) δ 8.62 (s, 1H), 7.79-7.65 (m, 3H), 6.27 (s, 1H), 6.04 (s, 1H), 3.75-3.58 (m, 5H), 3.25 - 3.11 (m, 3H), 3.07-2.89 (m, 3H), 2.23 (s, 4H), 2.21-2.07 (m, 4H).

### Example 6 Synthesis of compound KH06

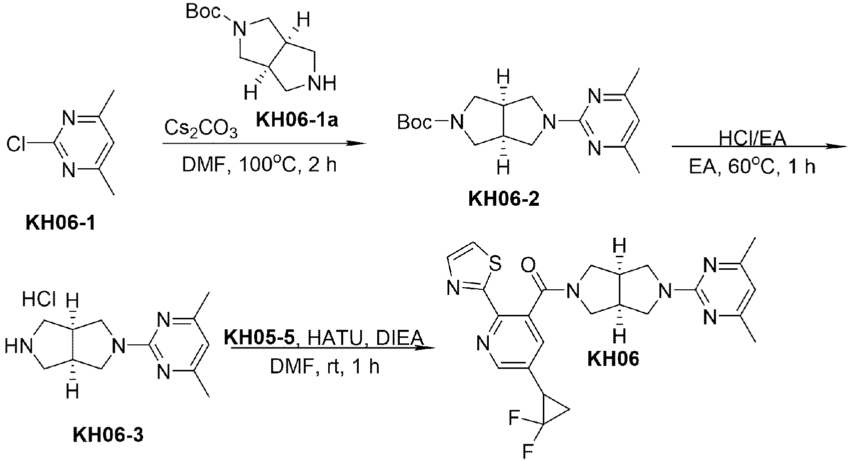

**Compound** KH06-2: compound KH06-1a (494 mg, 2.32 mmol) and cesium carbonate (826 mg, 2.53 mmol) were added to a solution of compound KH06-1 (300 mg, 2.10 mmol) in DMF (6 mL), and the mixture was allowed to react at 100°C for 2 hours. After the reaction was complete, the reaction system was diluted with water, extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=3: 1) to obtain compound **KH06-2.** LCMS (ESI, *m*/*z):* 319.1 [M + 1]⁺. ¹H NMR (400 MHz, DMSO) δ 6.38 (s, 1H), 3.67 (br s, 2H), 3.50 (br s, 2H), 3.35 (d, *J=* 12.5 Hz, 2H), 3.17-3.12 (m, 2H), 2.91 (s, 2H), 2.21 (s, 6H), 1.38 (s, 9H).

**Compound KH06-3:** a solution of hydrogen chloride in ethyl acetate (8 mL) was added to a solution of compound **KH06-2** (320 mg, 1.01 mmol) in ethyl acetate (16 mL), and the mixture was allowed to react at 60°C for 1 hour. After the reaction was complete, the reaction solution was concentrated in vacuum under reduced pressure to obtain **compound KH06-3.** LCMS (ESI, *m*/*z):* 219.2 [M + 1]⁺. ¹H NMR (400 MHz, DMSO) δ 9.65 (d, *J =* 50.5 Hz, 2H), 6.69 (s, 1H), 3.86-3.79 (m, 4H), 3.47-3.42 (m, 2H), 3.16 - 3.06 (m, 4H), 2.39 (s, 6H).

**Compound KH06: KH05-5** (160 mg, 0.57 mmol), HATU (324 mg, 0.85 mmol) and DIEA (0.23 mL, 2.27 mmol were added to a solution of compound **KH06-3** (174 mg, 0.68 mmol) in DMF (3 mL), and the mixture was stirred at room temperature for 1 hour. After the reaction was complete, the reaction solution was purified by preparative reverse-phase chromatography to obtain compound **KH06.** LCMS (ESI, m/z): 483.3 [M + 1]+. 1H NMR (400 MHz, DMSO) δ 8.63 (s, 1H), 7.79-7.65 (m, 3H), 6.38 (s, 1H), 3.75 (m, 2H), 3.72-3.43 (m, 5H), 3.18-3.14 (m, 1H), 3.06 - 2.82 (m, 3H), 2.28-2.21 (m, 7H), 2.11 (s, 1H).

### Example 7 Synthesis of compound KH07

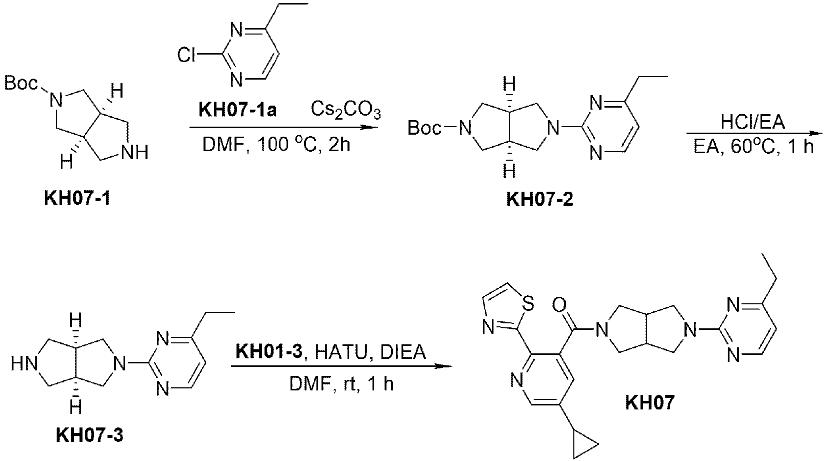

**Compound KH07-2:** compound KH07-1 (983 mg, 4.63 mmol) and cesium carbonate (1.646 g, 5.05 mmol) were added to a solution of compound KH07-1a (600 mg, 4.21 mmol) in DMF (12 mL), and the mixture was allowed to react at 100°C for 2 hours. After the reaction was complete, the reaction system was quenched with water, extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=10: 1) to obtain **compound KH07-2.** LCMS (ESI, *m*/*z):* 319.2 [M + 1]⁺. ¹H NMR (400 MHz, DMSO) δ 8.20 (d, *J =* 4.9 Hz, 1H), 6.50 (d, *J =* 4.9 Hz, 1H), 3.69 (br s, 2H), 3.51 (br s, 2H), 3.36 (s, 2H), 3.15 (d, *J =* 9.5 Hz, 2H), 2.93 (br s, 2H), 2.56 - 2.50 (m, 2H), 1.38 (s, 9H), 1.19-1.14 (m, 3H).

**Compound KH07-3:** a solution of hydrogen chloride in ethyl acetate (2 mL) was added to a solution of compound **KH07-2** (200 mg, 0.63 mmol) in EA (4 mL), and the mixture was allowed to react at 60°C for 1 hour. After the reaction was complete, the reaction solution was concentrated in vacuum under reduced pressure to obtain **compound KH07-3.** LCMS (ESI, *m*/*z):* 219.2 [M + 1]⁺. ¹H NMR (400 MHz, DMSO) δ 9.60 (d, J= 32.3 Hz, 2H), 8.30 (d, J= 5.7 Hz, 1H), 6.78 (d, *J =* 5.6 Hz, 1H), 3.82-3.78 (m, 2H), 3.69-3.63 (m, 2H), 3.45 - 3.37 (m, 2H), 3.13 (br s, 4H), 2.69 (q, *J =* 7.5 Hz, 2H), 1.20 (t, *J =* 7.5 Hz, 3H).

**Compound KH07: KH07-3** (103 mg, 0.40 mmol), HATU (191 mg, 0.50 mmol) and DIEA (0.22 mL, 1.33 mmol) were added to a solution of compound **KH01-3** (80 mg, 0.33 mmol) in DMF (3 mL), and the mixture was stirred at room temperature for 1 hour. After the reaction was complete, the reaction solution was purified by preparative reverse-phase chromatography to obtain **compound KH07.** LCMS (ESI, *m*/*z):* 447.2 [M + 1]⁺. ¹H NMR (400 MHz, DMSO) δ 8.48 (d, *J =* 1.8 Hz, 1H), 8.23 (s, 1H), 7.79-7.52 (m, 2H), 7.47 (d, *J =* 2.0 Hz, 1H), 6.61 (d, *J* = 4.7 Hz, 1H), 3.78-3.25 (m, 8H), 2.91 (br s, 2H), 2.60 (d, *J =* 6.9 Hz, 2H), 2.05 (br s, 1H), 1.19 (s, 3H), 1.10 - 0.84 (m, 4H).

### Example 8 Synthesis of compound KH08

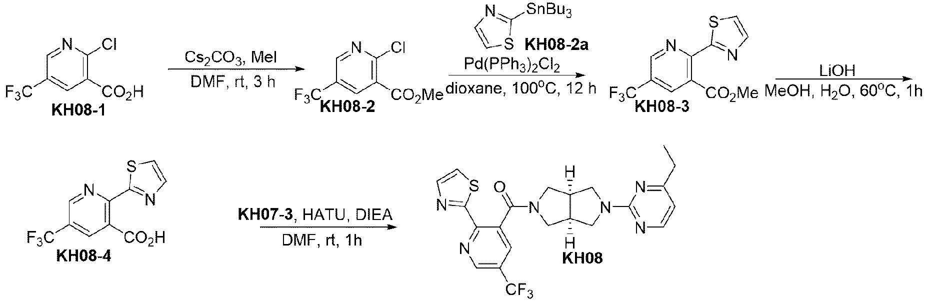

**Compound** KH08-2: MeI (639 mg, 4.50 mmol) and cesium carbonate (1.955 g, 6.00 mmol) were added to a solution of compound KH08-1 (677 mg, 3.00 mmol) in DMF (14 mL), and the mixture was allowed to react at room temperature for 3 hours. After the reaction was complete, the reaction system was quenched with water, extracted with ethyl acetate, washed with saturated brine, dried with anhydrous sodium sulfate, and concentrated in vacuum under reduced pressure to obtain **compound KH08-2**. LCMS (ESI, *m*/*z*): 240.1 [M + 1]⁺. ¹H NMR (400 MHz, DMSO) δ 9.05 (dd, *J* = 2.4, 0.8 Hz, 1H), 8.64 (dd, *J* = 2.4, 0.4 Hz, 1H), 3.92 (s, 3H).

**Compound KH08-3**: compound **KH08-2a** (0.9 mL, 2.75 mmol) and bis(triphenylphosphine)palladium(II) dichloride (176 mg, 0.25 mmol) were added to a solution of compound **KH08-2** (600 mg, 2.50 mmol) in 1,4-dioxane (12 mL) , and the mixture was allowed to react at 100°C for 12 hours. After the reaction was complete, the reaction solution was concentrated in vacuum under reduced pressure to obtain a crude product. The crude product was purified by silica gel column chromatography (petroleum ether: ethyl acetate=3: 1) to obtain **compound KH08-3**. LCMS (ESI, *m*/*z*): 289.0 [M + 1]⁺. ¹H NMR (400 MHz, DMSO) δ 9.20 - 9.13 (m, 1H), 8.58 (d, *J* = 1.6 Hz, 1H), 8.12-8.05 (m, 2H), 3.86 (s, 3H).

**Compound KH08-4**: lithium hydroxide monohydrate (88 mg, 2.08 mmol) and water (4 mL) were added to a solution of compound **KH08-3** (200 mg, 0.69 mmol) in methanol (8 mL), and the mixture was stirred at 60°C for 1 hour. After the reaction was complete, pH was adjusted to be 5 with dilute hydrochloric acid. The mixture was extracted with ethyl acetate, washed with saturated brine, dried with anhydrous sodium sulfate, and concentrated in vacuum under reduced pressure to obtain **compound KH08-4**. LCMS (ESI, *m*/*z*): 275.0 [M + 1]⁺. ¹H NMR (400 MHz, DMSO) δ 13.74 (s, 1H), 9.12 (s, 1H), 8.50 (s, 1H), 8.05 (t, *J* = 4.9 Hz, 2H).

**Compound KH08**: **KH07-3** (121 mg, 0.47 mmol), HATU (271 mg, 0.71 mmol) and DIEA (0.31 mL, 1.90 mmol) were added to a solution of compound **KH08-4** (130 mg, 0.47 mmol) in DMF (3 mL), and the mixture was stirred at room temperature for 1 hour. After the reaction was complete, the reaction solution was purified by preparative reverse-phase chromatography to obtain **compound KH08**. LCMS (ESI, *m*/*z*): 475.2 [M + 1]⁺. ¹H NMR (400 MHz, DMSO) δ 9.09 (d, *J* = 1.2 Hz, 1H), 8.39 (s, 1H), 8.20 (d, *J* = 4.8 Hz, 1H), 8.15-7.62 (m, 2H), 6.50 (d, *J* = 5.0 Hz, 1H), 3.93 - 3.43 (m, 6H), 3.36 (br s, 1H), 3.16 - 2.86 (m, 3H), 2.54 (d, *J* = 7.6 Hz, 2H), 1.18 (br s, 3H).

### Example 9 Synthesis of compound KH09

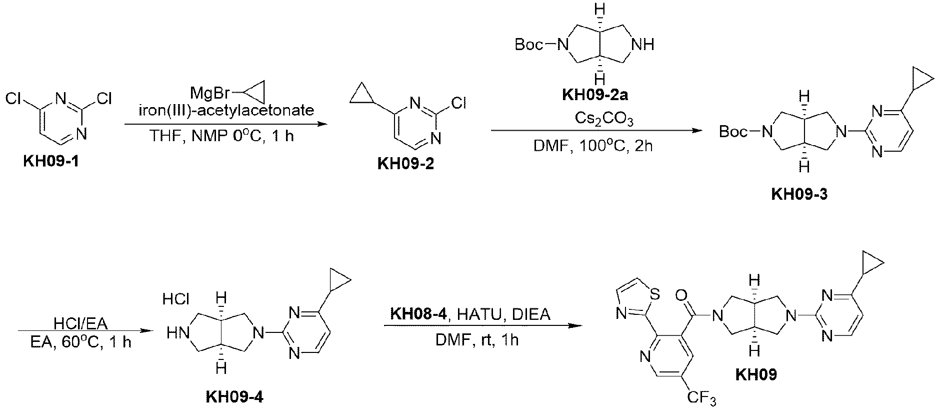

**Compound KH09-2:** cyclopropylmagnesium bromide (67 mL, 67.0 mmol) was added to a solution of compound **KH09-1** (5 g, 33.61 mmol) and iron acetylacetonate (2.370 g, 6.70 mmol) in tetrahydrofuran (30 mL) and NMP (5 mL) at 0°C, and the mixture was allowed to react at room temperature for 1 hour. After the reaction was complete, the reaction system was quenched with a saturated ammonium chloride solution, extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=10: 1) to obtain **compound KH09-2.** LCMS (ESI, *m*/*z):* 155.2 [M + 1]⁺.

**Compound KH09-3:** compound **KH09-2a** (412 mg, 1.94 mmol) and cesium carbonate (759 mg, 2.33 mmol) were added to a solution of compound **KH09-2** (500 mg, 3.23 mmol) in DMF (10 mL), and the mixture was allowed to react at 100°C for 2 hours. After the reaction was complete, the reaction system was quenched with water, extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=5: 1) to obtain **compound KH09-3.** LCMS (ESI, *m*/*z):* 331.2 [M + 1]⁺. ¹H NMR (400 MHz, DMSO) δ 8.09 (d, *J =* 5.0 Hz, 1H), 6.53 (d, *J* = 5.0 Hz, 1H), 3.64 (d, *J* = 5.0 Hz, 2H), 3.49 (br s, 2H), 3.32 - 3.27 (m, 2H), 3.16-3.12 (m, 2H), 2.90 (br s, 2H), 1.92 - 1.85 (m, 1H), 1.38 (s, 9H), 1.00 - 0.90 (m, 4H).

**Compound KH09-4:** a solution of hydrogen chloride in ethyl acetate (6 mL) was added to a solution of compound **KH09-3** (240 mg, 0.73 mmol) in ethyl acetate (12 mL), and the mixture was allowed to react at 60°C for 1 hour. After the reaction was complete, the reaction solution was concentrated in vacuum under reduced pressure to obtain **compound KH09-4.** LCMS (ESI, *m*/*z):* 231.1 [M + 1]⁺.

**Compound KH09:** KH09-4 (108 mg, 0.40 mmol), HATU (229 mg, 0.60 mmol) and DIEA (0.27 mL, 1.60 mmol) were added to a solution of compound KH08-4 (110 mg, 0.40 mmol) in DMF (3 mL), and the mixture was stirred at room temperature for 1 hour. After the reaction was complete, the reaction solution was purified by preparative reverse-phase chromatography to obtain **compound KH09.** LCMS (ESI, m/z): 487.2 [M + 1]⁺. ¹H NMR (400 MHz, DMSO) δ 9.09 (s, 1H), 8.39 (s, 1H), 8.13 - 7.80 (m, 3H), 6.53 (d, *J= 4.9* Hz, 1H), 3.84 - 3.42 (m, 6H), 3.29 - 3.18 (m, 1H), 3.09 - 2.84 (m, 3H), 1.92-1.87 (m, 1H), 0.95 (br s, 4H).

### Example 10 Synthesis of compound KH10

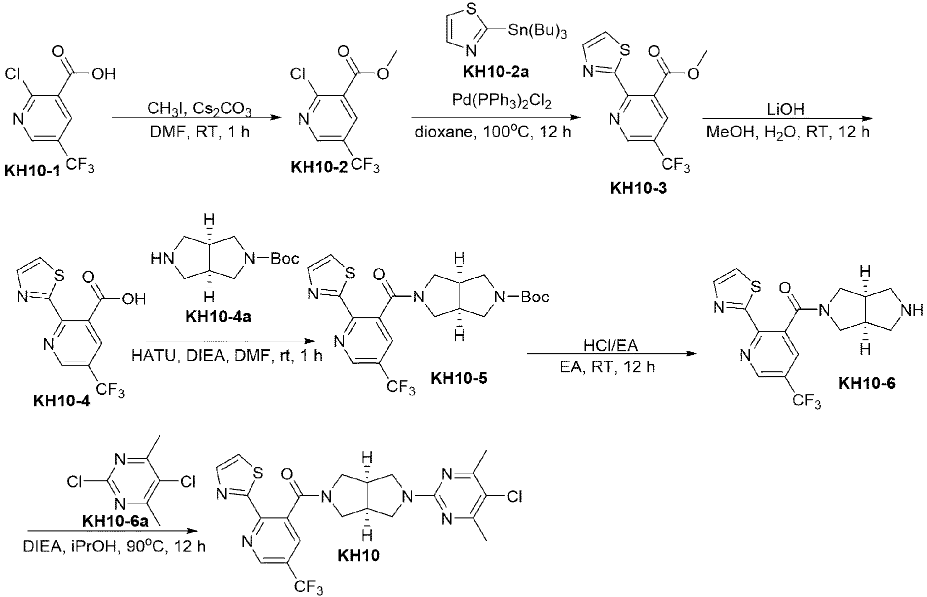

**Compound KH10-2:** iodomethane (4.72 g, 33.25 mmol) and cesium carbonate (14.446 g, 44.34 mmol) were added to a solution of compound **KH10-1** (5 g, 22.17 mmol) in DMF (50 mL), and the mixture was allowed to react at room temperature for 2 hours. After the reaction was complete, water was added to quench the reaction. The mixture was extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=5: 1) to obtain **compound KH10-2.** LCMS (ESI, *m*/*z):* 239.9 [M + 1]⁺. ¹H NMR (400 MHz, DMSO) δ 9.04 (s, 1H), 8.63 (s, 1H), 3.92 (s, 3H).

**Compound KH10-3:** a compound **KH10-2a** (7.628 g, 20.39 mmol) and Pd(PPh₃)Cl₂ (650 mg, 0.93 mmol) were added to a solution of compound **KH10-2** (4.44 g, 18.53 mmol) in dioxane (50 mL), and the mixture was allowed to react at 100°C for 12 hours. After the reaction was complete, water was added to quench the reaction. The mixture was extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=3: 1) to obtain **compound KH10-3**. LCMS (ESI, *m*/*z*): 288.9 [M + 1]⁺. ¹H NMR (400 MHz, DMSO) δ 9.17 (d, *J* = 1.1 Hz, 1H), 8.58 (d, *J* = 1.5 Hz, 1H), 8.07 (dd, *J* = 10.1, 3.1 Hz, 2H), 3.86 (s, 3H).

**Compound KH10-4**: lithium hydroxide (812 mg, 33.93 mmol) was added to a solution of compound **KH10-3** (4.89 g, 16.97 mmol) in methanol (40 mL) and water (10 mL), and the mixture was allowed to react at room temperature for 12 hours. After the reaction was complete, an aqueous hydrochloric acid solution was added to adjust pH of the reaction solution to be 5. The mixture was extracted with ethyl acetate, washed with saturated brine, dried with anhydrous sodium sulfate, and concentrated in vacuum under reduced pressure to obtain **compound KH10-4**. LCMS (ESI, *m*/*z*): 274.9 [M + 1]⁺.

**Compound KH10-5**: compound **KH10-4a** (1.548 g, 7.29 mmol), HATU (3.328 g, 8.75 mmol) and DIEA (2.54 mL, 14.59 mmol) were added to a solution of compound **KKH10-4** (2 g, 7.29 mmol) in DMF (20 mL), and the mixture was allowed to react at room temperature for 1 hour. After the reaction was complete, water was added to quench the reaction. The mixture was extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=1: 1) to obtain **compound KH10-5**. LCMS (ESI, *m*/*z*): 469.1 [M + 1]⁺.

**Compound KH10-6**: a solution of hydrogen chloride in ethyl acetate (20 mL) was added to a solution of compound **KH10-5** (1 g, 2.13 mmol) in ethyl acetate (10 mL), and the mixture was allowed to react at room temperature for 12 hours. After the reaction was complete, the reaction solution was concentrated in vacuum under reduced pressure to obtain **compound KH10-6**. LCMS (ESI, *m*/*z*): 369.0 [M + 1]⁺.

Compound **KH10**: compound **KH10-6a** (48 mg, 0.27 mmol) and DIEA (0.16 mL, 0.95 mmol) were added to a solution of compound **KH10-6** (150 mg, 0.41 mmol) in isopropanol (10 mL), and the mixture was allowed to react at 90°C for 12 hours. After the reaction was complete, the reaction solution was subjected to suction filtration to obtain **compound KH10**. LCMS (ESI, *m*/*z*): 509.1 [M + 1]⁺. ¹H NMR (400 MHz, CD₃OD_SPE) δ 8.97 (s, 1H), 8.23 (d, *J* = 1.7 Hz, 1H), 7.96-7.67 (m, 2H), 3.89-3.81 (m, 2H), 3.78-3.56 (m, 4H), 3.52-3.42 (m, 1H), 3.16 - 3.00 (m, 3H), 2.40 (s, 6H).

### Example 11 Synthesis of compound KH11

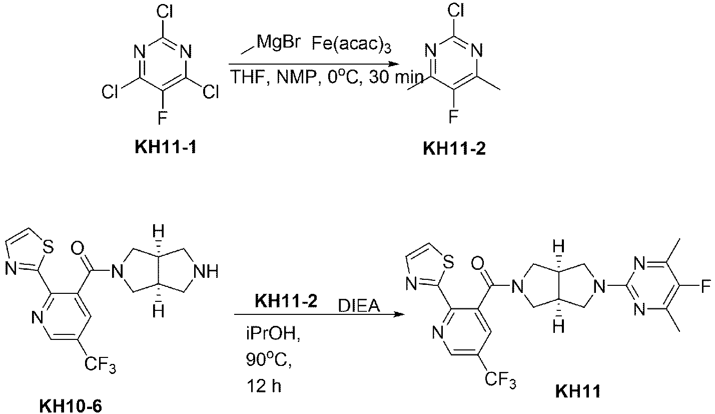

**Compound KH11-2:** Fe(acac)₃ (175 mg, 0.49 mmol) was added to a solution of compound **KH11-1** (1 g, 4.96 mmol) in THF: NMP=10: 1 (10 mL/1 mL) at room temperature, the reaction system was cooled to 0°C and methylmagnesium bromide (4 mL, 12.41 mmol) was added, and the mixture was allowed to react at 0°C for 0.5 hour. After the reaction was complete, the reaction solution was cooled to room temperature and then a saturated aqueous ammonium chloride solution was added to quench the reaction. The mixture was extracted with dichloromethane, washed with saturated brine, dried with anhydrous sodium sulfate, and concentrated in vacuum under reduced pressure to obtain a crude product. The crude product was purified by silica gel column chromatography (petroleum ether: ethyl acetate=4: 1) to obtain **compound KH11-2.** LCMS (ESI, *m*/*z):* 161.0 [M + 1]⁺. ¹H NMR (400 MHz, DMSO) δ 2.43 (d, *J =* 2.7 Hz, 6H).

**Compound KH11:** compound **KH11-2** (70 mg, 0.44 mmol) and DIEA (0.27 mL, 1.53 mmol) were added to a solution of compound **KH10-6** (241 mg, 0.65 mmol) in isopropanol (4 mL), and the mixture was stirred at 90°C overnight. After the reaction was complete, the reaction solution was purified by preparative reverse-phase chromatography to obtain **compound KH11.** LCMS (ESI, *m*/*z):* 493.1 [M + 1]⁺. ¹H NMR (400 MHz, DMSO) δ 9.09 (d, *J* = 1.3 Hz, 1H), 8.38 (s, 1H), 8.11-7.62 (m, 2H), 3.73 (dd, *J =* 11.4, 7.6 Hz, 2H), 3.63-3.45 (m, 4H), 3.33 (s, 1H), 3.11 - 2.86 (m, 3H), 2.27 (s, 6H).

### Example 12 Synthesis of compound KH12

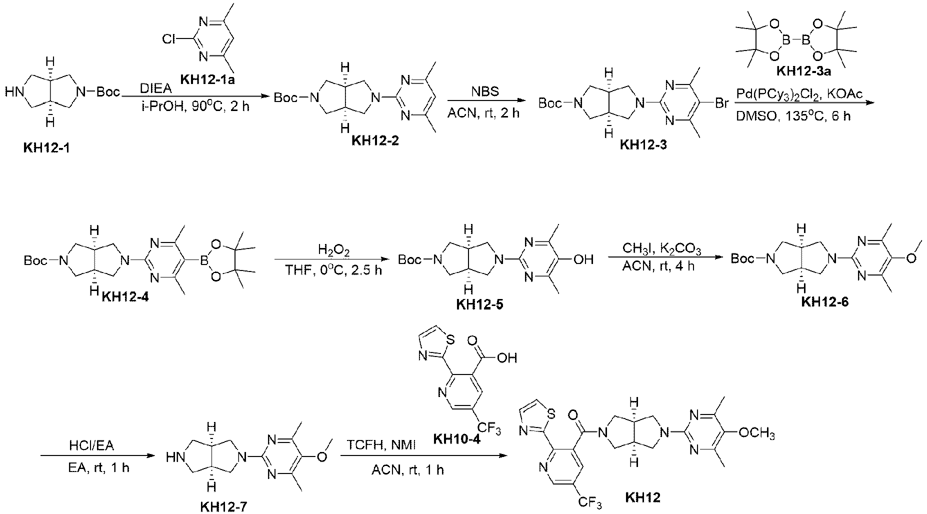

**Compound KH12-2:** compound **KH12-1a** (2.015 g, 14.1 mmol) and DIEA (2.435 g, 18.8 mmol) were added to a solution of compound **KH12-1** (2 g, 9.4 mmol) in isopropanol (20 mL), and the mixture was allowed to react at 90°C for 2 hours. After the reaction was complete, water was added to quench the reaction. The mixture was extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=5: 1) to obtain **compound KH12-2.** LCMS (ESI, *m*/*z):* 319.1 [M + 1]⁺. ¹H NMR (400 MHz, DMSO) δ 6.38 (s, 1H), 3.73 - 3.63 (m, 2H), 3.58 - 3.47 (m, 2H), 3.38 - 3.36 (m, 1H), 3.14 (dd, *J=* 11.1, 3.7 Hz, 2H), 2.99 - 2.87 (m, 2H), 2.51 (s, 1H), 2.21 (s, 6H), 1.39 (s, 9H).

**Compound KH12-3:** NBS (837 mg, 4.7 mmol) was added to a solution of compound **KH12-2** (1 g, 3.13 mmol) in acetonitrile (10 mL), and the mixture was allowed to react at room temperature for 2 hours. After the reaction was complete, water was added to quench the reaction. The mixture was extracted with ethyl acetate, washed with saturated brine, dried with anhydrous sodium sulfate, and concentrated in vacuum under reduced pressure to obtain a crude product. The crude product was purified by silica gel column chromatography (petroleum ether: ethyl acetate=5: 1) to obtain **compound KH12-3.** LCMS (ESI, *m*/*z):* 399.1 [M + 1]⁺. ¹H NMR (400 MHz, DMSO) δ 3.74 - 3.60 (m, 2H), 3.58 - 3.43 (m, 2H), 3.33 - 3.29 (m, 1H), 3.14 (dd, *J=* 11.2, 4.0 Hz, 2H), 2.97 - 2.88 (m, 2H), 2.54 - 2.48 (m, 1H), 2.38 (s, 6H), 1.39 (s, 9H).

Compound **KH12-4:** compound **KH12-3a** (597 mg, 2.35 mmol), Pd(Pcy₃)₂Cl₂ (87 mg, 0.12 mmol) and potassium acetate (404 mg, 4.11 mmol) were added to a solution of compound **KH12-3** (467 mg, 1.18 mmol) in DMSO (5 mL), and the mixture was allowed to react at 135°C for 6 hours. After the reaction was complete, water was added to quench the reaction. The mixture was extracted with ethyl acetate, washed with saturated brine, dried with anhydrous sodium sulfate, and concentrated in vacuum under reduced pressure to obtain **compound KH12-4**. LCMS (ESI, *m*/*z*): 445.3 [M + 1]⁺.

**Compound KH12-5**: hydrogen peroxide (4.67 g, 41.23 mmol) was added to a solution of compound **KH12-4** (916 mg, 2.06 mmol) in THF (10 mL), and the mixture was allowed to react at 0°C for 2.5 hours. After the reaction was complete, sodium sulfite was added to quench the reaction, and ethyl acetate was added for extraction of the organic phase. The organic phase was washed with saturated brine, dried with anhydrous sodium sulfate, and concentrated in vacuum under reduced pressure to obtain a crude product. The crude product was purified by silica gel column chromatography (petroleum ether: ethyl acetate=2: 1) to obtain **compound KH12-5**. LCMS (ESI, *m*/*z*): 335.7 [M + 1]⁺. ¹H NMR (400 MHz, DMSO) δ 7.90 (s, 1H), 3.65 - 3.56 (m, 2H), 3.53 - 3.46 (m, 2H), 3.30 - 3.25 (m, 2H), 3.15 - 3.10 (m, 2H), 2.94 - 2.86 (m, 2H), 2.22 (s, 6H), 1.39 (s, 9H).

**Compound KH12-6**: iodomethane (181 mg, 1.27 mmol) and potassium carbonate (211 mg, 1.53 mmol) were added to a solution of compound **KH12-5** (170 mg, 0.51 mmol) in acetonitrile (2 mL), and the mixture was allowed to react at room temperature for 4 hours. After the reaction was complete, water was added to quench the reaction, and ethyl acetate was added for extraction of the organic phase. The organic phase was washed with saturated brine, dried with anhydrous sodium sulfate, and concentrated in vacuum under reduced pressure to obtain a crude product. The crude product was purified by silica gel column chromatography (petroleum ether: ethyl acetate=2: 1) to obtai**n compound KH12-6**. LCMS (ESI, *m*/*z*): 349.3 [M + 1]⁺. ¹H NMR (400 MHz, DMSO) δ 3.63 (d, *J* = 6.9 Hz, 2H), 3.59 (s, 3H), 3.50 (d, *J* = 5.1 Hz, 2H), 3.33 - 3.29 (m, 2H), 3.13 (dd, *J* = 11.1, 4.1 Hz, 2H), 2.99 - 2.83 (m, 2H), 2.25 (s, 6H), 1.39 (s, 9H).

**Compound KH12-7**: a solution of hydrochloric acid in ethyl acetate (5 mL) was added to a solution of compound **KH12-6** (150 mg, 0.41 mmol) in ethyl acetate (2 mL), and the mixture was allowed to react at room temperature for 1 hour. After the reaction was complete, the reaction solution was concentrated in vacuum under reduced pressure to obtai**n compound KH12-7**. LCMS (ESI, *m*/*z*): 249.2 [M + 1]⁺.

**Compound KH12**: compound **KH10-4** (110 mg, 0.4 mmol), TCFH (135 mg, 0.5 mmol) and NMI (115 mg, 1.4 mmol) were added to a solution of compound **KH12-7** (150 mg, 0.6 mmol) in acetonitrile (2 mL), and the mixture was allowed to react at room temperature for 1 hour. After the reaction was complete, the reaction solution was purified by preparative reverse-phase chromatography to obtain compound KH12. LCMS (ESI, *m*/*z):* 505.1 [M + 1]⁺. ¹H NMR (400 MHz, DMSO) δ 9.09 (s, 1H), 8.39 (s, 1H), 8.17-7.83 (m, 2H), 3.62 (s, 3H), 3.59 - 3.44 (m, 5H), 3.39-3.32 (m, 2H), 3.12-3.07 (m, 3H), 2.30 (s, 6H).

### Example 13 Synthesis of compound KH13

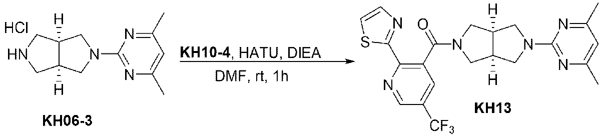

**Compound KH13:** compound **KH06-3** (15 g, 56.40 mmol), HATU (23.38 g, 61.53 mmol) and DIEA (25 mL, 153.82 mmol) were added to a solution of compound **KKH10-4** (14.06 g, 51.27 mmol) in DMF (200 mL), and the mixture was allowed to react at room temperature for 1 hour. After the reaction was complete, water and ethyl acetate were added for extraction. The organic phase was washed with saturated brine, dried with anhydrous sodium sulfate, and concentrated in vacuum under reduced pressure to obtain a crude product. The crude product was triturated with petroleum ether and then filtered to obtain **compound KH13.** LCMS (ESI, *m*/*z):* 475.2 [M + 1]⁺. ¹H NMR (400 MHz, DMSO) δ 9.09 (d, *J =* 1.0 Hz, 1H), 8.39 (s, 1H), 8.13 - 7.81 (m, 2H), 6.44 (s, 1H), 3.88-3.72 (m, 2H), 3.67 - 3.37 (m, 5H), 3.04-2.89 (m, 3H), 2.25 (s, 6H).

### Example 14 Synthesis of compound KH14

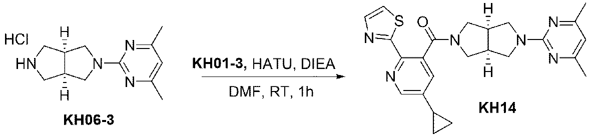

**Compound KH14: KH06-3** (312 mg, 1.21 mmol), HATU (579 mg, 1.52 mmol) and DIEA (0.74 mL, 4.06 mmol) were added to a solution of compound **KH01-3** (250 mg, 1.01 mmol) in DMF (4 mL), and the mixture was stirred at room temperature for 1 hour. After the reaction was complete, the reaction solution was purified by preparative reverse-phase chromatography to obtain **compound KH14.** LCMS (ESI, *m*/*z):* 447.2 [M + 1]⁺. ¹H NMR (400 MHz, DMSO) δ 8.47 (d, *J =* 2.1 Hz, 1H), 7.89-7.69 (m, 2H), 7.46 (d, *J =* 2.1 Hz, 1H), 6.37 (s, 1H), 3.86 - 3.66 (m, 2H), 3.64 - 3.33 (m, 5H), 3.04 - 2.83 (m, 3H), 2.22 (s, 6H), 2.08 - 2.01 (m, 1H), 1.07 (d, *J =* 6.9 Hz, 2H), 0.88 (s, 2H).

### Example 15 Synthesis of compound KH15

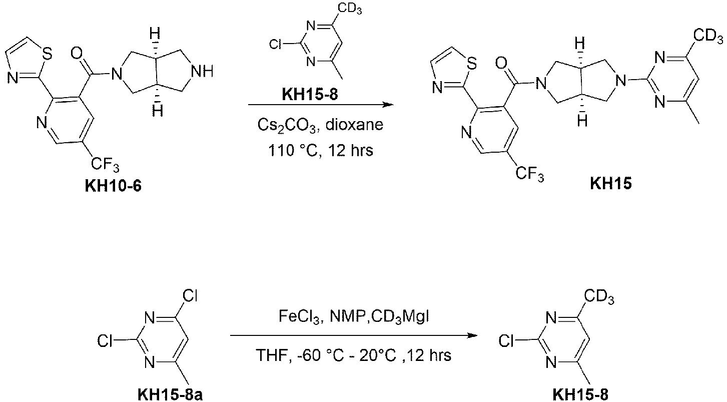

**Compound KH15-8:** NMP (2.19 g, 22.0 mmol, 2.15 mL, 1.2 eq) and FeCl₃ (59.7 mg, 368 µmol, 21.3 µL, 0.02 eq) added to a solution of compound **KH15-8a** (3.00 g, 18.4 mmol, 1 eq) in THF (30 mL), and after the addition was finished, CD₃MgI (1 M, 18.4 mL, 1 eq) was dropwise added at -60°C. After the dropwise addition was finished, the system was stirred at 20°C for 12 hours. TLC (petroleum ether/ethyl acetate=2/1, compound **KH15-8a** Rf=0.40, new spot Rf=0.25) showed that the starting materials were substantially consumed and the target product was generated. After the reaction was complete, water (50 mL) was added to the reaction mixture, and then the mixture was extracted with ethyl acetate (50 mL*2). The organic layers were combined, washed with saturated brine (50 mL*2), dried with anhydrous sodium sulfate, filtered, and concentrated under reduced pressure to obtain a crude product. The crude product was purified by silica gel column chromatography (petroleum ether/ethyl acetate=20/1-3/1) to obtain the target compound **KH15-8.** LCMS(ESI, m/z)= 146.1 [M+1]⁺ . ¹HNMR: DMSO-*d₆*, 400MHz δ 7.32 (s, 1 H), 2.41 (s, 3 H).

**Compound KH15:** the compound **KH15-8** (31.6 mg, 217 µmol, 1 eq) and Cs₂CO₃ (176 mg, 542 µmol, 2.5 eq) were added to a solution of compound **KH10-6** (80.0 mg, 217 µmol, 1 eq) in 1,4-dioxane (2 mL), and after the addition was finished, the system was subjected to nitrogen replacement three times. Under nitrogen protection, the mixture was stirred at 110°C for 12 hours. LCMS (EW30597-215-P1A) showed that the starting materials were substantially consumed and the target product was generated. After the reaction was complete, 10 mL of water was added to the reaction mixture, and then the mixture was extracted with ethyl acetate (20 mL*2). The organic layers were combined, washed with saturated brine (20 mL*2), dried with anhydrous sodium sulfate, filtered, and concentrated under reduced pressure to obtain a crude product. The crude product was purified by preparative high-performance liquid chromatography to obtain the target compound KH15. LCMS(ESI, m/z) = 478.1 [M+1]⁺. ¹HNMR: 400 MHz, CDCl₃ δ 8.87 (s, 1 H), 7.85 - 8.00 (m, 2 H), 7.35 - 7.65 (m, 1 H), 6.27 - 6.34 (m, 1 H), 3.62 - 4.17 (m, 5 H), 3.41 - 3.60 (m, 2 H), 2.87 - 3.21 (m, 3 H), 2.22 - 2.38 (m, 3 H).

### Example 16 Synthesis of compound KH16

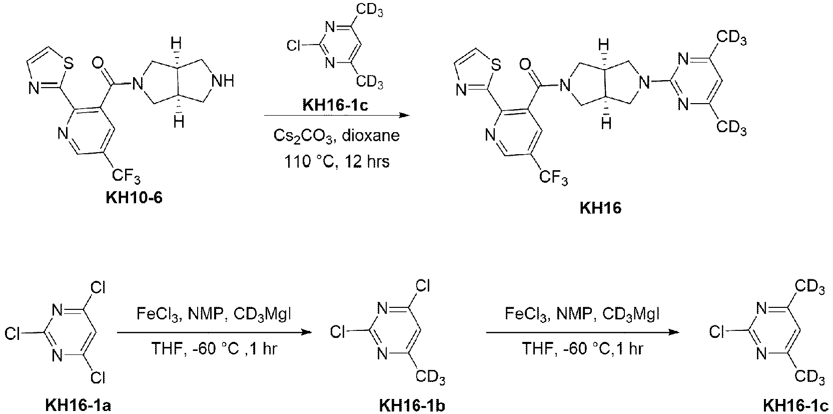

**Compound KH16-1b:** FeCl₃ (70.7 mg, 436 µmol, 25.2 µL, 0.04 eq) and NMP (2.59 g, 26.1 mmol, 2.54 mL, 2.4 eq) were added to a solution of compound **KH16-1a** (2.00 g, 10.9 mmol, 1.25 mL, 1 eq) in tetrahydrofuran (40 mL), and after the addition was finished, CD₃MgI (1 M, 21.8 mL, 2 eq) was dropwise added at -60°C. After the dropwise addition was finished, the mixture was stirred at -60°C for 1 hour. TLC (petroleum ether/ethyl acetate=5/1, compound **KH16-1a** Rf/=0.50, new spot Rf=0.40) and LCMS showed that the starting materials were substantially consumed and the target product was generated. The reaction mixture was quenched with water (50 mL), and then extracted with ethyl acetate (80 mL*2). The organic layers were combined, washed with saturated brine (50 mL), dried with anhydrous sodium sulfate, filtered, and concentrated under reduced pressure to obtain a crude product. The crude product was purified by silica gel column chromatography (petroleum ether/ethyl acetate=1/0-0/1) to obtain the target compound **KH16-1b.** LCMS(ESI, m/z) = 166.0 [M+1]⁺.

**Compound KH16-1c:** NMP (788 mg, 7.95 mmol, 772 µL, 1.2 eq) and FeCl₃ (21.4 mg, 132 µmol, 7.68 µL, 0.02 eq) were added to a solution of compound **KH16-1b** (1.10 g, 6.63 mmol, 1 eq) in tetrahydrofuran (40 mL), and then CD₃MgI (1 M, 6.63 mL, 1 eq) was dropwise added at -60°C. After the dropwise addition was finished, the reaction mixture was stirred at -60°C for 1 hour. TLC (petroleum ether/ethyl acetate=5/1, compound **KH16-1b** Rf=0.40, new spot Rf=0.25) showed that the starting materials were substantially consumed and the target product was generated. The reaction mixture was quenched with water (50 mL), and then extracted with ethyl acetate (80 mL*2). The organic layers were combined, washed with saturated brine (50 mL), dried with anhydrous sodium sulfate, filtered, and concentrated under reduced pressure to obtain a crude product. The crude product was purified by silica gel column chromatography (petroleum ether/ethyl acetate=1/0-0/1) to obtain the target compound **KH16-1c**. LCMS(ESI, m/z) = 149.0 [M+1]⁺.

Compound KH16: compound KH16-1c (48.4 mg, 325 µmol, 1.5 eq) and Cs₂CO₃ (176 mg, 542 µmol, 2.5 eq) were added to a solution of compound KH10-6 (80.0 mg, 217 µmol, 1 eq) in 1,4-dioxane (2 mL), and the system was subjected to nitrogen replacement three times. Under nitrogen protection, the mixture was stirred at 110°C for 12 hours. LCMS showed that the starting materials were substantially consumed and the target product was generated. 10 mL of water was added to the reaction mixture, and then the mixture was extracted with ethyl acetate (20 mL*2). The organic layers were combined, washed with saturated brine (10 mL*2), dried with anhydrous sodium sulfate, filtered, and concentrated under reduced pressure to obtain a crude product. The crude product was purified by high-performance liquid chromatography to obtain the target compound KH16. LCMS(ESI, m/z) = 481.2 [M+1]+. ¹H NMR (400 MHz, DMSO-d6) δ 9.09 (s, 1H), 8.39 (s, 1H), 8.08 - 7.83 (m, 2H), 6.38 (s, 1H), 3.87 - 3.70 (m, 2H), 3.54 (d, J = 29.5 Hz, 4H), 3.40 - 3.35 (m, 1H), 3.06 - 2.85 (m, 3H).

### Example 17 Synthesis of compound KH17

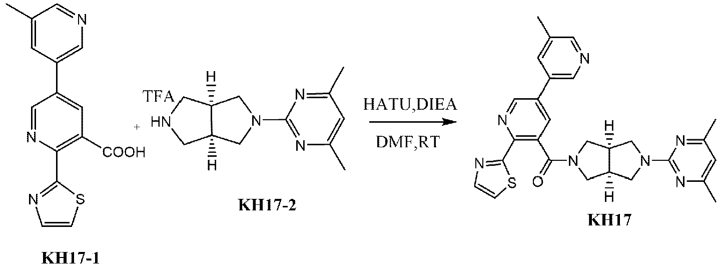

**Compound KH17**: HATU (103 mg, 2 eq), DIEA (174 mg, 10 eq) and compound KH17-2 (90 mg, 2 eq) were successively added to a solution of compound **KH17-1** (40 mg, 1 eq) in DMF (3 mL), and after the addition was finished, the mixture was stirred at room temperature overnight. After the reaction was complete, water (30 mL) was added for dilution, the mixture was extracted with ethyl acetate (30 mL×2), and organic phases were collected. The organic phases were dried with anhydrous sodium sulfate, filtered and concentrated to obtain a crude product. The crude product was purified by column chromatography to obtain **KH17**. LCMS (ESI, *m*/*z*): 498.3 [M + 1]⁺.

### Example 18 Synthesis of compound KH18

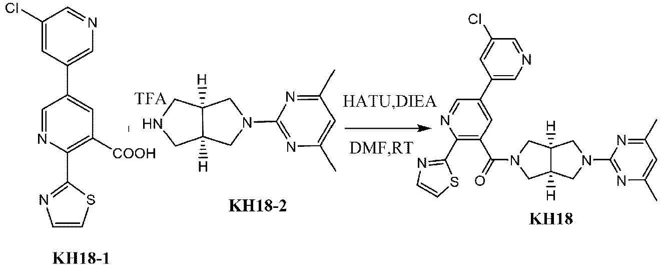

**Compound KH18**: HATU (272mg, 2 eq), DIEA (460mg, 10 eq) and compound KH18-2 (238 mg, 2 eq) were successively added to a solution of compound **KH18-1** (114 mg, 1 eq) in DMF (10 mL), and after the addition was finished, the mixture was stirred at room temperature overnight. After the reaction was complete, water (30 mL) was added for dilution, the mixture was extracted with ethyl acetate (50 mL×2), and organic phases were collected. The organic phases were dried with anhydrous sodium sulfate, filtered and concentrated to obtain a crude product. The crude product was purified by column chromatography to obtain **KH18**. LCMS (ESI, *m*/*z*): 518.2 [M + 1]⁺.

### Example 19 Synthesis of compound KH19

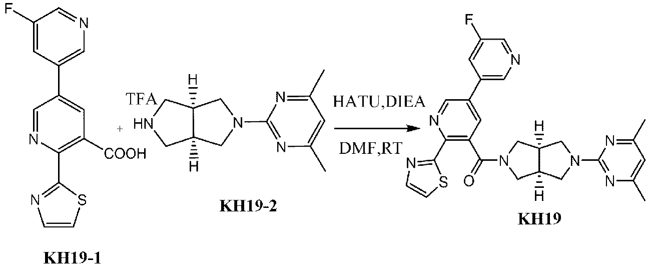

**Compound KH19**: HATU (329 mg, 2 eq), DIEA (169 mg, 3 eq) and compound KH19-2 (288 mg, 2 eq) were successively added to a solution of compound **KH19-1** (130 mg, 1 eq) in DCM (10 mL), and after the addition was finished, the mixture was stirred at room temperature overnight. After the reaction was complete, water (200 mL) was added for dilution, the mixture was extracted with dichloromethane (100 mL), and the organic phase was collected. The organic phase was dried with anhydrous sodium sulfate, filtered and concentrated to obtain a crude product. The crude product was purified by column chromatography to obtain **KH19**. LCMS (ESI, *m*/*z*): 501.2 [M + 1]⁺.

### Example 20 Synthesis of compound KH20

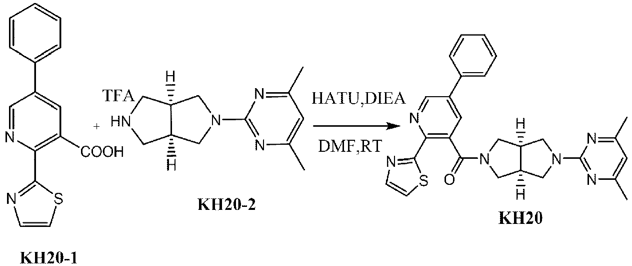

**Compound KH20:** compound KH20-2 (124 mg, 2 eq), HATU (142 mg, 2 eq) and DIEA (241 mg, 10 eq) were successively added to a solution of compound **KH20-1** (53 mg, 1 eq) in DMF (6 mL), and after the addition was complete, the mixture was stirred at room temperature overnight. After the reaction was complete, water (30 mL) was added for dilution, the mixture was extracted with ethyl acetate (30 mL×2), and organic phases were collected. The organic phases were dried with anhydrous sodium sulfate, filtered and concentrated to obtain a crude product. The crude product was purified by column chromatography to obtain **KH20.** LCMS (ESI, *m*/*z):* 483.3 [M + 1]⁺.

### Example 21 Synthesis of compound KH21

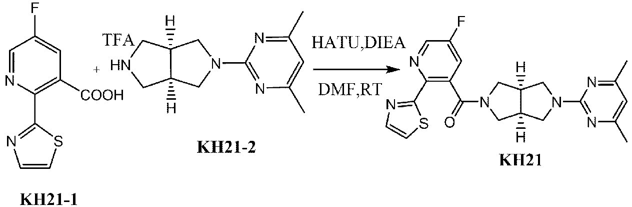

**Compound KH21:** KH21-2 (119 mg, 2 eq), HATU (136 mg, 2 eq) and DIEA (70 mg, 3 eq) were successively added to a solution of compound **KH21-1** (40 mg, 1 eq) in DCM (5 mL), and after the addition was complete, the mixture was stirred at room temperature overnight. After the reaction was complete, water (50 mL) was added for dilution, the mixture was extracted with dichloromethane (100 mL), and the organic phase was collected. The organic phase was dried with anhydrous sodium sulfate, filtered and concentrated to obtain a crude product. The crude product was purified by column chromatography to obtain **KH21.** LCMS (ESI, *m*/*z*): 425 .1[M + 1]⁺.

### Example 22 Synthesis of compound KH22

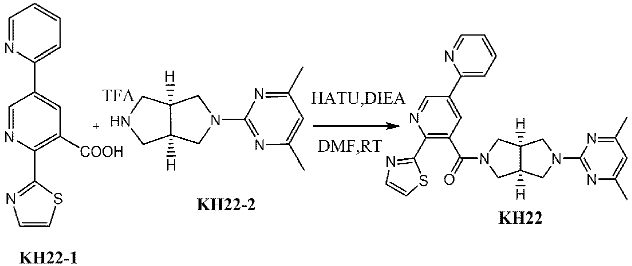

**Compound KH22:** HATU (161 mg, 2 eq), DIEA (273 mg, 10 eq) and KH22-2 (140 mg, 2 eq) were successively added to a solution of compound **KH22-1** (60 mg, 1 eq) in DMF (6 mL), and after the addition was finished, the mixture was stirred at room temperature overnight. After the reaction was complete, water (20 mL) was added for dilution, the mixture was extracted with ethyl acetate (30 mL×2), and organic phases were collected. The organic phases were dried with anhydrous sodium sulfate, filtered and concentrated to obtain a crude product. The crude product was purified by column chromatography to obtain **KH22.** LCMS (ESI, m/z): 484.2 [M + 1]⁺.

### Example 23 Synthesis of compound KH25

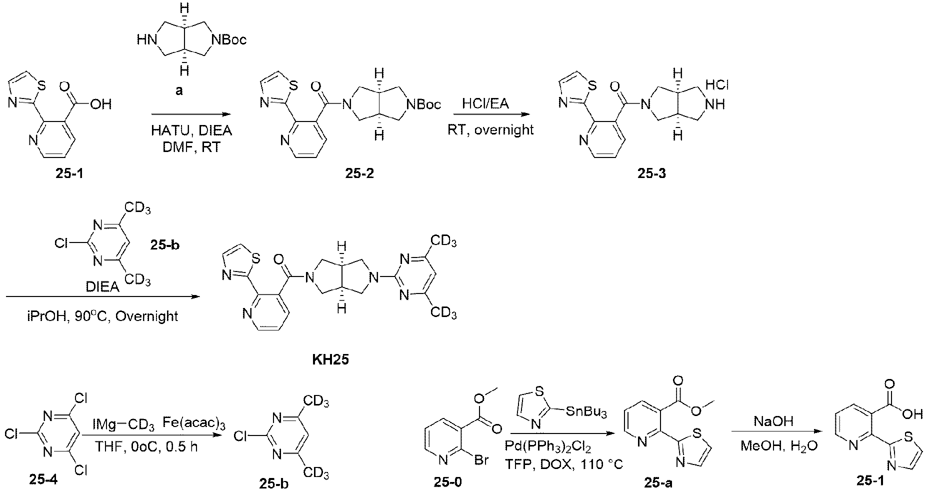

**Compound** 25-b: Fe(acac)₃ (192 mg, 0.55 mmol) was added to a solution of compound 25-4 (1 g, 5.45 mmol) in THF (10 mL) at room temperature, the reaction system was cooled to 0°C and deuterated methylmagnesium iodide (1.0 M) (13.6 mL, 13.63 mmol) was added, and the mixture was allowed to react at 0°C for 1 hour. After the reaction was complete, a saturated aqueous ammonium chloride solution (50 mL) was added to quench the reaction. The mixture was extracted with ethyl acetate (50 mL×3), the organic phases were washed with saturated brine (50 mL×2), dried with anhydrous sodium sulfate and filtered, and concentrated in vacuum under reduced pressure to obtain a crude product. The crude product was purified by silica gel column chromatography (petroleum ether/ethyl acetate=10 : 1) to obtain a colorless oily substance (580 mg, yield 71.6%). LCMS (ESI, m/z): 149.2 [M + H]⁺. ¹H NMR (400 MHz, DMSO-d₆) δ 7.33 (s, 1H).

**Compound 25-a**: **25-0** (5.71 g, 26.4 mmol) and 1,4-dioxane (80 mL) were added to a reaction flask, and then 2-(tributylstannyl)thiazole (9.9 g, 26.4 mmol), bis(triphenylphosphine)palladium(II) dichloride (1.85 g, 2.6 mmol) and tris(tetrahydrofuran-2-yl)phosphine (1.25 g, 5.4 mmol) were added. Under nitrogen protection, the mixture was heated to 110°C and stirred for 16 hours. The reaction solution was cooled to room temperature, and evaporated under reduced pressure to remove the solvent, so as obtain a crude product. The crude product was purified by silica gel column chromatography [eluent: petroleum ether-methyl tert-butyl ether (100: 0-60: 40)]. An eluate was collected, and evaporated under reduced pressure to remove the solvent, so as to obtain a colorless oily substance **25-a** (4.2 g, yield: 68%). LCMS purity: 95.50% MS Calculated: 220.0; MS Found: 221.3 [M+H] ⁺.

**Compound 25-1**: **25-a** (2.00 g, 9.1 mmol) was dissolved in a mixed solution of methanol (18 mL) and water (9 mL). NaOH (726 mg, 18.2 mmol) was added to a reaction flask, and under nitrogen protection, the mixture was stirred at room temperature for 1 hour. After the reaction was complete, 2 mol/L hydrochloric acid was added to adjust pH to be 5. The mixture was filtered, and the filter cake was washed with a small amount of water. The filtrate was purified by C18 reverse-phase column chromatography [eluent: water-acetonitrile (100: 0-95: 5)]. An eluate was collected, and evaporated under reduced pressure to remove the acetonitrile. The residue was mixed with the filter cake and then lyophilized to obtain a light yellow solid **25-1** (2.3 g, yield: 100%). LCMS (ESI, *m*/*z*) purity: 100%; MS Calculated: 206.2; MS Found: 207.1 [M+H] ⁺.

**Compound 25-2**: DIEA (0.24 mL, 1.45 mmol), HATU (188 mg, 0.73 mmol) and compound **a** (123 mg, 0.58 mmol) were added to a solution of compound **25-1** (100 mg, 0.49 mmol) in DMF (3 mL) at room temperature, and the mixture was allowed to react at room temperature for 2 hours. After the reaction was complete, water (10 mL) was added to the reaction solution, and the mixture was extracted with ethyl acetate (15 mL×3). The organic phases were washed with saturated brine (20 mL×2), dried with anhydrous sodium sulfate, and concentrated in vacuum under reduced pressure to obtain a crude product. The crude product was purified by silica gel column chromatography (ethyl acetate) to obtain a yellow oily substance (120 mg, yield 61.7%). LCMS (ESI, *m*/*z*): 401.1 [M + H]⁺.

**Compound 25-3**: a solution of hydrogen chloride in ethyl acetate (2 M) (2 mL) was added to a solution of compound 25-2 (120 mg, 0.30 mmol) in ethyl acetate (2 mL), and the mixture was allowed to react at room temperature overnight. After the reaction was complete, the reaction solution was concentrated in vacuum under reduced pressure to obtain a white solid crude product (90 mg). LCMS (ESI, *m*/*z*): 301.0 [M + H]⁺.

**Compound KH25**: compound **25-b** (41 mg, 0.27 mmol) and DIEA (0.12 mL, 0.75 mmol) were added to a solution of compound **25-3** (75 mg, 0.25 mmol) in isopropanol (2 mL), and the mixture was allowed to react at 90°C overnight. After the reaction was complete, the reaction solution was concentrated in vacuum under reduced pressure to obtain a crude product. The crude product was purified by preparative reverse-phase chromatography to obtain a white solid compound (35.06 mg, yield 34.0%). LCMS (ESI, *m*/*z*): 413.2 [M + H]⁺. ¹H NMR (400 MHz, DMSO-d₆) δ 8.70 - 8.65 (m, 1H), 7.94 - 7.76 (m, 3H), 7.58 - 7.52 (m, 1H), 6.38 (s, 1H), 3.77 - 3.73 (m, 1H), 3.60 - 3.48 (m, 3H), 3.43 - 3.34 (m, 3H), 3.04 - 2.89 (m, 3H).

### Example 24 Synthesis of compound KH26

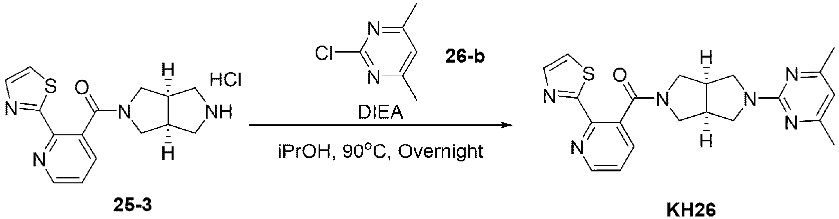

**Compound KH26:** compound **26-b** (90 mg, 0.413 mmol), HATU (188 mg, 0.496 mmol) and DIEA (160 mg, 1.24 mmol) were added to a solution of compound **25-3** (94 mg, 0.454 mmol) in DMF (2 mL), and the mixture was allowed to react at room temperature. After the reaction was complete, the reaction solution was diluted with water (20 mL), and extracted with ethyl acetate (15 mL×3). The organic phases were washed with saturated brine (20 mL), dried with anhydrous sodium sulfate, filtered, concentrated, and then prepared by Prep-HPLC to obtain a white solid (42.04 mg, 25.0%). LCMS (ESI, *m*/*z*): 407.1 [M+H]⁺. ¹H NMR (400 MHz, DMSO) δ 8.67 (m, *J* = 4.8, 1.6 Hz, 1H), 7.85 (m, *J* = 7.7, 1.6 Hz, 3H), 7.55 (m, *J* = 7.7, 4.8 Hz, 1H), 6.38 (s, 1H), 3.75 (m, *J* = 11.5, 7.6 Hz, 2H), 3.55 (s, 5H), 3.06 - 2.83 (m, 3H), 2.22 (s, 6H).

### Example 25 Synthesis of compound KH27

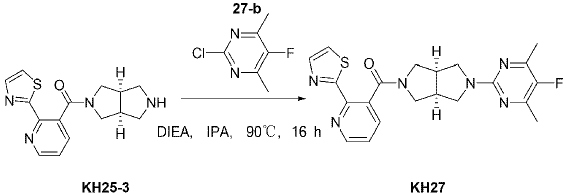

**Compound KH27:** compound **27-b** (53 mg, 0.33 mmol) and diisopropylethylamine (0.16 mL, 1.00 mmol) were added to a solution of compound **KH25-3** (100 mg, 0.33 mmol) in isopropanol (3 mL), and the mixture was allowed to react at 90°C overnight. After the reaction was complete, the reaction solution was concentrated in vacuum under reduced pressure to obtain a crude product. The crude product was purified by preparative reverse-phase chromatography (FA) to obtain a white solid compound (23.19 mg, yield 16.4 %). LCMS (ESI, *m*/*z):* 425.3 [M + 1]⁺. ¹H NMR (400 MHz, DMSO-d6) δ 8.67 (s, 1H), 7.97 - 7.70 (m, 3H), 7.55 (s, 1H), 3.80 - 3.66 (m, 2H), 3.59 - 3.45 (m, 3H), 3.35 - 3.27 (m, 2H), 3.05 - 2.84 (m, 3H), 2.27 (s, 6H).

### Example 26 Synthesis of compound KH28

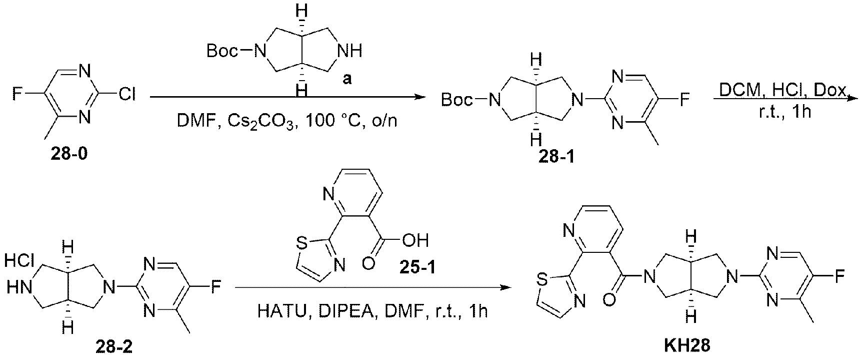

**Synthesis of compound 28-1: 28-0** (646 mg, 4.45 mmol), N,N-dimethylformamide (20 mL), compound a (942 mg, 4.45 mmol) and cesium carbonate (2.89 g, 8.9 mmol) were successively added to a reaction flask. Under nitrogen protection, the mixture was stirred in an oil bath at 100°C to react overnight. After the reaction was complete, the reaction solution was cooled to room temperature, and water was added until the reaction solution became clear. The mixture was extracted with ethyl acetate (50 mL×3), and the organic phases were combined, washed twice with a saturated ammonium chloride solution, dried with anhydrous magnesium sulfate, and evaporated under reduced pressure to remove the solvent, so as to obtain a crude product. The crude product was purified by silica gel column chromatography [eluent: petroleum ether-methyl tert-butyl ether (100: 0-40: 60)]. An eluate was collected, and evaporated under reduced pressure to remove the solvent, so as to obtain a yellow solid **28-1** (1.2 g, yield: 83%). LCMS(ESI, *m*/*z*) purity: 100.00%; MS Calculated: 322.1; MS Found: 323.0 [M+H]⁺.

**Synthesis of compound 28-2**: **28-1** (700 mg, 2.17 mmol) and dichloromethane (5 mL) were added to a reaction flask, and after full dissolution, a solution of hydrochloric acid in dioxane (60 mL) was added. Under nitrogen protection, the mixture was stirred at room temperature to react for 1 hour. After the reaction was complete, the reaction solution was evaporated under reduced pressure to remove the solvent, so as to obtain a crude product, which was a brown solid **28-2** (650 mg, yield: 100%). LCMS (ESI, *m*/*z*) purity: 100%; MS Calculated: 222.2; MS Found: 223.4 [M+H]⁺.

**Synthesis of compound KH28**: **28-2** (482 mg, 2.17 mmol) was dissolved in N,N-dimethylformamide (6 mL) in a reaction flask, **25-1** (447 mg, 2.17 mmol), DIPEA (1122 mg, 8.70 mmol) and HATU (1.24 g, 3.25 mmol) were successively added, and under nitrogen protection, the mixture was stirred at room temperature to react for 2 hours. After the reaction was complete, water was added to the reaction solution, and the mixture was extracted with ethyl acetate (40 mL×5). The organic phases were combined, washed twice with a saturated ammonium chloride solution, dried with anhydrous magnesium sulfate, and evaporated under reduced pressure to remove the solvent, so as to obtain a crude product. The crude product was purified by silica gel column chromatography [eluent: dichloromethane-methanol (100: 0-15: 85)]. An eluate was collected, and evaporated under reduced pressure to remove the solvent, so as to obtain a crude product with 90% purity. The crude product was purified by C18 reverse-phase column chromatography [eluent: water-acetonitrile (100: 0-40: 60)]. An eluate was collected, and evaporated under reduced pressure to remove the acetonitrile. The residue was mixed with the filter cake and then lyophilized to obtain **KH28** (582 mg, yield: 65%). LCMS (ESI, *m*/*z*)purity: 100.00% ; MS Calculated: 410.2; MS Found: 411.0 [M+H] ⁺.¹H NMR (400 MHz, CD₃OD) δ: 8.66 (dd, *J =*5.2, 1.6 Hz*,* 1H), 8.08 (d, *J =* 2.0 Hz, 1H), 7.83-7.81 (m, 2H), 7.62 (s, 1H), 7.51 (dd, *J* = 8.0, 5.2 Hz, 1H), 3.93-3.89 (m, 1H), 3.85-3.80 (m, 1H), 3.73-3.63 (m, 4H), 3.48-3.40 (m, 1H), 3.16-3.12 (m, 1H), 3.10-3.03 (m, 2H), 2.36 (d, *J* = 2.8 Hz, 3H).

### Example 27 Synthesis of compound KH29

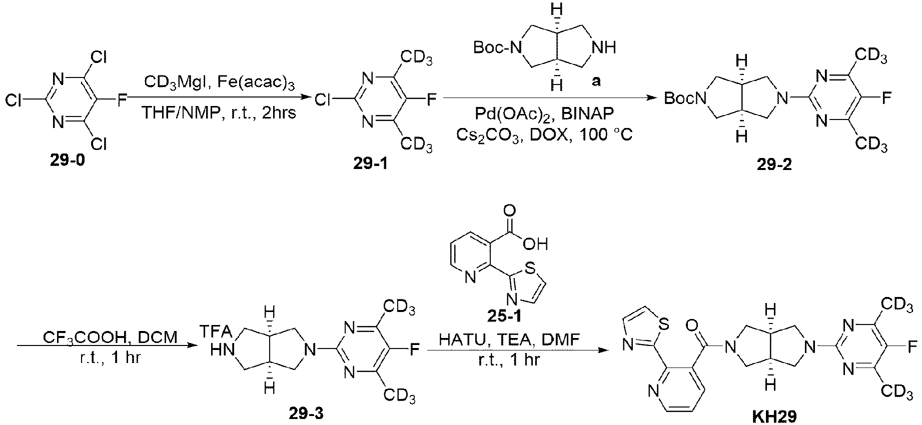

**Synthesis of compound 29-1: 29-0** (1 g, 4.97 mmol) and Fe(acac)₃ (176 mg, 0.5 mmol) were successively added to a mixed solution of THF (15 mL) and NMP (1.5 mL) in a single-neck flask, and then the mixture was cooled to 0°C in ice water and CD₃MgI (12 mL, 12 mmol) was added slowly. Under nitrogen protection, the mixture was stirred at room temperature for 2 hours. After the reaction was complete, water was added, and the mixture was extracted with MTBE (50 mL×3). The organic phases were combined, and evaporated under reduced pressure to remove the solvent, so as to obtain a crude product. The crude product was purified by silica gel column chromatography [eluent: petroleum ether-ethyl acetate (100: 0-70: 30)]. An eluate was collected, and evaporated under reduced pressure to remove the solvent, so as to obtain a colorless oily substance **29-1** (570 mg, yield: 69%).

**Synthesis of compound 29-2: 29-1** (570 mg, 3.43 mmol), 1,4-dioxane (12 mL), compound a (728 mg, 3.43 mmol), palladium acetate (77 mg, 0.34 mmol), BINAP (427 mg, 0.69 mmol) and cesium carbonate (2231 mg, 6.86 mmol) were successively added to a reaction flask. Under nitrogen protection, the mixture was stirred in an oil bath at 100°C to react overnight. After the reaction was complete, the reaction solution was cooled to room temperature, and subjected to suction filtration in vacuum. A filtrate was collected, and evaporated under reduced pressure to remove the solvent, so as obtain a crude product. The crude product was purified by silica gel column chromatography [eluent: petroleum ether-methyl tert-butyl ether (100: 0-40: 60)]. An eluate was collected, and evaporated under reduced pressure to remove the solvent, so as to obtain a white solid **29-2** (600 mg, yield: 51%). LCMS(ESI, m/z) purity: 87.00% ; MS Calculated: 342.2; MS Found: 343.8 [M+H]⁺.

**Synthesis of compound 29-3:** 29-2 (140 mg, 0.41 mmol) and dichloromethane (1 mL) were added to a reaction flask, and after full dissolution, trifluoroacetic acid (4 mL) was added. Under nitrogen protection, the mixture was stirred at room temperature to react for 1 hour. After the reaction was complete, the reaction solution was evaporated under reduced pressure to remove the solvent, so as to obtain a crude product, which was a yellow solid **29-3** (90 mg, yield: 91%). LCMS(ESI, m/z) purity: 99.99% ; MS Calculated: 242.2; MS Found: 243.3 [M+H] ⁺.

**Synthesis of compound KH29: 29-3** (90 mg, 0.37 mmol) was dissolved in N,N-dimethylformamide (2 mL) in a reaction flask, and **25-1** (76 mg, 0.37 mmol), HATU (212 mg, 0.56 mmol) and DIPEA (120 mg, 0.93 mmol) were successively added. Under nitrogen protection, the mixture was stirred at room temperature to react for 1 hour. After the reaction was complete, the reaction solution was poured into water, and extracted with ethyl acetate (40 mL×3). The organic phases were combined, washed with a saturated ammonium chloride solution, dried with anhydrous magnesium sulfate, and evaporated under reduced pressure to remove the solvent, so as to obtain a crude product. The crude product was purified by preparative HPLC to obtain **KH29** (70 mg, yield: 44%). LCMS(ESI, *m*/*z*) purity: 99.44%, Rt = 1.939 min; MS Calculated: 430.2; MS Found: 431.2 [M+H]⁺.HPLC (method J) purity: 99.76%, Rt = 8.521 min.¹H NMR (400 MHz, DMSO-*d*₆) δ: 8.67 (dd, *J* =4.8, 1.6 Hz, 1H), 7.86-7.83 (m, 3H), 7.55 (dd, *J* ₁=7.6, 4.8 Hz, 1H), 3.75-3.69 (m, 2H), 3.53-3.51 (m, 4H), 3.45-3.38 (m, 1H), 3.03-2.99 (m, 1H), 2.91 (brs, 2H).

### Example 28 Synthesis of compound KH30

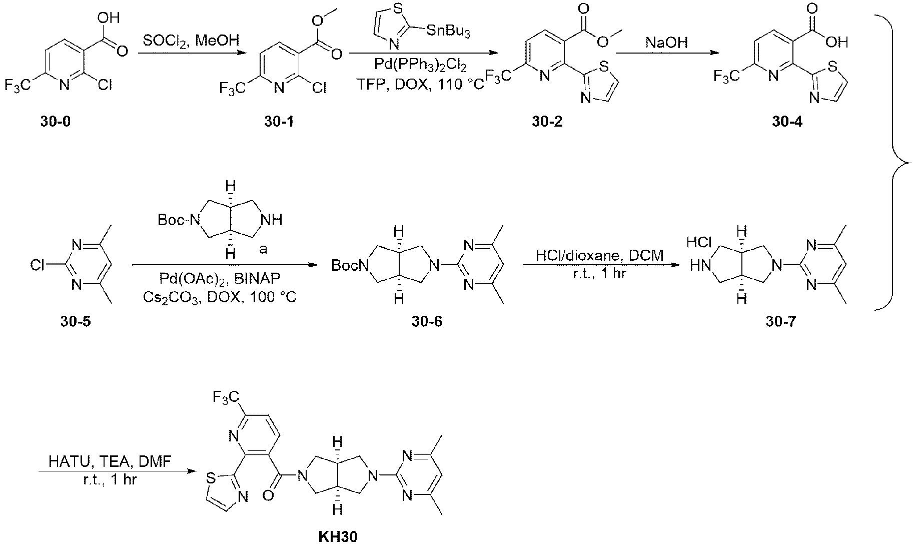

**Synthesis of compound 30-1: 30-0** (2.5 g, 11.1 mmol) and methanol (50 mL) were added to a reaction flask, and stirred evenly, and then thionyl chloride (2.64 g, 22.2 mmol) was dropwise added. After the dropwise addition was finished, the mixture was heated to 60°C in an oil bath and stirred for 2 hours. The mixture was cooled to room temperature, and evaporated under reduced pressure to remove the solvent, so as to obtain a crude product. The crude product was purified by silica gel column chromatography [eluent: petroleum ether-methyl tert-butyl ether (100: 0-30: 70)]. An eluate was collected, and evaporated under reduced pressure to remove the solvent, so as to obtain a yellow solid **30-1** (2.1 g, yield: 79%). LCMS (ESI, *m*/*z*) purity: 94%, Rt = 0.709 min; MS Calculated: 239.0; MS Found: 240.0 [M+H] ⁺.

**Synthesis of** c**ompound 30-2**: **30-1** (2.1 g, 8.78 mmol) and 1,4-dioxane (30 mL) were added to a reaction flask, and then 2-(tributylstannyl)thiazole (3.6 g, 9.6 mmol), bis(triphenylphosphine)palladium(II) dichloride (617 mg, 0.88 mmol) and tris(tetrahydrofuran-2-yl)phosphine (411 mg, 1.77 mmol) were successively added. Under nitrogen protection, the mixture was heated to 110°C and stirred for 16 hours. The reaction solution was cooled to room temperature, and evaporated under reduced pressure to remove the solvent, so as obtain a crude product. The crude product was purified by silica gel column chromatography [eluent: petroleum ether-methyl tert-butyl ether (100: 0-50: 50)]. An eluate was collected, and evaporated under reduced pressure to remove the solvent, so as to obtain a colorless oily substance **30-2** (2 g, yield: 79%). LCMS (ESI, *m*/*z*)purity: 65.91%, Rt = 1.906 min; MS Calculated: 288.0; MS Found: 288.9 [M+H]⁺.

**Synthesis of compound 30-4**: **30-2** (2.00 g, 6.9 mmol) was dissolved in a mixed solution of methanol (16 mL) and water (4 mL). **NaOH (**1.1 mg, 27.5 mmol) was added to a reaction flask, and under nitrogen protection, the mixture was stirred at room temperature for 2 hours. After the reaction was complete, the solvent was evaporated to dryness, and 2 mol/L hydrochloric acid was added to adjust pH to be 4. A large amount of white solid was precipitated and filtered. The filter cake was washed with a small amount of water, and dried in vacuum to obtain a light white solid **30-4** (2.0 g, yield: 100%). LCMS (ESI, *m*/*z*) purity: 100%, Rt = 0.499 min; MS Calculated: 274.00; MS Found: 275.1 [M+H]⁺.

**Synthesis of compound 30-6**: **30-5** (250 mg, 1.76 mmol), 1,4-dioxane (10 mL), compound **a** (373 mg, 1.76 mmol), palladium acetate (40 mg, 0.17 mmol), 1,1'-Binaphthyl-2,2'-bis(diphenylphosphine)(219 mg, 0.34 mmol) and cesium carbonate (1.14 g, 3.5 mmol) were successively added to a reaction flask. Under nitrogen protection, the mixture was stirred at 100°C to react for 16 hours. After the reaction was complete, the reaction solution was filtered, the filter cake was washed with methanol until no UV fluorescence was observed in the filtrate, and the filtrate was evaporated under reduced pressure to remove the solvent, so as obtain a crude product. The crude product was purified by silica gel column chromatography [eluent: petroleum ether-methyl tert-butyl ether (100: 0-40: 60)]. An eluate was collected, and evaporated under reduced pressure to remove the solvent, so as to obtain a yellow oily substance **30-6** (350 mg, yield: 62%). LCMS (ESI, *m*/*z*) purity: 80%, Rt = 0.940 min; MS Calculated: 318.2; MS Found: 319.3 [M+H]⁺.

**Synthesis of compound 30-7**: **30-6** (210 mg, 0.96 mmol) and dichloromethane (2 mL) were added to a reaction flask, and after full dissolution, a solution of hydrochloric acid in dioxane (10 mL) was added. Under nitrogen protection, the mixture was stirred to react for 1 hour. After the reaction was complete, the reaction solution was evaporated under reduced pressure to remove the solvent, so as to obtain a crude product, which was a brown solid **30-7** (200 mg, yield: 100%). The crude product was directly used in the next step of reaction. LCMS (ESI, *m*/*z*) purity: 100%, Rt = 0.530 min; MS Calculated: 218.1; MS Found: 219.3 [M+H]⁺.

**Synthesis of compound KH30**: 30-7 (140 mg, 0.66 mmol), N,N-dimethylformamide (6 mL), 30-4 (127 mg, 0.46 mmol), 2-(7-azabenzotriazol-1-yl)-N,N,N',N'-tetramethyluronium hexafluorophosphate (320 mg, 0.84 mmol) and triethylamine (400 mg, 3.96 mmol) were successively added to a reaction flask. Under nitrogen protection, the mixture was stirred at room temperature to react for 1 hour. After the reaction was complete, the reaction solution was poured into water, and extracted with ethyl acetate (50 mL×4). The organic phases were combined, washed with a saturated ammonium chloride solution, dried with anhydrous magnesium sulfate, and evaporated under reduced pressure to remove the solvent, so as to obtain a crude product. The crude product was purified by preparative HPLC to obtain **KH30** (130.68 mg, yield: 59%). LCMS (ESI, *m*/*z*) purity: 99.42%, Rt = 1.919 min; MS Calculated: 474.1; MS Found: 475.0 [M+H] +. ¹H NMR (400 MHz, DMSO-d6) δ: 8.19 (d, J = 7.6 Hz, 1H), 8.06 (d, J = 8.0 Hz, 1H), 7.94-7.83 (m, 2H), 6.38 (s, 1H), 3.78-3.74 (m, 2H), 3.56-3.37 (m, 5H), 3.07-2.93 (m, 3H), 2.22 (m, 6H).

### Comparative Example 1: synthesis of compound A

The compound A was synthesized according to the method described in Example 476 of WO2012145581A1, and had a structure as follows.

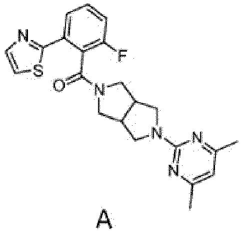

### Comparative Example 2: synthesis of compound B

The compound B was synthesized according to the method described in Example 237 of WO2012145581A1, and had a structure as follows.

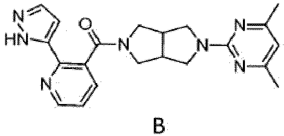

### Example 29: In-vitro cellular activity assay

### 1. Cell culture and reagent preparation

Experimental reagents and materials

| Material | Supplier | Catalog No. |
|---|---|---|
| Fetal bovine serum (FBS, 500 mL) | Gibco | 10999141 |
| Penicillin-Streptomycin liquid (100 mL, 100X) | Gibco | 15140122 |
| Ham's F-12K medium (500 mL) | Hyclone | SH30526.01 |
| 1×phosphate buffer (pH 7.2-7.4, 500 mL) | Solarbio | P1020-500 |
| 1X TrypLE Express Enzyme, no phenol red (500 mL) | ThermoFisher Scientific | 12604021 |
| Hygromycin B Gold solution (5 g, 1 x 50 mL, 100 mg/mL) | Invivogen | ant-hg-5 |
| Hank's balanced salt solution, with calcium and magnesium, no phenol red (1000 mL) | Gibco | 14025076 |
| 4-(2-hydroxyethyl)piperazine-1-ethanesulfonic acid buffer (HEPES, 1 M, 100 mL) | Gibco | 15630080 |
| FLIPR Calcium 6 Assay Kit (Bulk Kit, 1 vial=10 plates x 10 vials) | Molecular Devices | R8191 |
| 384-well plate for cell culture (black transparent bottom plate, with lid, sterility, 100 plates/case) | Corning | 3764 |
| 384-well polypropylene microplate qualified by Echo Acoustic Liquid Handling System, with transparent and flat bottom (100 plates/case) | LABCYTE | PP-0200 |
| 384-well plate for compound dilution (standard, transparent, round, 100 plates/case) | Corning | 3657 |
| 384-well FLIPR Tetra pipette tip (50 boxes/case) | Molecular Devices | 9000-0764 |
| RNase/DNase-free 96-well conical bottom PP plate (120 plates/case) | ThermoFisher Scientific | 249944 |
| Cell line: Flp-In-CHO-OX1/OX2 | Pharmaron | Prepared in-house |
| Water-soluble probenecid (10 x 77 mg) | Invitrogen | P36400 |
| Orexin A (human, rat, mouse) | TOCRIS | 1455 |

| | | |
|---|---|---|
| Cell line: Flp-In-CHO-OX1/OX2 Complete medium: Ham's F-12K + 10% FBS + 1x Penicillin-Streptomycin (PS) + 600 µg/ml Hygromycin B Cell seeding medium: Ham's F-12K + 10% FBS Experimental buffer: 1X HBSS + 20mM HEPES Assay kit: FLIPR Calcium 6 Assay Kit | | |

### 2. Experimental method

### 2.1 Determination of IC₅₀ value of test compound

1) Flp-In-CHO-OX1/OX2 stable pool cell lines were cultured in a complete medium at 37°C in a 5% CO₂ incubator, and maintained at 70%-90% confluency.
2) The cells were dissociated with trypsin (TrypLE Express, ThermoFisher Scientific), then resuspended in a seeding medium, and seeded into a 384-well cell culture plate (Corning, 3764) at a density of 6500 cells/well in a volume of 25 µL for OX1, and 7000 cells/well in a volume of 25 µL for OX2. Subsequently, the cells were cultured at 37°C in 5% CO₂ overnight.
3) 20× Component A (calcium indicator probe provided in a kit, FLIPR Calcium 6 Assay Kit, Molecular Devices) was thawed to room temperature, diluted with an experimental buffer to a 2× working concentration, containing 5 mM probenecid, and kept at room temperature until use.
4) The cell culture plate was taken out and allowed to stand at room temperature for 10 minutes. A concentration of fetal calf serum (FBS, Gibco) was diluted to 0.03% using an Apricot (384-channel liquid handling workstation) and the experimental buffer, and 20 µL of buffer was finally left in the 3764 culture plate. Subsequently, 20 µL of 2× Component A containing 5 mM probenecid was added to each experimental well. The cells were centrifuged at room temperature at 200 g for 3-5 seconds, and then incubated at 37°C for 2 hours.
5) 6× working solutions of positive control compound and test compound were prepared: compounds were serially diluted 3-fold in DMSO at 10 concentrations, wherein the starting concentration of the positive control compound was 1 mM, and the starting concentration of the test compound was 50 mM; and subsequently, 240 nL of each serially diluted compound above was transferred to a 384-well working plate using an Echo 550, 40 µL of buffer was added, and then the mixture was shaken for 20 minutes, mixed evenly and kept at room temperature until use.
6) The cell culture plate was taken out and allowed to stand at room temperature for 10 minutes. 10 µL of each 6× working solution of the positive control compound and the test compound in step 5) was added to the corresponding experimental well of the 384-well cell culture plate, and the cells were incubated at room temperature for 30 minutes.
7) For an orexin type-1 receptor, Orexin A was diluted to 18 nM (6×) with the experimental buffer, and 50 µL was transferred to a 384-well plate (Corning, 3657) and kept at room temperature until use; and for an orexin type-2 receptor, Orexin A was diluted to 12 nM (6×) with the experimental buffer, and 50 µL was transferred to a 384-well plate (Corning, 3657) and kept at room temperature until use.
8) 10 µL of diluted Orexin A in step 7) was added to each experimental well using FLIPR Tetra (Molecular Devices), and data were collected.

### 2.2 Data analysis

Fluorescence signal values/relative optical baseline (ROB) values were collected by FLIPR Tetra, and the maximum ROB value was taken. Percentage inhibition data were calculated according to readings of negative control (0.1% DMSO) and positive control (1,000 nM positive control Filorexant): inhibition %=100-(ROBₛₐₘₚₗₑ-ROB_{Min})/(ROBₘₐₓ-ROB_{Min})x100. IC₅₀ values were calculated by fitting the percentage inhibition and compound data at different concentrations to a nonlinear four-parameter logistic formula using Prism 8.

### 2.3 Experimental results

**Table 1 OX2/OX1 receptor antagonistic effects of different compounds**

| Example | OX2 IC₅₀ (nM) | OX1 IC₅₀ (nM) | Selectivity (OX1/OX2) |
|---|---|---|---|
| 3 | 52.94 | 4291 | 81.05 |
| 13 | 95.46 | 3990 | 41.80 |
| 14 | 75.84 | 3880 | 51.16 |
| 15 | 57.69 | 1819 | 31.53 |
| 16 | 61.45 | 2097 | 34.12 |
| 17 | 159.60 | 320 | 2.01 |
| 18 | 78.40 | 220 | 2.81 |
| 20 | 219.10 | 910 | 0.24 |
| 25 | 18.98 | 798.3 | 42.06 |
| 26 | 4.13 | 816.1 | 197.60 |
| 27 | 4.46 | 50.8 | 11.39 |
| 28 | 35.38 | 1103 | 31.18 |
| 29 | 4.93 | 82.96 | 16.83 |
| 30 | 93.3 | 2133 | 22.86 |
| Seltorexant | 134.50 | 2130 | 15.83 |

### Example 30 Animal pharmacodynamic study

**Experimental objective:** pharmacodynamic effects of test compounds on sleep in male SD rats were investigated by telemetry technology.

### Experimental Animals: SD rats (male, 5-6 weeks old, body weight approximately 300 g)

### Administration information:

**Table 2**

| Group | Number of animals per group | Vehicle | Administration regimen (dose \| frequency \| route) | Administrat ion volume (mL/kg) |
|---|---|---|---|---|
| Negative control group | | 20% HP-β-CD aqueous cyclodextrin solution | 0 mg/kg, once daily, oral gavage | |
| KH16 | | | 30 mg/kg, once daily, oral gavage | |
| KH25 | 6 | | 20 mg/kg, once daily, oral gavage | 5 |
| KH27 | | | 20 mg/kg, once daily, oral gavage | |
| KH30 | | | 20 mg/kg, once daily, oral gavage | |

**Experimental method:** the animals were housed under a 12-h light/dark-cycle condition during an acclimation period (light on at 19:00; light off at 07:00). On the day of surgery, the animals were anesthetized with Zoletil (i.p., 20 mg/kg) in combination with xylazine (i.p., 8 mg/kg), and electrodes were implanted by surgery. A recovery period of 7 days was allowed after surgery. 3 days before compound administration, rats were given vehicle via gavage at 9:00 (at 2 hours into the dark phases) to acclimate the drug administration operation. Electroencephalogram (EEG) and electromyogram (EMG) signals were recorded from 1 hour before to 6 hours after vehicle administration, on the day before administration drugs. Subsequently, each group of rats dosed gavage their respective compounds at 9:00 (at 2 hours into the dark phases) on the day of administration drugs. EEG and EMG signals were recorded from 1 hour before to 6 hours after administration. The architectural changes in wake, rapid eye movement (REM) sleep, non-rapid eye movement (NREM) sleep were analyzed after administration (including temporal parameters and latency physiological indicators), wherein, 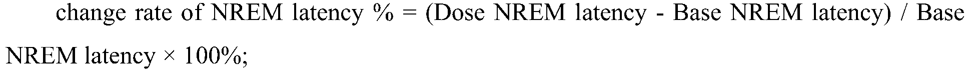 change rate of NREM latency % = (Dose NREM latency - Base NREM latency) / Base NREM latency × 100%; 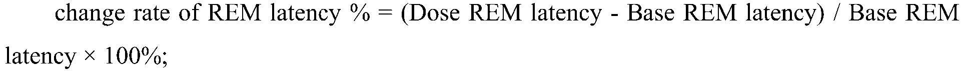 change rate of REM latency % = (Dose REM latency - Base REM latency) / Base REM latency × 100%; 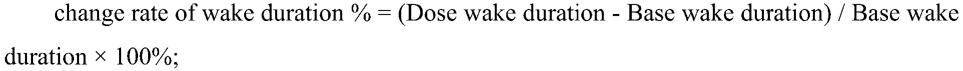 change rate of wake duration % = (Dose wake duration - Base wake duration) / Base wake duration × 100%; 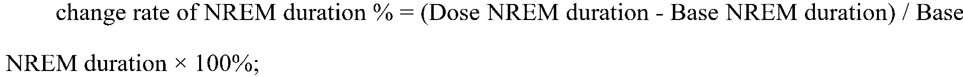 change rate of NREM duration % = (Dose NREM duration - Base NREM duration) / Base NREM duration × 100%; 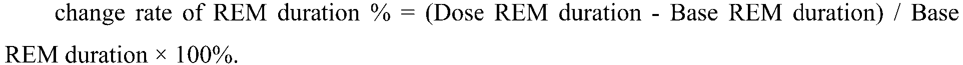 change rate of REM duration % = (Dose REM duration - Base REM duration) / Base REM duration × 100%.

Data analysis: The original data were collected by Ponemah software of DSI system and analyzed by NeuroScore software. The experimental data were represented by Mean±S.E.M. using Graph Pad Prism 8.0 software, One-way ANOVA was used for statistical analysis, and Dunnet post hoc was used for verification. *P or ^{#}P<0.05 indicated a significant difference, **P or ^{##}P<0.01 indicated a very significant difference, and ***P or ^{###}P<0.001 indicated an extremely significant difference.

### Experimental results:

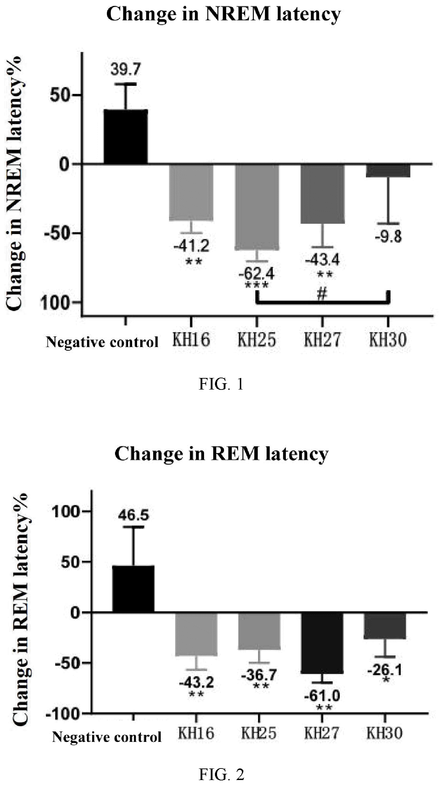

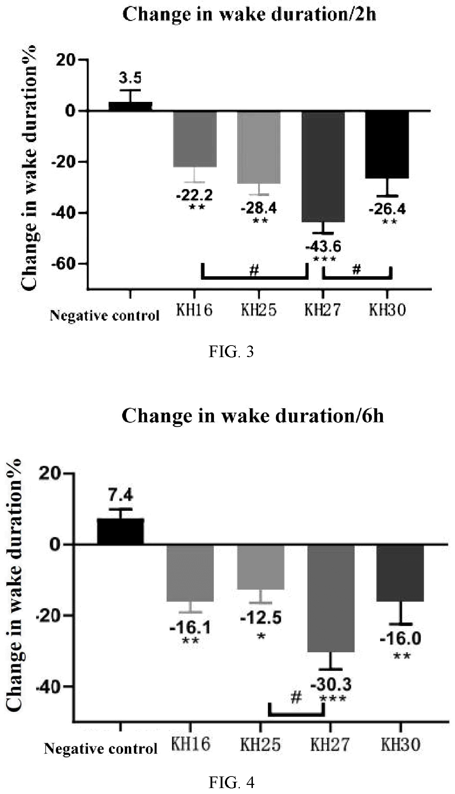

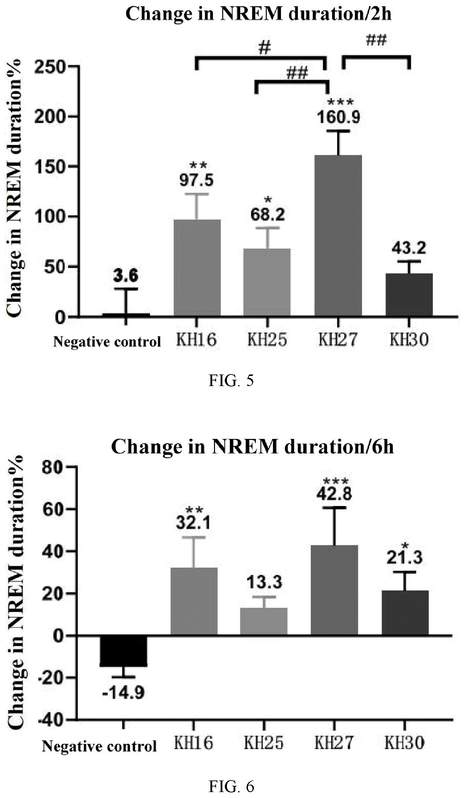

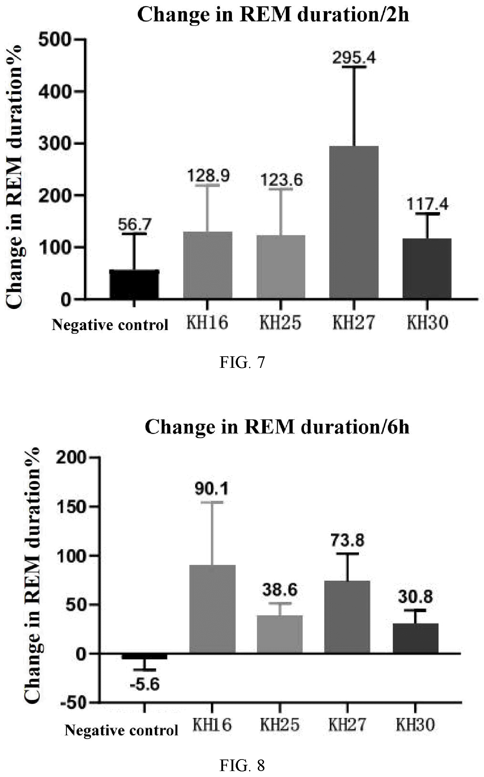

1) Effects of the control group and the drug administration groups on the NREM and REM latency in the male SD rats were shown in FIG. 1 and FIG. 2. As shown in FIG. 1, for the change rate of NREM latency: compared with the negative control group, the drug administration groups of example KH16, KH25 and KH27 compounds all showed significantly reduced in change rates of NREM latency. As shown in FIG. 2, for the change rate of REM latency: compared with the negative control group, the drug administration groups of example KH16, KH25, KH27 and KH30 compounds all showed significantly shortened in REM latency.
2) Effects of the control group and the drug administration groups in the wake duration of the SD rats were shown in FIG. 3 and FIG. 4. Results showed that, compared with the negative control group, the drug administration groups of example KH16, KH25, KH27 and KH30 compounds significantly reduced the wake duration within 2-hour after administered, and the drug administration group of example KH27 compound was significantly superior to other drug administration groups. Compared with the negative control group, all the drug administration groups significantly reduced the wake duration within 6-hour after administered.
3) Effects of the control group and the drug administration groups in the NREM duration (non-rapid eye movement sleep duration) of the SD rats were shown in FIG. 5 and FIG. 6, wherein, results showed that, compared with the negative control group, the drug administration groups of example KH16, KH25 and KH27 compounds significantly increased the NREM duration whitin 2-hour after administered, and the drug administration group of example KH27 compound was significantly superior to other drug administration groups. Compared with the negative control group, the drug administration groups of KH16, KH27 and KH30 compounds all significantly increased the NREM duration whtin 6-hour after administered.
4) Effects of the control group and the drug administration groups in the REM duration (rapid eye movement sleep duration) of the SD rats were shown in FIG. 7 and FIG. 8, wherein, results showed that, compared with the negative control group, all the drug administration groups showed an increasing trend in the REM duration within both 2-hour and 6-hour after administered.

### Example 31 Animal pharmacodynamic study

**Experimental objective:** pharmacodynamic effects of test compounds in sleep of male SD rats were investigated by telemetry technology.

### Experimental animals: SD rats (male, 5-6 weeks old, body weight approximately 300 g)

### Administration information:

**Table 3**

| Group | Number of animals per group | Vehicle | Administration regimen (dose \| frequency \| route) | Administrat ion volume (mL/kg) |
|---|---|---|---|---|
| Negative control group | | 20% HP-β-CD aqueous cyclodextrin solution | 0 mg/kg, once daily, oral gavage | |
| Positive control (seltorexant) | 6 | | 10 mg/kg, once daily, oral gavage | 5 |
| KH27 | | | 10 mg/kg, once daily, oral gavage | |
| KH28 | | | 10 mg/kg, once daily, oral gavage | |
| KH29 | | | 10 mg/kg, once daily, oral gavage | |

**Experimental method:** the animals were housed under a 12-h light/dark-cycle condition during an acclimation period (light on at 19:00; light off at 07:00). On the day of surgery, the animals were anesthetized with Zoletil (i.p., 20 mg/kg) in combination with xylazine (i.p., 8 mg/kg), and electrodes were implanted by surgery. A recovery period of 7 days was allowed after surgery. 3 days before compound administration, rats were given vehicle via gavage at 9:00 (at 2 hours into the dark phases) to acclimate the drug administration operation. Electroencephalogram (EEG) and electromyogram (EMG) signals were recorded from 1 hour before to 6 hours after vehicle administration on the day before administration drugs. Subsequently, each group rats dosed gavage their respective compounds at 9:00 (at 2 hours into the dark phases) on the day of administration drugs. EEG and EMG signals were recorded from 1 hour before to 6 hours after administration. The architectural changes in wake, rapid eye movement (REM) sleep, non-rapid eye movement (NREM) sleep, were analyzed after administration (including temporal parameters and latency physiological indicators), wherein, 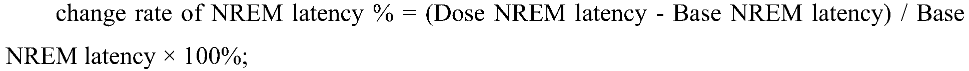 change rate of NREM latency % = (Dose NREM latency - Base NREM latency) / Base NREM latency × 100%; 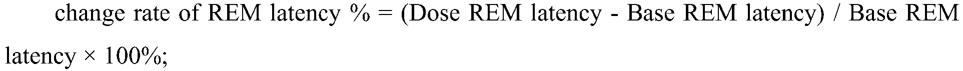 change rate of REM latency % = (Dose REM latency - Base REM latency) / Base REM latency × 100%; 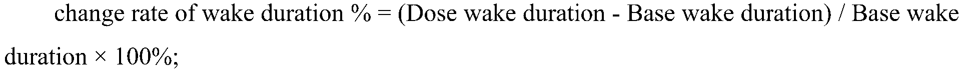 change rate of wake duration % = (Dose wake duration - Base wake duration) / Base wake duration × 100%; 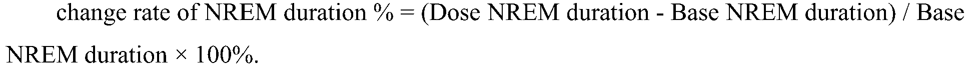 change rate of NREM duration % = (Dose NREM duration - Base NREM duration) / Base NREM duration × 100%. 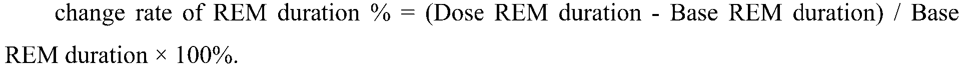 change rate of REM duration % = (Dose REM duration - Base REM duration) / Base REM duration × 100%.

**Data analysis:** The original data were collected by Ponemah software of DSI system and analyzed by NeuroScore software. The experimental data were represented by Mean±S.E.M. using Graph Pad Prism 8.0 software, One-way ANOVA was used for statistical analysis, and Dunnet post hoc was used for verification. *P or ^{#}P<0.05 indicated a significant difference, **P or ^{##}P<0.01 indicated a very significant difference, and ***P or ^{###}P<0.001 indicated an extremely significant difference.

### Experimental results:

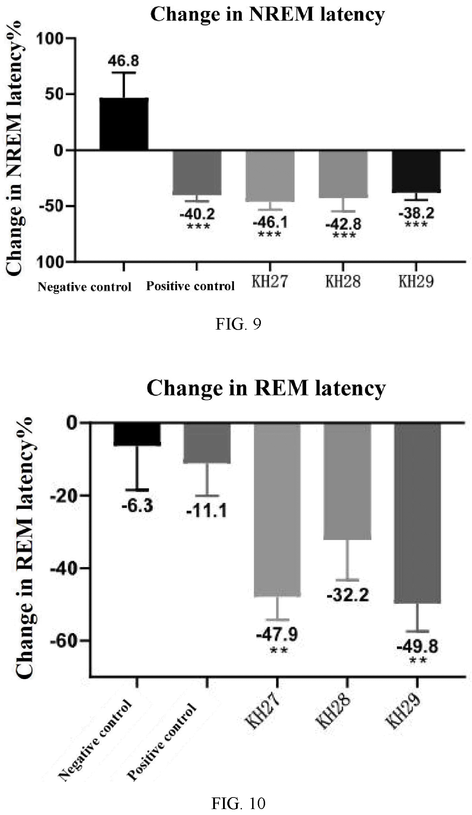

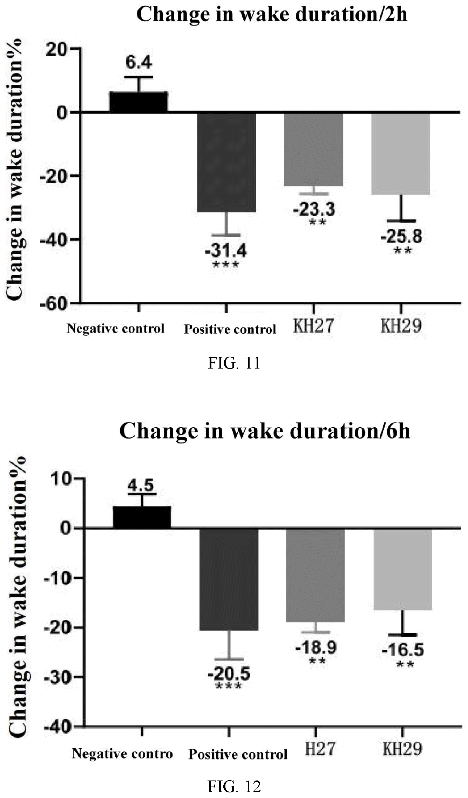

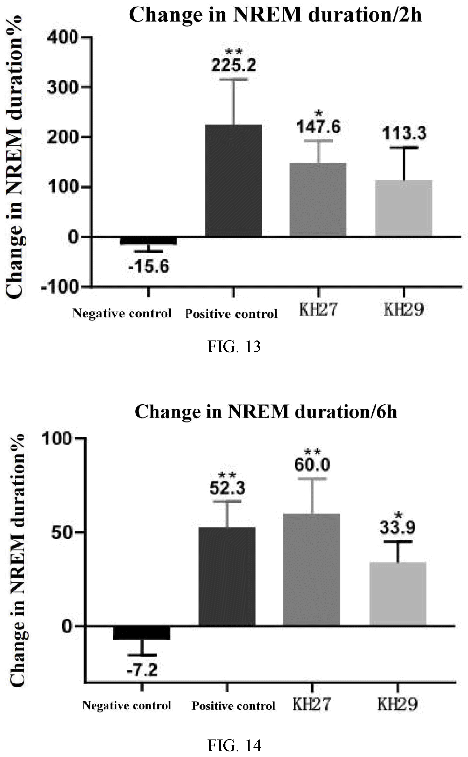

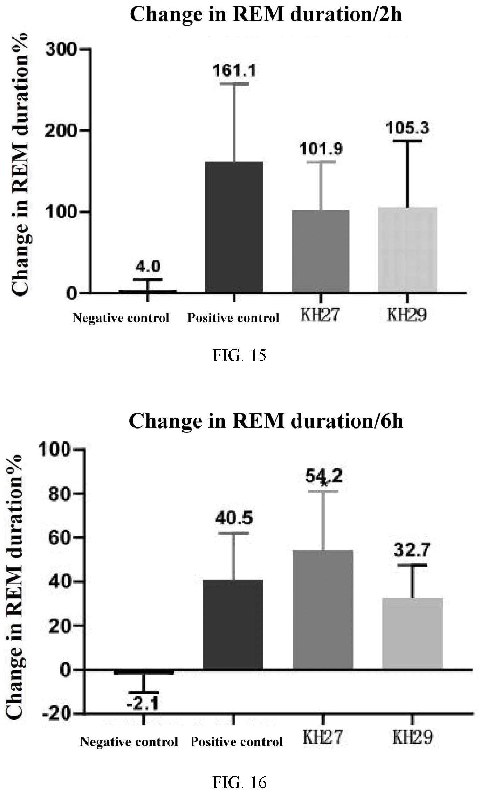

1) Effects of the control group and the drug administration groups on the NREM and REM latency in the male SD rats were shown in FIG. 9 and FIG. 10. As shown in FIG. 9, for the change rate of NREM latency: compared with the negative control group, the drug administration groups of example KH27, KH28 and KH29 compounds all showed significantly reduced change rates of the NREM latency. As shown in FIG. 10, for the change rate of REM latency: compared with the negative control group, the drug administration groups of example KH27 and KH29 compounds both showed significantly shortened in REM latency.
2) Effects of the control group and the drug administration groups on the wake duration of the SD rats were shown in FIG. 11 and FIG. 12. Results showed that, compared with the negative control group, the drug administration groups of example KH27 and KH29 compounds significantly reduced the wake durations within 2-hour and 6-hour after administered.
3) Effects of the control group and the drug administration groups on the NREM duration (non-rapid eye movement sleep duration) of the SD rats were shown in FIG. 13 and FIG. 14, wherein, results showed that, compared with the negative control group, the drug administration group of example KH27 compound significantly increased NREM duration within 2-hour after administered. Compared with the negative control group, the drug administration groups of KH27 and KH29 compounds both significantly increased the NREM duration within 6-hour after administered.
4) Effects of the control group and the drug administration groups on the REM duration (rapid eye movement sleep duration) of the SD rats were shown in FIG. 15 and FIG. 16. Results showed that, compared with the negative control group, the drug administration groups of example KH27 and KH29 compounds both significantly increased the NREM duration within 2 hours after administered. Compared with the negative control group, the drug administration group of KH27 compound significantly increased the NREM duration within 6 hours after administered.

### Example 32 Metabolic stability in human hepatocytes

**Experimental objective:** the metabolic stability of test compounds in human hepatocytes was investigated.

**Experimental materials:** human suspended hepatocytes.

**Experimental operation:** cryopreserved hepatocytes were taken out from a liquid nitrogen container and thawed, and cell viability was determined by trypan blue staining. The hepatocyte suspension was added to a pre-warmed incubation plate, then working solutions of test compounds and control compound were added, mixed evenly and immediately placed on a plate shaker inside an incubator, and a timer was started to initiate the reaction. Incubation time points were set at 0, 15, 30, 60 and 90 minutes for the reaction under incubation conditions of 37°C, saturated humidity, and 5% CO₂. In the reaction system, final concentrations of the test compounds were 1 µM, a final concentration of the control compound was 3 µM, and a final concentration of the hepatocytes was 0.5×10⁶ cells/mL. At the end of each corresponding incubation time point, the incubation plate was taken out, and an appropriate volume of cell suspension was transferred to a sample plate containing a certain volume of stop solution (acetonitrile solution containing 200 ng/mL tolbutamide and labetalol). After sealing, all sample plates were shaken at 600 rpm on a plate shaker for 10 minutes, and then centrifuged at 3220×g for 20 minutes. Supernatants of the test compounds and the control compound were diluted with ultrapure water at a ratio of 1: 3. All samples were mixed evenly and analyzed by LC/MS/MS, and half-lives (T₁/₂) thereof were calculated. Results were shown in Table 4.

**Table 4**

| Compound ID | T½ (min) |
|---|---|
| Positive control (seltorexant) | 24.0 |
| KH16 | 42.3 |
| KH25 | 52.1 |
| KH26 | 88.7 |
| KH28 | 91.9 |
| KH29 | 49.3 |

### Example 33 Pharmacokinetic evaluation of compounds

**Experimental objective:** the pharmacokinetics of test compounds in SD rats---brain tissue drug concentration was investigated.

### Experimental animals: SD rats (male, 6-8 weeks old, body weight 247.48 -255.52 g)

**Experimental operation:** pharmacokinetic characteristics of rodents after oral administration of the compounds were tested by a standard protocol. In the experiment, a corresponding compound was prepared into a 1 mg/mL clear solution, and administered orally to the rats as a single dose. An oral vehicle was 20% HP-β-CD aqueous solution. In the experiment, the male SD rats were administered by oral gavage at a dose of 5 mg/kg. At 0.5, 2 and 4 hours after administration, whole brains were collected. Tissue samples were homogenized in [methanol/15 mM phosphate buffer (volume ratio 1: 2)], with a dilution factor of 6. Finally, sample concentrations were quantitatively analyzed by LC-MS/MS. Results were shown in Table 5.

**Table 5**

| Serial number of compound | Time point (h) | Brain tissue drug concentration |
|---|---|---|
| Positive control (Seltorexant) | 0.5 | 84.3 |
| | 2 | 13.2 |
| | 4 | ND |
| KH16 | 0.5 | 109 |
| | 2 | 45.4 |
| | 4 | 7.87 |
| KH27 | 0.5 | 85 |
| | 2 | 22.7 |
| | 4 | 6.99 |
| Comparative Example 1 Compound A | 0.5 | 50.5 |
| | 2 | 16.4 |
| | 4 | ND |
| Comparative Example 2 Compound B | 0.5 | 9.33 |
| | 2 | ND |
| | 4 | ND |

### Example 34 Pharmacokinetic evaluation of compounds

**Experimental objective:** the pharmacokinetics of compounds in SD rats was investigated.

**Experimental animals:** SD rats (male, 6-8 weeks old, body weight 250.13-262.17 g)
**Experimental operation:** pharmacokinetic characteristics of rodents after oral administration of the compounds were tested by a standard protocol. In the experiment, a corresponding compound was prepared into a 1 mg/mL clear solution, and administered intravenously or orally to the rats as a single dose. A vehicle was 10% DMSO / 80% PEG400 / 10% water. In the experiment, the male SD rats were administered intravenously at 1 mg/kg or administered orally by gavage at 5 mg/kg. Plasma samples were collected at 0.083 (intravenous group only), 0.25, 0.5, 1, 2, 4, 8 and 24 hours after administration, and finally, plasma drug concentrations were quantitatively analyzed by LC-MS/MS, and pharmacokinetic parameters were calculated, including clearance (Cl), maximum plasma concentration (Cₘₐₓ), half-life (T_{1/2}), time to maximum concentration (Tₘₐₓ), area under the concentration-time curve (AUC₀₋ₗₐₛₜ), and the like. Results were shown in Table 6.

**Table 6**

| Pharmacokinetic parameter | Positive control (Seltorexant) | KH16 | KH27 |
|---|---|---|---|
| T_{1/2} (h) | 0.77 | 1.20 | 1.08 |
| Tₘₐₓ (h) | 0.333 | 0.500 | 0.417 |
| AUC₀₋ₗₐₛₜ (ng.h/mL) | 479 | 654 | 751 |
| Bioavailability (%)a | 19.2 | 29.5 | 31.1 |

### Example 35 Animal pharmacodynamic study

**Experimental objective:** pharmacodynamic effects of test compounds on sleep in male SD rats were investigated by telemetry technology.

**Experimental animals:** SD rats (male, 5-6 weeks old, body weight approximately 300 g)

### Administration information:

**Table 7**

| Group | Number of animals per group | Vehicle | Administration regimen (dose \| frequency \| route) | Administration volume (mL/kg) |
|---|---|---|---|---|
| Negative control group | | 0.5%MC | 0 mg/kg, once daily, oral gavage | |
| Positive control (seltorexant) | 8 | 0.5%MC | 10 mg/kg, once daily, oral gavage | 5 |
| KH16 | | 20% HP-β-CD aqueous cyclodextrin | 5 mg/kg, once daily, oral gavage | |
| | | solution | | |

**Experimental method:** the animals were housed under a 12-h light/dark-cycle condition during an acclimation period (light on at 19:00; light off at 07:00). On the day of surgery, the animals were anesthetized with Zoletil (i.p., 20 mg/kg) in combination with xylazine (i.p., 8 mg/kg), and electrodes were implanted by surgery. A recovery period of 7 days was allowed after surgery. 3 days before compound administration, rats were given vehicle via gavage at 9:00 (at 2 hours into the dark phases) to acclimate the drug administration operation. Electroencephalogram (EEG) and electromyogram (EMG) signals were recorded at 1 hour before to 6 hours after vehicle administration, on the day before administration drugs. Subsequently, each group of rats dosed gavage their respective compounds at 9:00 (at 2 hours into the dark phases) on the day of administration drugs. EEG and EMG signals were recorded from 1 hour before to 6 hours after administration. The architectural changes in wake, non-rapid eye movement (NREM) sleep were analyzed after administration (including temporal parameters and latency physiological indicators).

**Data analysis:** The original data were collected by Ponemah software of DSI system and analyzed by NeuroScore software. The experimental data were represented by Mean±S.E.M. using Graph Pad Prism 8.0 software, One-way ANOVA was used for statistical analysis, and Dunnet post hoc was used for verification. *P or ^{#}P<0.05 indicated a significant difference, **P or ^{##}P<0.01 indicated a very significant difference, and ***P or ^{###}P<0.001 indicated an extremely significant difference.

### Experimental results:

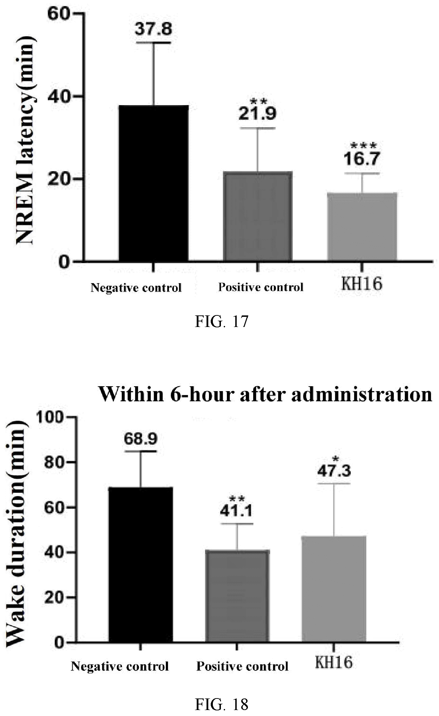

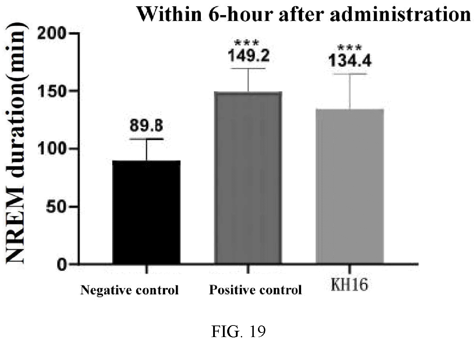

1) Effects of the control group and the drug administration groups on the NREM latency in male SD rats were shown in FIG. 17. As shown in FIG. 17, compared with the negative control group, the drug administration group of example KH16 compound showed significantly reduced NREM latency.
2) Effects of the control group and the drug administration groups on the wake duration of the SD rats were shown in FIG. 18. Results showed that, compared with the negative control group, the drug administration group of example KH16 compound significantly reduced the wake duration within 6 hours after administered.
3) Effects of the control group and the drug administration groups on the NREM duration (non-rapid eye movement sleep duration) of the SD rats were shown in FIG. 19, wherein, results showed that, compared with the negative control group, the drug administration group of example KH16 compound significantly increased the NREM duration within 6 hours after administered.

The above results showed that KH16 at the dose of 5 mg/kg had a sleep-improving effect in rats equivalent to that of the positive control drug at the dose of 10 mg/kg.

## Claims

1. A compound as shown in the following formula, a stereoisomer thereof or a pharmaceutically acceptable salt thereof, wherein the compound has the following structure: 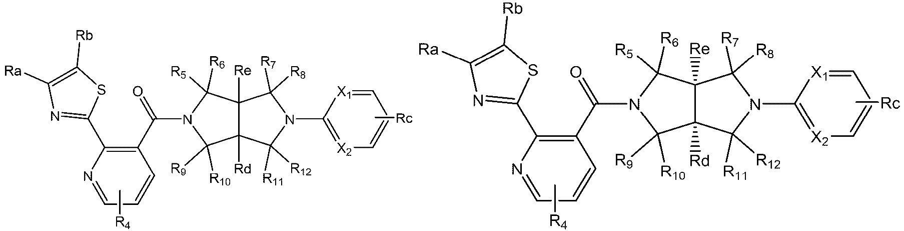
Ra, Rb and Rc are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, and the C3-8 cycloalkyl, C3-8 heterocyclyl, C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₄ is selected from H, deuterium, halogen, hydroxy, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C6-10 aryl and C5-10 heteroaryl, and the C3-8 cycloalkyl, C3-8 heterocyclyl, C6-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₅-R₁₂, Re and Rd are each independently selected from hydrogen, deuterium, halogen, C1-6 alkyl, C1-6 haloalkyl and C1-6 deuterated alkyl;
X₁ and X₂ are independently selected from CH and N; and
heteroatoms in the C3-8 heterocyclyl and C5-10 heteroaryl are one, two or three selected from N, O and S, and the number of the heteroatoms is 1, 2 or 3.

2. The compound, the stereoisomer thereof or the pharmaceutically acceptable salt thereof according to claim 1, wherein Ra, Rb and Rc are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, the C3-8 cycloalkyl, C3-8 heterocyclyl, C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₄ is selected from H, deuterium, halogen, hydroxy, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl and C3-8 heterocyclyl, and the C3-8 cycloalkyl and C3-8 heterocyclyl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy;
R₅-R₁₂, Re and Rd are each independently selected from hydrogen, deuterium, halogen, C1-6 alkyl, C1-6 haloalkyl and C1-6 deuterated alkyl; and
X₁ and X₂ are independently selected from CH and N.

3. The compound, the stereoisomer thereof or the pharmaceutically acceptable salt thereof according to claim 2, wherein the compound has a structure as shown in formula Ia or formula Ib: 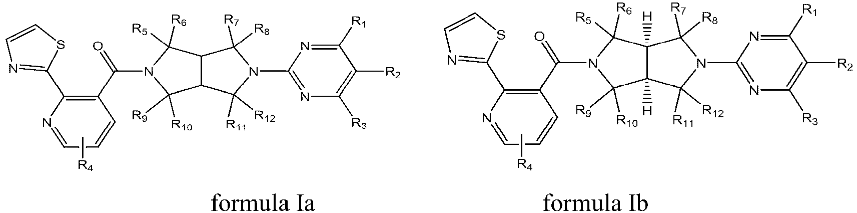
R₁, R₂ and R₃ are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, and the C3-8 cycloalkyl, C3-8 heterocyclyl, C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy; and
definitions of other substituents are as described in claim 2.

4. The compound, the stereoisomer thereof or the pharmaceutically acceptable salt thereof according to claim 2, wherein the compound has a structure as shown in formula IIIa or formula IIIb: 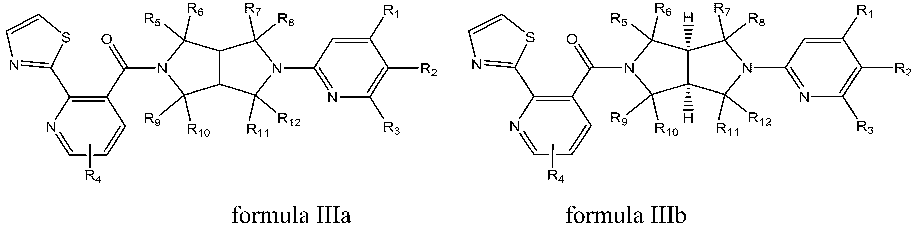
R₁, R₂ and R₃ are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy, C3-8 cycloalkyl, C3-8 heterocyclyl, C5-10 aryl and C5-10 heteroaryl, and the C3-8 cycloalkyl, C3-8 heterocyclyl, C5-10 aryl and C5-10 heteroaryl are optionally substituted with deuterium, halogen, amino, nitro, mercapto, cyano, carboxyl C1-6 alkyl, C1-6 haloalkyl, C1-6 deuterated alkyl, C1-6 hydroxyalkyl, C1-6 alkoxy, C1-6 haloalkoxy or C1-6 hydroxyalkoxy; and
definitions of other substituents are as described in claim 2.

5. The compound, the stereoisomer thereof or the pharmaceutically acceptable salt thereof according to claim 3, wherein the compound has a structure as shown in formula IIa or formula IIb: 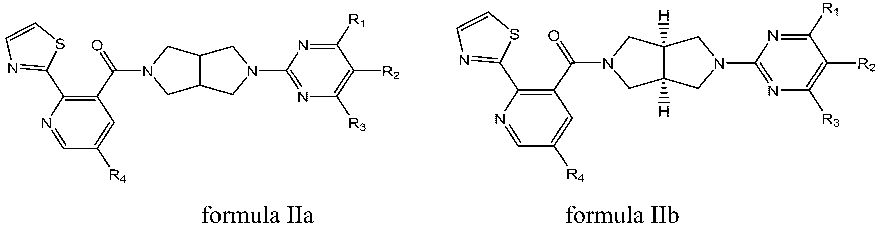 definitions of substituents are as described in claim 3.

6. The compound, the stereoisomer thereof or the pharmaceutically acceptable salt thereof according to claim 3, wherein R₁, R₂ and R₃ are each independently selected from H, deuterium, halogen, amino, nitro, mercapto, cyano, carboxyl, C1-6 alkyl, C1-6 haloalkyl, C1-6 deuterated alkyl, C1-6 hydroxyalkyl, C1-6 alkoxy, C1-6 haloalkoxy, C1-6 hydroxyalkoxy and C3-8 cycloalkyl; preferably, R₁, R₂ and R₃ are independently selected from C1-3 alkyl, C1-3 haloalkyl, C1-3 deuterated alkyl, C1-3 hydroxyalkyl, C1-3 alkoxy, C1-3 haloalkoxy, C1-3 hydroxyalkoxy and C3-8 cycloalkyl; preferably, R₁ and R₃ are independently selected from C1-3 alkyl, C1-3 deuterated alkyl, C1-3 haloalkyl, C1-3 hydroxyalkyl, C1-3 alkoxy and C1-3 haloalkoxy, and R₂ is hydrogen; preferably, R₁ and R₃ are independently selected from methyl, deuterated methyl or hydroxymethyl, and R₂ is hydrogen; and preferably, R₁, R₂ and R₃ are each independently selected from H, deuterium, halogen, C1-6 alkyl, C1-6 haloalkyl, C1-6 deuterated alkyl, C1-6 hydroxyalkyl, C1-6 alkoxy, C1-6 haloalkoxy and C1-6 hydroxyalkoxy.

7. The compound, the stereoisomer thereof or the pharmaceutically acceptable salt thereof according to claim 2, wherein R₄ is selected from halogen, hydroxy, C1-3 alkyl, C1-3 haloalkyl, C1-3 deuterated alkyl, C1-3 hydroxyalkyl, C1-3 alkoxy, C1-3 haloalkoxy, C1-3 hydroxyalkoxy and C3-8 cycloalkyl, and the C3-8 cycloalkyl is optionally substituted with deuterium, halogen or C1-3 alkyl; preferably, R₄ is selected from fluoro, trifluoromethyl or cyclopropyl, and the cyclopropyl is optionally substituted with halogen or methyl; and preferably, R₄ is hydrogen.

8. The compound, the stereoisomer thereof or the pharmaceutically acceptable salt thereof according to claim 2, wherein R₅-R₁₂ are each independently selected from hydrogen, deuterium and methyl.

9. The compound, the stereoisomer thereof or the pharmaceutically acceptable salt thereof according to claim 3 or 5, wherein the compound satisfies one or more of the following conditions:
(1) R₁ and R₃ are each independently selected from hydrogen, C1-6 alkyl, C1-6 deuterated alkyl and C1-6 hydroxyalkyl;
(2) R₂ is hydrogen or halogen;
(3) R₄ is H, C1-6 alkyl, C1-6 haloalkyl or C3-8 cycloalkyl; and
(4) R₅-R₁₂ are each independently hydrogen.

10. The compound, the stereoisomer thereof or the pharmaceutically acceptable salt thereof according to claim 9, wherein the compound satisfies one or two of the following conditions:
(1) R₁ and R₃ are each independently selected from hydrogen, C1-3 alkyl and C1-3 deuterated alkyl; and
(2) R₄ is hydrogen, C1-3 alkyl or C1-3 haloalkyl.

11. The compound, the stereoisomer thereof or the pharmaceutically acceptable salt thereof according to claim 9, wherein the compound satisfies one or more of the following conditions:
(1) R₁ and R₃ are each independently selected from hydrogen, methyl and deuterated methyl;
(2) R₂ is hydrogen or fluorine; and
(3) R₄ is hydrogen or trifluoromethyl.

12. The compound, the stereoisomer thereof or the pharmaceutically acceptable salt thereof according to claim 10, wherein R₁ and R₃ are each independently selected from C1-3 alkyl and C1-3 deuterated alkyl; and preferably, R₁ and R₃ are each independently selected from methyl and deuterated methyl.

13. The compound, the stereoisomer thereof or the pharmaceutically acceptable salt thereof according to claim 5, wherein the compound satisfies one or more of the following conditions:
(1) R₁ and R₃ are C1-3 deuterated alkyl;
(2) R₂ is hydrogen; and
(3) R₄ is hydrogen or C1-3 haloalkyl.

14. The compound, the stereoisomer thereof or the pharmaceutically acceptable salt thereof according to claim 13, wherein R₁ and R₃ are deuterated methyl.

15. The compound, the stereoisomer thereof or the pharmaceutically acceptable salt thereof according to claim 5, wherein R₁ and R₃ are independently C1-3 alkyl, R₂ is halogen, and R₄ is hydrogen; preferably, R₁ and R₃ are independently methyl; and preferably, R₂ is fluorine.

16. The compound, the stereoisomer thereof or the pharmaceutically acceptable salt thereof according to claim 1, wherein the compound has any one of the following structures: 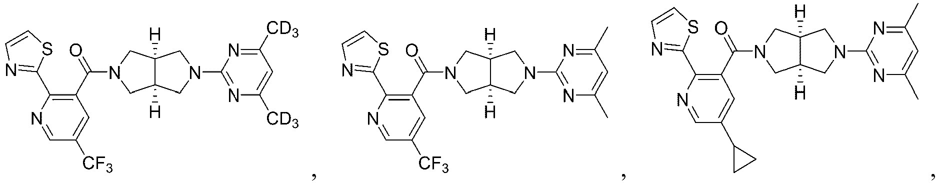 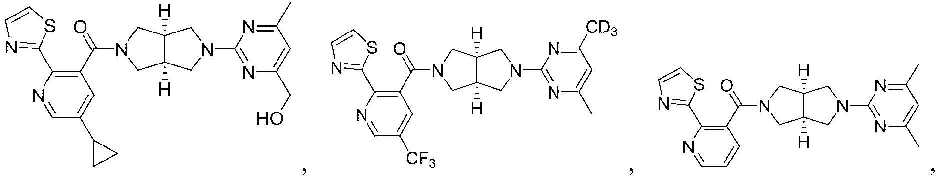 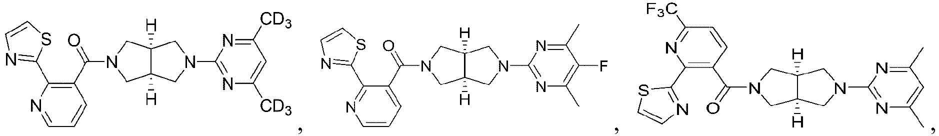 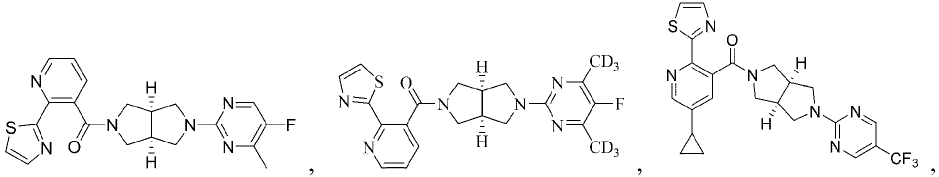 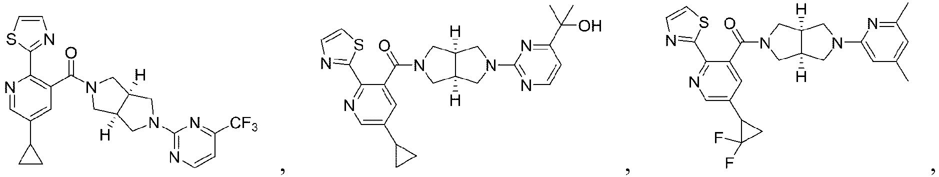 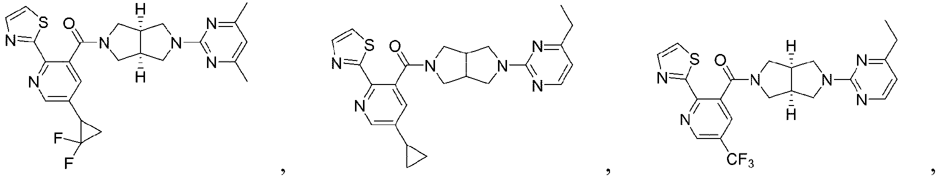 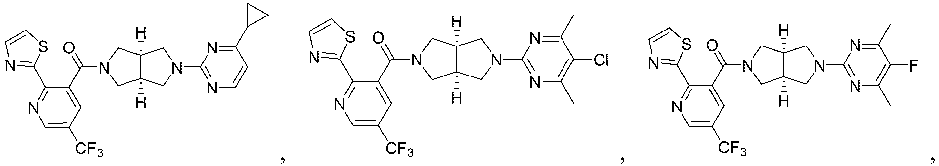 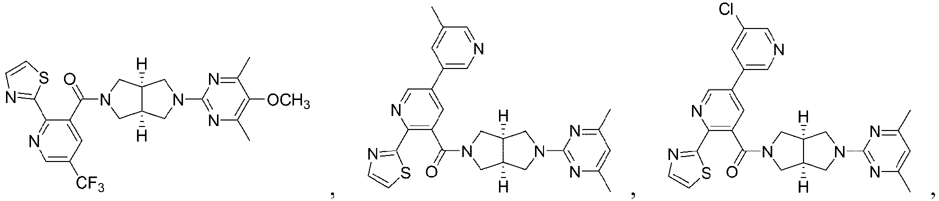 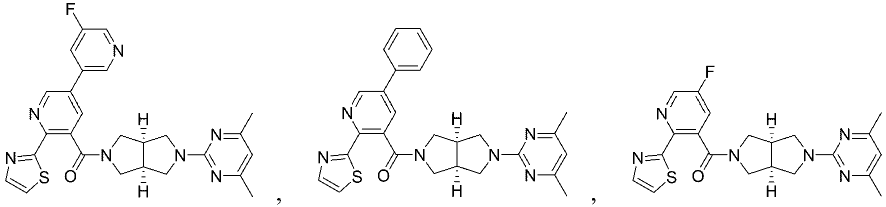 or

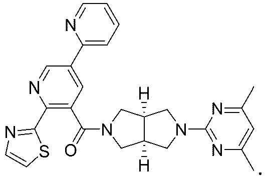

17. A pharmaceutical composition, comprising a therapeutically effective amount of the compound, the stereoisomer thereof or the pharmaceutically acceptable salt thereof according to any one of claims 1 to 16, and one or more pharmaceutically acceptable carriers or excipients.

18. A use of the compound, the stereoisomer thereof or the pharmaceutically acceptable salt thereof according to any one of claims 1 to 16, or the pharmaceutical composition according to claim 17, in manufacture of an orexin receptor antagonist; preferably in manufacture of an orexin-2 receptor antagonist.

19. A use of the compound, the stereoisomer thereof or the pharmaceutically acceptable salt thereof according to any one of claims 1 to 16, or the pharmaceutical composition according to claim 17, in manufacture of a drug for treating a nervous system disease, wherein the nervous system disease is preferably insomnia, depression or anxiety, or drug addiction, and more preferably major depressive disorder, primary and secondary insomnia, or depression accompanied by insomnia.
